# EUROPEAN PATENT APPLICATION

(11) **EP 1 582 585 A1**
(43) Date of publication of application: **05.10.2005**
(21) Application number: 03768314.1
(22) Date of filing: 26.12.2003
(51) Int. Cl.: C12N 15/09, C12N 9/14, C12Q 1/34, C12Q 1/68, C12Q 1/02, A61K 45/00, A61P 37/06, A61P 43/00

(54) **METHOD OF SELECTING NOVEL IMMUNOSUPPRESSANT**

(30) Priority: 27.12.2002 JP 2002378800
(71) Applicant: Astellas Pharma Inc., Tokyo 103-8411 (JP)
(72) Inventor: MATSUOKA, Hideaki, Astellas Pharma Inc., Chuo-ku, Tokyo 103-8411 (JP); FUJIMURA, Takao, Astellas Pharma Inc., Chuo-ku, Tokyo 103-8411 (JP); HAYASHI, Masako, Astellas Pharma Inc., Chuo-ku, Tokyo 103-8411 (JP); ARAMORI, Ichiro, Astellas Pharma Inc., Chuo-ku, Tokyo 103-8411 (JP)
(74) Representative: Gille Hrabal Struck Neidlein Prop Roos
(86) International application number: PCT/JP2003/016895
(87) International publication number: WO 2004/061101

(57) **Abstract**

The present invention provides a method of selecting an IL-2 production inhibitor and/or an immunocyte proliferation inhibitor having low GATA-1 production inhibitory activity, which comprises measuring the HDAC4 and/or HDAC8 inhibitory activity of a test substance, and a method of selecting an immunosuppressant having low thrombocytopenic activity, which comprises measuring the HDAC4 and/or HDAC8 inhibitory activity of a test HDAC inhibitor.

## Description

### Field of the Invention

The present invention relates to a method of selecting an immunosuppressant having low thrombocytopenic activity, and an IL-2 production inhibitor and/or immunocyte proliferation inhibitor having low GATA-1 production inhibitory activity, which selectively suppress HDAC4 and/or HDAC8 only, as well as an agent for suppressing immunity having low thrombocytopenic activity, and an agent for inhibiting IL-2 production and/or an agent for inhibiting immunocyte proliferation having low GATA-1 production inhibitory activity, which are obtained by the method, a kit used for the method, and the like.

### Background Art

Cyclosporin A (CsA) and tacrolimus (FK506), which are major immunosuppressants that are today widely used in clinical settings to suppress acute graft rejection reactions following organ transplantation, inhibit the activity of calcineurin, which is a Ca²⁺/calmodulin-dependent protein phosphatase, by binding to respective specific immunophilins (for example, cyclophilin for CsA, and FKBP12 for FK506). It is known that as a result, the dephosphorylation reaction of NF-AT (Nuclear Factor of Activated T-cell) is inhibited, which in turn inhibits the transfer of NF-AT into nucleus and suppresses the transcriptional activity of the IL-2 gene. Research into this action mechanism has shown that the specific suppression of the expression of the IL-2 gene at the transcription level in activated T-cells is of paramount importance to the suppression of organ graft rejection and the therapeutic efficacy for various autoimmune diseases and the like.

The cell nuclear DNA of eukaryotic organisms has a chromatin structure with nucleosome as the structural unit, and is folded in strata. Each nucleosome comprises two molecules of each of the H2A, H2B, H3 and H4 histones, and one molecule of the H1 histone. A particular lysine residue present on the N-terminal side of these histone proteins is known to often undergo modification by acetylation and to be associated with the expression regulation of various genes.

The level of the acetylation on the histone N-terminal side is exquisitely controlled in a balance of the activities of two mutually counteracting series of enzymes, that is, histone acetyltransferase (histone acetylation enzyme, HAT) and histone deacetylase (histone deacetylation enzyme, hereinafter abbreviated HDAC as required). Of these enzymes, histone deacetylase, a metal enzyme having zinc coordinated at the active center thereof, is responsible for the function of removing the acetyl group from acetylated histone; this acetyl group removal is considered to cause aggregation of the chromatin structure and lead to suppressed transcription.

A series of studies have shown that human histone deacetylase occurs in at least 11 kinds of isoforms, from HDAC1 to HDAC11 (see, for example, Pandey R et al., Nucleic Acids Research, 30(23): 5036-55 (2002); L. Gao et al., Journal of Biological Chemistry, 277(28): 25748-25755 (2002); Joseph J. BUGGY et al., Biochemical Journal, 350: 199-205 (2000); International Patent Publication No. WO00/10583). As such, each isoform has been shown not to function as a single enzyme, but to interact with many other proteins to form a larger complex. Of these HDAC isoforms, HDAC1, HDAC2, HDAC3 and HDAC8 are structurally highly homologous to the yeast Rpd3 protein, and are classified under class I. HDAC1 and HDAC2 are components of a complex comprising a large number of subunit structures, and are found in, for example, the SIN3 complex and the NURD/Mi2 complex. HDAC3 is known to form a complex with N-CoR (nuclear receptor corepressor) or SMRT (silencing mediator of retinoid and thyroid) and mediate the transcription suppression by, for example, thyroid hormone receptor (TR) or v-ErbA. On the other hand, a group of enzymes that are homologous to the yeast Hda1 protein are classified under class II, and HDAC4, HDAC5, HDAC6 and HDAC7 are known to belong to this group. HDAC4, HDAC5 and HDAC7, which are class II histone deacetylases, are known to be capable of interacting with BCoR, which is a supplementary co-repressor that mediates the suppression by N-CoR, SMRT or BCL-6.

Although the recombinant HDAC3 itself is inactive as a single substance, data suggestive of its capability of functioning as an active enzyme with the addition of SMRT or N-CoR have been reported (see, for example, Matthew G. Guenther et al., Molecular and Cellular Biology, 21(18): 6091-6101 (2001)), and HDAC4, which is a class II enzyme, has been reported to interact with HDAC3, which is a class I enzyme, via SMRT or N-CoR in vivo (see, for example, Wolfgang Fischie et al., Molecular Cell, 9: 45-57 (2002)). However, much remains unknown concerning the accurate mechanism by which these HDAC isoforms are activated.

To date, extensive screening has discovered a large number of compounds that have HDAC inhibitory activity, including compounds that have remarkable IL-2 production inhibitory activity (see, for example, I. Takahashi et al., The Journal of Antibiotics, 49: 453-457 (1995)), which are attracting attention as candidates for immunosuppressants that supplement cyclosporin and tacrolimus. In fact, among the compounds thus selected, those that exhibit excellent immunosuppressive action in vivo have been found. FR225497 has been found to be excellently effective as a therapeutic agent or prophylactic agent for organ transplant rejection and autoimmune diseases with its potent immunosuppressive action (see, for example, International Patent Publication No. WO00/08048) and, in addition, has been suggested to be useful as a therapeutic agent or prophylactic agent for many other diseases considered to develop due to an abnormality in gene expression. Examples of such diseases include inflammatory disorders, diabetes, diabetic complications, homozygous thalassemia, fibrosis, liver cirrhosis, acute promyelocytic leukemia (APL), protozoal infection, cancers (tumors) and the like. However, despite this utility, many of these compounds that have HDAC inhibitory activity pose the problem of being likely to cause serious thrombocytopenia as a side effect when administered to the body, and this aspect has made it difficult to use such compounds as actual therapeutic drugs.

The reason why many of immunosuppressive HDAC inhibitors are likely to cause thrombocytopenia as a side effect has not yet fully been elucidated. However, the present inventors previously showed that the suppression of the expression of the gene for GATA-1 (also called GATA-1 binding protein, GF-1, NF-E1 or Eryf 1) is profoundly involved in the thrombocytopenic activity of HDAC inhibitors as a serious side effect, and that compounds with more potent platelet suppressive action tend to more strongly suppress the transcription of the GATA-1 gene (see Japanese Patent Application No. 2002-203901).

GATA-1 is a DNA-binding protein that recognizes the (A/T)GATA(A/G) consensus sequence, which is characteristically present in the transcription control regions of hematopoietic genes. This GATA motif sequence is found in a variety of control regions such as enhancer regions of various globin genes, locus control regions (LCRs) of the β-globin gene, enhancer regions of the T-cell receptor α-chain and δ-chain genes, and in promoters. Also, GATA-1 mRNA is expressed at high levels in mature erythrocytes, mast cells, megakaryocyte and the like, with slight expression observed in multifunctional precursor cells and juvenile mouse testis. Furthermore, mice having the GATA-1 gene knocked out by an ordinary method are lethal due to primary hematopoietic cell hypoplasia in the stage of embryogenesis (see, for example, Y. Fujiwara et al., Proceedings of the National Academy of Sciences of the USA, 93: 12355-12358 (1996)). On the other hand, it is possible to prepare mice having only the expression of the GATA-1 gene in megakaryocytic lineage knocked out selectively, and these mice have been shown to suffer dramatically decreased platelet counts and to lack normal maturation of megakaryocytic lineage (see, for example, R.A. Shivdasani et al. The EMBO Journal, 16: 3965-3973 (1997)).

In HDAC, a large number of isozymes exist, some of which have a side effect serious to the body when inhibited. For example, administering a conventional HDAC inhibitor, which has no isozyme selectivity, causes thrombocytopenia as a side effect, and the present inventors showed that this action is possibly attributable to the transcription suppressive action of the GATA-1 gene, which plays a decisive role in the differentiation and maturation of erythrocytic lineage cells and megakaryocytic lineage, and provided a method of screening for an immunosuppressant having low thrombocytopenic activity based on this action (see Japanese Patent Application No. 2002-203901). However, in this screening method, to screen for an immunosuppressant having low thrombocytopenic activity, it is necessary to separately evaluate the platelet suppressive action of a compound possessing HDAC inhibitory action after the compound is screened for, so that much labor and time are taken and the screening method is not always satisfactory. Accordingly, to select an immunosuppressant from among HDAC inhibitors, there is a strong demand for the provision of an excellent method of screening for an immunosuppressant that inhibits a particular isozyme involved in immunosuppressive effects, but does not inhibit other isozymes involved in side effects such as thrombocytopenic activity, conveniently and in a short time, and the present invention is directed to resolve these problems.

### Disclosure of the Invention

The present inventors diligently studied the association of the transcription suppressive activities for the IL-2 gene and the GATA-1 gene and various HDACs, and found that if either or both of HDAC4 or HDAC8 out of the various HDACs, are selectively suppressed, IL-2 production can be inhibited without noticeably suppressing GATA-1 production inhibitory activity, the proliferation of immunocytes can be inhibited, and immunity can be suppressed without significantly decreasing the platelet count. The present inventors confirmed that HDAC4 forms a complex with N-CoR, and the complex of HDAC4 and N-CoR further forms a complex with HDAC3. Accordingly, the present inventors realized that an IL-2 production inhibitor having low GATA-1 production inhibitory activity, an immunocyte proliferation inhibitor, or an immunosuppressant having low thrombocytopenic activity, can be obtained by selecting a compound that selectively suppresses HDAC4 and/or HDAC8 out of various HDACs, and inhibiting the formation of a complex of HDAC4 and N-CoR and, as required, the formation of a higher complex of HDAC4 and the N-CoR-HDAC3 complex, and developed the present invention.

Accordingly, the present invention relates to the inventions shown below.
[1] an agent for inhibiting IL-2 production and/or an agent for inhibiting immunocyte proliferation which have low GATA-1 production inhibitory activity, comprising a selective HDAC4 and/or HDAC8 inhibitor.
[2] an agent for suppressing immunity having low thrombocytopenic activity, comprising a selective HDAC4 and/or HDAC8 inhibitor.
[3] a method of selecting an IL-2 production inhibitor and/or an immunocyte proliferation inhibitor which have low GATA-1 production inhibitory activity, which comprises measuring the HDAC4 and/or HDAC8 enzyme inhibitory activity of a test substance.
[4] a method of selecting an immunosuppressant having low thrombocytopenic activity, which comprises measuring the HDAC4 and/or HDAC8 enzyme inhibitory activity of a test substance.
[5] a method of selecting an immunosuppressant having low thrombocytopenic activity, which comprises steps (i) and (ii) below:
   (i) measuring the HDAC4 and/or HDAC8 enzyme inhibitory activity of a test substance, and
   (ii) measuring the HDAC enzyme inhibitory activity of the test substance, wherein the HDAC enzyme inhibitory activity is one or more HDAC enzyme inhibitory activities selected from a group consisting of HDAC1, HDAC2, HDAC3, HDAC5, HDAC6 and HDAC7 enzyme inhibitory activities.
[6] the method described in [3], which is a method of selecting an IL-2 production inhibitor and/or an immunocyte proliferation inhibitor which have low GATA-1 production inhibitory activity, and which comprises steps (i) to (iii) below, wherein the IL-2 production inhibitor and/or the immunocyte proliferation inhibitor selectively inhibits HDAC4 and/or HDAC8 enzyme activity:
   (i) expressing each of the HDAC1, HDAC2, HDAC3, HDAC4, HDAC5, HDAC6, HDAC7 and HDAC8 genes to obtain respective enzyme solutions;
   (ii) measuring HDAC enzyme activity for each of the enzyme solutions in the presence of a test substance;
   (iii) selecting a test substance that selectively suppresses HDAC enzyme activity only when using an enzyme solution obtained by expressing the HDAC4 gene or the HDAC8 gene.
[7] the method described in [4], which is a method of selecting an immunosuppressant having low thrombocytopenic activity, and which comprises steps (i) to (iii) below, wherein the immunosuppressant selectively inhibits HDAC4 and/or HDAC8 enzyme activity:
   (i) expressing each of the HDAC1, HDAC2, HDAC3, HDAC4, HDAC5, HDAC6, HDAC7 and HDAC8 genes to obtain respective enzyme solutions;
   (ii) measuring HDAC enzyme activity for each of the enzyme solutions in the presence of a test substance;
   (iii) selecting a test substance that selectively suppresses HDAC enzyme activity only when using an enzyme solution obtained by expressing the HDAC4 gene or the HDAC8 gene.
[8] the method described in [4], which is a method of selecting an immunosuppressant having low thrombocytopenic activity, and which comprises steps (i) to (iii) below, wherein the immunosuppressant selectively inhibits HDAC4 and/or HDAC8 enzyme activity:
   (i) measuring HDAC enzyme activity for an enzyme solution partially purified from human cells, in the presence of a test substance;
   (ii) measuring HDAC enzyme activity for an enzyme solution obtained by expressing the HDAC4 gene and/or the HDAC8 gene, in the presence of a test substance;
   (iii) selecting a test substance that selectively suppresses HDAC enzyme activity only when using an enzyme solution obtained by expressing the HDAC4 gene and/or the HDAC8 gene, by comparing the enzyme activities measured in (i) and (ii).
[9] a method of selecting an immunosuppressant having low thrombocytopenic activity, which comprises steps (i) to (iii) below, wherein an HDAC inhibitor that specifically inhibits the formation of a complex of HDAC4 and N-CoR is selected from among test HDAC inhibitors:
   (i) expressing the HDAC4 gene and the N-CoR gene to obtain an enzyme solution;
   (ii) allowing the enzyme solution to coexist with a test HDAC inhibitor;
   (iii) determining whether or not a complex of HDAC4 and N-CoR is formed in the mixture of enzyme solution and test HDAC inhibitor obtained in (ii).
[10] a method of selecting an immunosuppressant having low thrombocytopenic activity, which comprises steps (i) and (ii) below, wherein an HDAC inhibitor that specifically inhibits the formation of a complex of the HDAC4 fusion protein comprising at least a portion of the HDAC4 protein, and the N-CoR fusion protein comprising at least a portion of the N-CoR protein, is selected from among test HDAC inhibitors:
   (i) expressing the gene that encodes the HDAC4 fusion protein and the gene that encodes the N-CoR fusion protein in cells;
   (ii) determining whether or not the test HDAC inhibitor inhibits the formation of a complex of the HDAC4 fusion protein and the N-CoR fusion protein.
[11] a method of selecting a compound having low thrombocytopenic activity and having immunosuppressive activity, which comprises steps (i) and (ii) below, wherein an HDAC inhibitor that suppresses the expression level of HDAC4 and/or HDAC8 is selected from among test HDAC inhibitors:
   (i) allowing cells that express HDAC4 and/or HDAC8 to coexist with a test HDAC inhibitors;
   (ii) measuring the HDAC4 and/or HDAC8 expression level in the cells.
[12] a method of selecting a compound having low thrombocytopenic activity and having immunosuppressive activity, which comprises steps (i) and (ii) below, wherein an HDAC inhibitor that selectively inhibits the binding of HDAC4 and/or HDAC8 and an HDAC4- and/or HDAC8-specific ligand is selected from among test HDAC inhibitors:
   (i) expressing the HDAC4 and/or HDAC8 gene to obtain an enzyme solution;
   (ii) measuring the binding activity of the HDAC4 and/or HDAC8 enzyme and the HDAC4- and/or HDAC8-specific ligand for the enzyme solution in the presence of an HDAC4- and/or HDAC8-specific ligand and a test HDAC inhibitor.
[13] an assay kit for the selection of an immunosuppressant having low thrombocytopenic activity including a selective HDAC4 and/or HDAC8 inhibitor, which includes at least (i) and (ii) below:
   (i) enzyme solutions prepared by expressing each of the HDAC1, HDAC2, HDAC3, HDAC5, HDAC6 and HDAC7 genes;
   (ii) an enzyme solution prepared by expressing each of the HDAC4 and/or HDAC8 gene;
[14] a DNA having the base sequence that encodes the amino acid sequence shown by SEQ ID NO:4 or SEQ ID NO:6.
[15] an HDAC4 variant that is the amino acid sequence shown by SEQ ID NO:4 or SEQ ID NO:6.
[16] a method of inhibiting IL-2 production and/or immunocyte proliferation with low GATA-1 production inhibitory activity, which comprises administering an effective amount of selective HDAC4 and/or HDAC8 inhibitor to a subject (for example, any animal, preferably a mammal (for example, human, mouse, rat, rabbit, dog, cat, bovine, horse, goat, sheep and the like), most preferably a human).
[17] a method of suppressing immunity with low thrombocytopenic activity, which comprises administering an effective amount of selective HDAC4 and/or HDAC8 inhibitor to a subject (same as above).
[18] use of a selective HDAC4 and/or HDAC8 inhibitor for the production of an agent for inhibiting IL-2 production and/or an agent for inhibiting immunocyte proliferation which have low GATA-1 production inhibitory activity.
[19] use of a selective HDAC4 and/or HDAC8 inhibitor for the production of an agent for suppressing immunity having low thrombocytopenic activity.

### Brief Description of the Drawings

FIG. 1 shows the results of a confirmation of the base sequence a 731-basepair fragment in the human IL-2 promoter region, amplified by PCR. The base sequence in the upper panel was obtained from GenBank accession number X00695 (6684 bp in full length), a portion of which (SEQ ID NO:29) was used as a reference control. The base sequence in the lower panel is the base sequence of the IL-2 promoter region in pGL3 IL2 pro prepared in Example 1 (SEQ ID NO:23).
FIG. 2 shows the results of a confirmation of the base sequence a 368-basepair fragment in the human IL-2 promoter region, amplified by PCR. The base sequence in the upper panel was obtained from GenBank accession number X00695 (6684 bp in full length), a portion of which (SEQ ID NO:31) was used as a reference control. The base sequence in the lower panel is the base sequence of the IL-2 promoter region in pGL3 IL2 pro43 prepared in Example 1 (SEQ ID NO:24).
FIG. 3 shows the results of a confirmation of the base sequence an 821-basepair fragment in the human GATA-1 promoter region, amplified by PCR. The base sequence in the upper panel was obtained from GenBank accession number AF196971 (113853 bp in full length), a portion of which (SEQ ID NO:84) was used as a reference control. The base sequences in the lower panel are the base sequence of the GATA-1 promoter region in pGL3-IE prepared in Example 2, and the base sequence of a portion of the GATA-1 promoter region in pGL3-HSI-IE Pro (SEQ ID NO:25).
FIG. 4 shows the results of a confirmation of the base sequence a 637-basepair fragment in the human GATA-1 promoter region, amplified by PCR. The base sequence in the upper panel was obtained from GenBank accession number AF196971 (113853 bp in full length), a portion of which (SEQ ID NO:85) was used as a reference control. The base sequence in the lower panel is the base sequence of a portion of the GATA-1 promoter region in pGL3-HSI-IE Pro, prepared in Example 2 (SEQ ID NO:26).
FIG. 5 shows the effects of the overexpression of various HDAC isozymes on IL-2 transcriptional activity (upper panel, n=3) and cell proliferation rate (lower panel).
FIG. 6 shows the effects of the overexpression of various HDAC isozymes on GATA-1 transcriptional activity (n=2).
FIG. 7 summarizes the HDAC4 dominant negative variants prepared in Example 6.
FIG. 8 shows IL-2 transcription suppression depending on the expression level of HDAC4 dominant negative variants (H802K, H803L, H863L). The amount of plasmid used for transfection (n=3) is shown on each graph.
FIG. 9 shows the Jurkat-cell-specific cell proliferation inhibition due to the expression of an HDAC4 dominant negative variant (H863L).
FIG. 10 shows the effects of the expression of HDAC4 dominant negative variants (H802K, H803L, H863L) in Jurkat cells and HEL cells on IL-2 and GATA-1 transcription. The upper panel shows the results of an IL-2 reporter gene assay in Jurkat cells for normal HDAC1, HDAC3 and HDAC4, and variants thereof. The lower panel shows the results of a GATA-1 reporter gene assay in HEL cells for normal HDAC1, HDAC3 and HDAC4, and variants thereof.
FIG. 11 shows the expression of HDAC4, HDAC5 and HDAC7 in PEAK Rapid cells (Edge BioSystems), and the formation of a complex of HDAC4, HDAC5 or HDAC7 with N-CoR and HDAC3.
FIG. 12 shows the capability of forming a complex of an HDAC4 dominant negative variant (H863L).
FIG. 13 shows the HDAC activity in wild-type HDAC4 and HDAC4 dominant negative variants (H802K, H803L, H863L).
FIG. 14 summarizes the interaction of HDAC-4 and N-CoR using a two-hybrid system. The upper panel shows the mode of transcription regulation with the interaction of HDAC-4 and N-CoR, and the lower panel shows the mode of transcription regulation without the interaction of HDAC-4 and N-CoR.
FIG. 15 shows the interaction of HDAC-4 and N-CoR using a two-hybrid system.
FIG. 16 shows the effects of HDAC4-specific siRNA on IL-2 and GATA-1 transcription in Jurkat cells and HEL cells. The left panel shows the results of an IL-2 reporter gene assay in Jurkat cells for HDAC4-specific siRNA. The right panel shows the results of a GATA-1 reporter gene assay in HEL cells for HDAC4-specific siRNA.

### DETAILED DESCRIPTION OF THE INVENTION

The present invention is hereinafter described in detail.

The present invention relates to an agent for inhibiting IL-2 production (IL-2 production inhibitor) and/or an agent for inhibiting immunocyte proliferation (immunocyte proliferation inhibitor), each of which having low GATA-1 production inhibitory activity, comprising a selective HDAC4 and/or HDAC8 inhibitor. An IL-2 production inhibitor and/or an immunocyte proliferation inhibitor, each of which having low GATA-1 production inhibitory activity, comprising a selective HDAC4 and/or HDAC8 inhibitor can be obtained by, for example, the selection method described below. It is also possible to obtain an IL-2 production inhibitor and/or an immunocyte proliferation inhibitor, each of which having low GATA-1 production inhibitory activity, comprising a selective HDAC4 and/or HDAC8 inhibitor, by using the selection method described below and a method known in the art in combination.

A "selective HDAC4 and/or HDAC8 inhibitor" refers to an inhibitor that suppresses the HDAC enzyme activity of HDAC4 and/or HDAC8 more than suppresses the HDAC enzyme activity of HDAC1-3 and HDAC5-7, and is preferably an inhibitor that does not or substantially does not suppress the HDAC enzyme activity of HDAC1-3 and HDAC5-7, and that suppresses the HDAC enzyme activity of HDAC4 and/or HDAC8. As examples of the selective HDAC4 and/or HDAC8 inhibitor, an antisense nucleic acid, ribozyme, decoy nucleic acid, siRNA and antibody (for example, single-chain antibody) that are specific for HDAC4 and/or HDAC8, a dominant negative variant of HDAC4 and/or HDAC8, an expression vector carrying a nucleic acid that encodes one of them, and a substance obtained by the selection method described below, can be mentioned.

"GATA-1 production inhibitory activity" may be any activity to suppress GATA-1 production. As examples of the mechanism of suppression of GATA-1 production, suppression at the transcription level, promotion of GATA-1 mRNA decomposition, suppression at the translation level and the like can be mentioned. GATA-1 production inhibitory activity can be evaluated by a method known in the art. Specifically, GATA-1 production inhibitory activity can be evaluated by a reporter assay using the GATA-1 reporter gene as described below, or by measuring the expression level of GATA-1 mRNA or GATA-1 protein in a cell line, primary culture cells and the like that are naturally expressing GATA-1. "Low" GATA-1 production inhibitory activity as evaluated with the addition of a compound means that the activity thereof to inhibit GATA-1 production is lower than that obtained when a compound for a reference control is added; for example, whether or not the GATA-1 production inhibitory activity is low can be evaluated by an assay using the GATA-1 reporter gene. As an example of the compound used as a reference control, tricostatin (TSA), which is a non-specific HDAC inhibitor, can be mentioned.

The "immunocyte proliferation inhibitor" may have any kind of activity, as long as it inhibits the proliferation of immunocytes. As examples of the mechanism of cell proliferation inhibition, cell cycle termination, apoptosis induction and the like can be mentioned. Immunocyte proliferation inhibitory activity can be evaluated by a method known in the art. Specifically, immunocyte proliferation inhibitory activity can be evaluated by thymidine uptake activity, the MTT method and the like. Here, "immunocytes" refer to cells responsible for immune function in the body. Preferably, "immunocytes" refer to lymphocytes, dendritic cells and the like that are capable of produce an antibody or of expressing a cellular immune reaction, more preferably T-cells.

The "IL-2 production inhibitor" may have any kind of activity, as long as it has IL-2 production inhibitory activity, that is, an activity to suppress IL-2 production. As examples of the mechanism of IL-2 production inhibition, suppression at the transcription level, promotion of IL-2 mRNA decomposition, suppression at the translation level and the like can be mentioned. IL-2 production inhibitory activity can be evaluated by a method known in the art. Specifically, IL-2 production inhibitory activity can be evaluated by a reporter assay using the IL-2 reporter gene as described in Examples below, or by measuring the expression level of IL-2 mRNA or IL-2 protein in a cell line, primary culture cells and the like that are naturally expressing the IL-2.

The present invention also relates to an agent for suppressing immunity (immunosuppressant) having low thrombocytopenic activity, comprising a selective HDAC4 and/or HDAC8 inhibitor. An immunosuppressant having low thrombocytopenic activity, comprising a selective HDAC4 and/or HDAC8 inhibitor can be obtained by, for example, the selection method described below. It is also possible to obtain an immunosuppressant having low thrombocytopenic activity, comprising a selective HDAC4 and/or HDAC8 inhibitor, by using the selection method described below and a method known in the art in combination.

"Thrombocytopenic activity" refers to an activity to decrease the platelet count, and this thrombocytopenic activity can be directly confirmed by, for example, a method known in the art, such as administering a compound to a laboratory animal. Also, because it is known that there is a correlation between thrombocytopenic activity and GATA-1 production inhibitory activity, the thrombocytopenic activity can also be indirectly confirmed with GATA-1 production inhibitory activity as an index. "Low" thrombocytopenic activity of a compound or a drug means that the action to decrease the platelet count is lower when the compound or the drug is administered to a human or a laboratory animal and the like, than when a compound used as a reference control is administered, and it is also possible to confirm whether or not the thrombocytopenic activity is low, by, for example, determining whether or not the GATA-1 production inhibitory activity is low by a method described above. As an example of the compound used as a reference control, Oxamflatin can be mentioned.

An "immunosuppressant" refers to an agent that induces immunosuppression; as an example of the immunosuppressant, an immunosuppressant having an activity to suppress IL-2 production can be mentioned. Therefore, the immunosuppressant can be obtained by, for example, determining whether or not it has IL-2 transcription suppressive activity using a method described above.

The present invention also relates to a method of selecting an IL-2 production inhibitor and/or an immunocyte proliferation inhibitor, each of which having low GATA-1 production inhibitory activity, or an immunosuppressant having low thrombocytopenic activity, which comprises measuring the HDAC4 and/or HDAC8 enzyme inhibitory activity of a test substance.

Specifically, the present invention relates to a method of selecting an IL-2 production inhibitor and/or an immunocyte proliferation inhibitor, each of which having low GATA-1 production inhibitory activity, or an immunosuppressant having low thrombocytopenic activity, which comprises (i) measuring the HDAC4 and/or HDAC8 enzyme inhibitory activity of a test substance, and (ii) measuring the HDAC enzyme inhibitory activity of the test substance (here, the HDAC enzyme inhibitory activity is one or more HDAC enzyme inhibitory activities selected from a group consisting of HDAC1, HDAC2, HDAC3, HDAC5, HDAC6 and HDAC7 enzyme inhibitory activities). In (ii) of this method, one or more HDAC enzyme inhibitory activities selected from a group consisting of HDAC1, HDAC2, HDAC3, HDAC5, HDAC6 and HDAC7 enzyme inhibitory activities are measured in the presence of the test substance; preferably all of HDAC1, HDAC2, HDAC3, HDAC5, HDAC6 and HDAC7 enzyme inhibitory activities are measured. This method may further comprises (iii) determining whether or not the test substance selectively inhibits HDAC4 and/or HDAC8 enzyme activity, on the basis of the results of (i) and (ii).

More specifically, the present invention relates to a method of selecting an IL-2 production inhibitor and/or an immunocyte proliferation inhibitor, each of which having low GATA-1 production inhibitory activity, or an immunosuppressant having low thrombocytopenic activity, wherein the IL-2 production inhibitor and/or the immunocyte proliferation inhibitor selectively inhibits HDAC4 and/or HDAC8 enzyme activity. In one embodiment of the present invention, the above-described method comprises (i) expressing the HDAC1, HDAC2, HDAC3, HDAC4, HDAC5, HDAC6, HDAC7 and HDAC8 genes to obtain respective enzyme solutions, then (ii) measuring HDAC enzyme activity for each of the enzyme solutions in the presence of a test substance, and (iii) selecting a test substance that selectively suppresses HDAC enzyme activity only when using an enzyme solution obtained by expressing the HDAC4 gene or the HDAC8 gene.

In another embodiment, the above-described method comprises (i) measuring HDAC enzyme activity for an enzyme solution partially purified from human cells, in the presence of a test substance, (ii) measuring HDAC enzyme activity for an enzyme solution obtained by expressing the HDAC4 gene and/or the HDAC8 gene, in the presence of the test substance, and (iii) selecting a test substance that selectively suppresses HDAC enzyme activity only when using an enzyme solution obtained by expressing the HDAC4 gene and/or the HDAC8 gene, by comparing the enzyme activities measured in (i) and (ii). The human cells used in (i) in this method are not subject to limitation, as long as they are of a human-derived cell line; preferably, a cell lines derived from the above-described immunocytes are used, and most preferably, Jurkat cells (ATCC accession number TIB-152) are used.

The above-described "test substance" is not subject to limitation, as long as it is a candidate substance that can be applied to an assay system according to the method of the present invention, and includes all compounds such as low-molecular organic compounds, low-molecular inorganic compounds, high-molecular compounds including proteins and nucleic acids, saccharides and the like, and also includes all of their mixtures, natural products, synthetic products, and extracts from animals, plants, fungi, algae and microorganisms. As examples of the "test substance", HDAC inhibitors (see, for example, International Patent Publication Nos. WO02/085883, WO02/085400, WO02/076941, WO02/062773, WO02/46129, WO02/07722, WO02/06307, WO01/70675, WO01/38322, WO01/16106, WO00/71703, WO00/21979 and WO00/08048; Japanese Patent Publication Nos. 2001-348340 and HEI-11-302173; US Patent Publications US 2002/0120099 and US 2002/0115826; US Patent Nos. 6,638,530, 6,541,661 and 6,399,568) can be used. The "test substance" may be a known compound or a newly discovered or synthesized compound.

An "HDAC inhibitor" refers to a compound that inhibits HDAC enzyme activity, that is, a compound having HDAC enzyme inhibitory activity.

"HDAC enzyme activity" refers to an activity to remove the acetyl group from the lysine residue of an acetylated protein, for example, a histone (i.e., deacetylation activity), and may be the deacetylation activity of a particular HDAC isozyme, or the deacetylation activity of one or two or more unspecified HDAC isozymes. On the other hand, "HDAC4 enzyme activity" refers to the deacetylation activity of HDAC4, and "HDAC8 enzyme activity" refers to the deacetylation activity of HDAC8. HDAC1 enzyme activity, HDAC2 enzyme activity, HDAC3 enzyme activity, HDAC4 enzyme activity, HDAC5 enzyme activity, HDAC6 enzyme activity, and HDAC7 enzyme activity are also defined similarly. HDAC enzyme activity can be measured by reacting an enzyme solution containing each HDAC enzyme (hereinafter abbreviated "enzyme solution" as required) and a substrate of the HDAC enzyme in the presence/absence of a test substance by a method known in the art.

"HDAC enzyme inhibitory activity" refers to an activity to suppress the deacetylation of the lysine residue of a protein, and may be an activity to specifically or non-specifically suppress the deacetylation by a particular HDAC isozyme. On the other hand, "HDAC4 enzyme inhibitory activity" refers to an activity to suppress the deacetylation by HDAC4, and "HDAC8 enzyme inhibitory activity" refers to an activity to suppress the deacetylation by HDAC8. HDAC1 enzyme inhibitory activity, HDAC2 enzyme inhibitory activity, HDAC3 enzyme inhibitory activity, HDAC4 enzyme inhibitory activity, HDAC5 enzyme inhibitory activity, HDAC6 enzyme inhibitory activity, and HDAC7 enzyme inhibitory activity are also defined similarly.

As the substrate used in the measurement of HDAC enzyme activity, a substrate known in the art can be used, and representatively, a [³H]acetyl-labeled histone, a fluorescently labeled acetylated peptide and the like can be used. For example, when a [³H]acetyl-labeled histone is used as a substrate, the [³H]acetyl-labeled histone can be obtained from Jurkat cells. Specifically, the following procedure is followed. 1 × 10⁸ Jurkat cells are incubated with 300 MBq [³H] sodium acetate in an RPMI-1640 medium supplemented with 10% FBS, penicillin (50 units/ml) and streptomycin (50 µg/ml), in the presence of 5mM sodium butyrate, for 30 minutes (5% CO₂, 37°C), the cells are recovered in a centrifugal tube (50 ml), collected via centrifugation (1000 rpm, 10 minutes), and once washed with phosphate-buffered saline. The washed cells are suspended in 15 ml of an ice-cooled lytic buffer (10mM Tris-HCl, 50mM sodium hydrogen sulfite, 1% Triton X-100, 10mM MgCl₂, 8.6% sucrose, pH 6.5). After Dounce homogenization (30 strokes), nuclei are collected via centrifugation (1000 rpm, 10 minutes), washed with 15 ml of the lytic buffer three times, and then once washed with 15 ml of an ice-cooled washing buffer (10mM Tris-HCl, 13mM EDTA, pH 7.4). The pellet is suspended in 6 ml of ice-cooled water using a mixer, and 68 ml of H₂SO₄ is added to this suspension to obtain a concentration of 0.4N. After incubation at 4°C for 1 hour, the suspension is centrifuged (15,000 rpm, 5 minutes), and the supernatant is mixed with 60 ml of acetone. Finally, overnight incubation at -20°C is followed by recovery of the aggregate to yield a [³H]acetyl-labeled histone. For the method of obtaining a [³H]acetyl-labeled histone, see, for example, International Patent Publication No. WO00/08048, Yoshida, M. et al., J. Biol. Chem., 265: 17174-17179 (1990) and the like. The [³H]acetyl-labeled histone thus obtained may be used in solution in an appropriate buffer and immediately used as is, or if it is not used immediately, it may be stored under freezing or refrigeration in a solution state, or may be stored under freezing in a dry solid state.

To "express each gene" can be performed by incorporating the gene to an expression vector having a promoter sequence, transferring the expression vector incorporating the gene to cells, and cultivating the cells, and the like. The promoter, expression vector and the like used differ variously depending on the host cells used for expression, and those skilled in the art having ordinary expertise can easily find the best combination in commercially available products. Alternatively, to "express each gene" can also be performed by transferring the gene to an expression vector having a promoter sequence, such as the SP6 phage or the T7 phage, and allowing the reaction in vitro by the in vitro transcription/translation method. Those skilled in the art having ordinary experimental knowledge can easily synthesize the transcription product and protein of each of the genes by this method.

As the method of transferring a gene used in the present invention to cells, a method of transformation known in the art, such as the calcium phosphate method, the liposome method, the lipofection method, the electroporation method and the like, can be used, which are not to be construed as limiting.

The cells to which a gene used in the present invention is transferred are not subject to limitation unless otherwise specified, as long as they are cells wherein a gene inserted in an expression vector is expressed, and mammalian cells including the above-described human cells, insect cells, yeast, *Escherichia coli* and the like can be used.

An "enzyme solution" refers to a fraction having HDAC enzyme activity extracted from a cell disruption solution or reaction solution having HDAC enzyme activity, wherein the HDAC enzyme may have been partially purified or purified to high purity, as long as the HDAC enzyme has been purified to the extent that enables a measurement of HDAC enzyme activity. A cell disruption solution having HDAC enzyme activity can be obtained from cells that are naturally expressing an HDAC enzyme, or cells wherein each HDAC gene is overexpressed. An enzyme solution can be obtained from cells that are naturally expressing an HDAC enzyme by, for example, cultivating the cells, and extracting a fraction having HDAC enzyme activity from a disruption solution of the cultured cells. Also, an enzyme solution can be obtained from cells allowed to overexpress each HDAC gene by, for example, inserting each HDAC gene in an expression vector, incorporating the expression vector to cells, cultivating the cells obtained, and extracting a fraction having HDAC enzyme activity from a disruption solution of the cultured cells. On the other hand, a reaction solution having HDAC enzyme activity can be prepared by, for example, synthesizing the transcription product and protein of each HDAC from each HDAC gene joined to an appropriate vector using the in vitro transcription/in vitro translation method.

"Partial purification" of an HDAC enzyme refers to purifying the HDAC enzyme to the extent that enables a measurement of HDAC enzyme activity, and can be conducted by a method known in the art, for example, a method described in International Patent Publication No. WO00/08048, Yoshida, M. et al., J. Biol. Chem. 265: p.17174-17179 (1990), and the like. Also, the partially purified HDAC enzyme can be purified to higher purity by another method of purification known in the art. For this partial purification, the following method of purification, for example, can be used. First, 5×10⁸ Jurkat cells are suspended in 40 ml of HDA buffer (composition: 15mM potassium phosphate, 5% glycerol and 0.2mM EDTA, pH 7.5). Subsequently, the suspension is homogenized, then nuclei are recovered via centrifugation (35,000 x g, 10 minutes), and the nuclei obtained are homogenized in 20 ml of an HDA buffer supplemented with 1M (NH₄)₂SO₄. Subsequently, the homogenate is subjected to sonication and clarified by centrifugation (35,000 x g, 10 minutes). Subsequently, deacetylase is precipitated by raising the (NH₄)₂SO₄ concentration to 3.5M. Subsequently, the precipitated protein is dissolved in 10 ml of HDA buffer, and this solution is dialyzed in 4 L of HDA buffer. After dialysis, the sample solution is loaded to a DEAE-cellulose (Whatman DE52) column (25×85mm), previously equilibrated with HDA buffer, and eluted with 300 ml of NaCl solution on a linear gradient (0 to 0.6M). As a result, HDAC enzyme activity appears as a single peak in the fraction eluted at 0.3 to 0.4M NaCl.

HDAC enzyme activity is measured by a method known in the art. For example, when a [³H]acetyl-labeled histone is used as a substrate, 10 µl of the [³H]acetyl-labeled histone is added to 90 µl of enzyme solution, and this mixture is incubated at 25°C for 30 minutes. The reaction is stopped by the addition of 10 µl of hydrochloric acid, and the released [³H] acetic acid is extracted with 1 ml of ethyl acetate. Finally, 0.9 ml of the solvent layer is transferred to a toluene scintillation solution, and the radioactivity of this solution is measured, thereby HDAC enzyme activity can be determined. When the HDAC enzyme activity of each enzyme solution is measured in the presence of a test substance, the test substance may be added around the time of preparation of a mixture of 10 µl of the [³H]acetyl-labeled histone and 90 µl of the enzyme solution, and the reaction may be initiated. For details about the measurement of HDAC enzyme activity, see, for example, International Patent Publication No. WO00/08048; Yoshida, M. et al., J. Biol. Chem., 265: 17174-17179 (1990) and the like, when a [³H] acetyl-labeled histone is used as a substrate, and see, for example, International Patent Publication No. WO01/040506 and the like, when a fluorescently labeled acetylated peptide is used as a substrate.

In the above-described method, a test substance that selectively suppresses HDAC enzyme activity only when using an enzyme solution obtained by expressing the HDAC4 gene or the HDAC8 gene is preferably selected, and a test substance that suppresses the HDAC enzyme activity of HDAC4 and/or HDAC8 more than suppresses the HDAC enzyme activity of HDAC1-3 and HDAC5-7 may be selected. More preferably, a test substance that does not suppress or substantially does not suppress the HDAC enzyme activity of HDAC1-3 and HDAC5-7, and that suppresses the HDAC enzyme activity of HDAC4 and/or HDAC8, is selected.

The present invention also relates to a method of selecting an IL-2 production inhibitor and/or an immunocyte proliferation inhibitor, each of which having low GATA-1 production inhibitory activity, or an immunosuppressant having low thrombocytopenic activity, which comprises selecting an inhibitor that specifically inhibits the formation of a complex of HDAC4 and N-CoR from among test HDAC inhibitors. For example, this method comprises (i) expressing the HDAC4 gene and the N-CoR gene to obtain an enzyme solution, then (ii) allowing the enzyme solution to coexist with a test HDAC inhibitor, and (iii) determining whether or not a complex of HDAC4 and N-CoR is formed in the mixture of enzyme solution and test HDAC inhibitor obtained in (ii). In this method, an enzyme solution obtained by expressing the HDAC3 gene, in addition to the HDAC4 gene and the N-CoR gene, may also be used. In this case, whether or not a higher complex is formed between HDAC4 and N-CoR and HDAC3 in the presence of a test HDAC inhibitor is determined. In (iii) in the above-described method, as examples of the method of determining whether or not a complex is formed, a method known in the art such as an analytical method using surface plasmon resonance, an analytical method using an antibody (for example, Western blotting) and the like can be mentioned.

As the labeled substance used to label the secondary antibody in an analytical method using an antibody, for example, Western blotting, an enzyme such as horseradish peroxidase and alkaline phosphatase, a fluorescent substance such as fluorescein isocyanate and rhodamine, a radioactive substance such as ³²P or ¹²⁵I, a chemical luminescent substance and the like can be mentioned.

To select an IL-2 production inhibitor and/or an immunocyte proliferation inhibitor, each of which having low GATA-1 production inhibitory activity, or an immunosuppressant having low thrombocytopenic activity, that specifically inhibits the formation of a complex of HDAC4 and N-CoR, a method based on a two-hybrid system known in the art can also be used. For example, when a two-hybrid system is used for this purpose, a test substance that specifically inhibits the formation of a complex of an HDAC4 fusion protein comprising at least a portion of the HDAC4 protein and an N-CoR fusion protein comprising at least a portion of the N-CoR protein is selected by (i) expressing a gene that encodes the HDAC4 fusion protein and a gene that encodes the N-CoR fusion protein in cells, and (ii) determining whether or not a test substance (preferably a test HDAC inhibitor) inhibits the formation of a complex of the HDAC4 fusion protein and the N-CoR fusion protein.

The HDAC4 fusion protein may be any one comprising a region that interacts with N-CoR in the HDAC4 protein, and may also comprise the entire HDAC4 protein. Likewise, the N-CoR fusion protein may be any one comprising a region that interacts with HDAC4 in the N-CoR protein (for example, RD3 described below), and may also comprise the entire N-CoR protein. Each of the HDAC4 fusion protein and the N-CoR fusion protein further comprises, in addition to a portion or the entire region of the HDAC4 protein and the N-CoR protein, a DNA-binding domain (for example, GAL4 DNA-binding domain and the like) or a transcription activation domain (for example, VP16 activation domain and the like) derived from transcription regulatory factor. However, if the HDAC4 fusion protein comprises a DNA-binding domain, the N-CoR fusion protein need to comprise a transcription activation domain; if the HDAC4 fusion protein comprises a transcription activation domain, the N-CoR fusion protein need to comprise a DNA-binding domain.

Whether or not a complex is formed between the two fusion proteins can be confirmed by the expression level of a gene located downstream of the promoter to which the DNA-binding region in a fusion protein binds. Accordingly, when a complex is formed between the fusion proteins, the expression level of the mRNA or protein encoded by the gene, and the expression level of a reporter gene comprising a promoter located upstream of the gene, increase, whereas when a complex is not formed between the fusion proteins, the expression level of the mRNA or protein encoded by the gene, and the expression level of the above-described reporter gene comprising a promoter remain unchanged.

Whether or not a test substance (preferably a test HDAC inhibitor) inhibits the formation of a complex of an HDAC4 fusion protein and an N-CoR fusion protein can be determined by determining whether or not the expression level of a gene located downstream of the promoter to which the DNA-binding region in a fusion protein binds is decreased in the presence of the test substance. Accordingly, (a) the expression level of the mRNA or protein encoded by the gene or the expression level of a reporter gene comprising a promoter located upstream of the gene is measured in the absence of a test substance, (b) the expression level of the mRNA, protein or reporter gene is measured in the presence of the test substance, and (c) comparing the expression level measured in (a) and the expression level measured in (b); if the expression level measured in (b) is lower than the expression level measured in (a), the test substance is selected as a substance that inhibits the formation of a complex of an HDAC4 fusion protein and an N-CoR fusion protein. It is preferable, from the viewpoint of assay simplicity, sensitivity and the like, that whether or not the test substance inhibits the formation of a complex of an HDAC4 fusion protein and an N-CoR fusion protein be evaluated by measuring the expression level of a reporter gene.

The present invention also relates to a method of selecting a compound having low GATA-1 production inhibitory activity, and having IL-2 production inhibitory activity and/or immunocyte proliferation inhibitory activity, or a compound having low thrombocytopenic activity and having immunosuppressive activity, which comprises selecting a compound that suppresses the expression level of HDAC4 and/or HDAC8 from among test HDAC inhibitors. This method comprises (i) allowing cells that express HDAC4 and/or HDAC8 to coexist with a test HDAC inhibitor, and (ii) measuring the HDAC4 and/or HDAC8 expression level in the cells. Specifically, this method can be conducted in the same manner as the method described below of measuring the expression level of GATA-1 mRNA, GATA-1 protein or GATA-1 reporter gene.

The present invention also relates to a method of selecting a compound having low GATA-1 production inhibitory activity and having IL-2 production inhibitory activity, or a compound having low thrombocytopenic activity and having immunosuppressive activity, wherein an HDAC inhibitor that selectively inhibits the binding of HDAC4 and/or HDAC8 and an HDAC4- and/or HDAC8-specific ligand is selected from among test HDAC inhibitors. This method comprises (i) expressing the HDAC4 and/or the HDAC8 gene to obtain an enzyme solution, and then (ii) measuring the binding activity of HDAC4 and/or HDAC8 enzyme and the HDAC4- and/or HDAC8-specific ligand for the enzyme solution in the presence of an HDAC4- and/or HDAC8-specific ligand and a test HDAC inhibitor.

An "HDAC4- and/or HDAC8-specific ligand" refers to a substance showing stronger binding activity to HDAC4 and/or HDAC8 than to HDAC1-3 and HDAC5-7, out of substances that specifically bind to HDAC4 and/or HDAC8. These substances include, but are not limited to, substances that promote the activity of each enzyme, those that inhibit the same, and the like. As "the HDAC4- and/or HDAC8-specific ligand", a low-molecular substance that shows weak binding activity to HDAC1-3 and HDAC5-7 or is substantially free from binding activity, and that shows strong binding activity to HDAC4 and/or HDAC8, is preferred.

In step (ii) in the above-described method, an "HDAC4- and/or HDAC8-specific ligand" is preferably used, but an "HDAC4- and/or HDAC8-nonspecific ligand" can also be used. An "HDAC4- and/or HDAC8-nonspecific ligand" refers to a substance that shows stronger binding activity to HDACl-3 and HDAC5-7 than to HDAC4 and/or HDAC8, or a substance that shows nearly equivalent binding activity to HDAC4 and/or HDAC8 and to HDAC1-3 and HDAC5-7. In the present invention, an "HDAC4- and/or HDAC8-specific ligand" and an "HDAC4- and/or HDAC8- nonspecific ligand" are also integrally referred to as an "HDAC4 and/or HDAC8 ligand".

In step (ii) in the above-described method, the binding activity of HDAC4 and/or HDAC8 enzyme and an HDAC4- and/or HDAC8-specific ligand is measured in the presence of a test HDAC inhibitor. Binding activity can be measured using, for example, a method known in the art, such as binding assay or mass analysis. By the above-described method, a substance that competitively inhibits an HDAC4- and/or HDAC8-specific ligand can be obtained.

In the present invention, human-derived HDAC4 is preferably used as HDAC4. Specifically, HDAC4 refers to, for example, a protein that comprises the amino acid sequence shown by SEQ ID NO:2 or an analogue thereof that has HDAC enzyme activity (for example, protein encoded by the base sequence of GenBank accession number AF132607). HDAC4 analogues include a variant having one or two or more amino acids substituted, deleted or added in the amino acid sequence shown by SEQ ID NO:2, a sugar chain added form and the like. As the HDAC4 analogue, a protein that comprises an amino acid sequence having a homology of 70% or more, preferably 80% or more, more preferably 90% or more, still more preferably 95% or more, to the amino acid sequence shown by SEQ ID NO:2, can also be used. The degree of amino acid sequence homology can be determined by, for example, using BLAST in the default settings between the amino acid sequence of interest and a reference amino acid sequence in NCBI (The National Center for Biotechnology Information). For details about HDAC4, see, for example, International Patent Publication No. WO00/10583.

Likewise, in the present invention, human-derived HDAC8 is preferably used as HDAC8. Specifically, HDAC8 refers to, for example, a protein that comprises the amino acid sequence shown by SEQ ID NO:8, or an analogue thereof that has HDAC enzyme activity (for example, protein encoded by the base sequence of GenBank accession number HSA277724). HDAC8 analogues include a variant having one or two or more amino acids substituted, deleted or added in the amino acid sequence shown by SEQ ID NO:8, a sugar chain added form and the like. As the HDAC8 analogue, a protein that comprises an amino acid sequence having a homology of 70% or more, preferably 80% or more, more preferably 90% or more, still more preferably 95% or more, to the amino acid sequence shown by SEQ ID NO:8 can also be used. For details about HDAC8, see, for example, Joseph J. BUGGY et al., Biochemical Journal 350: 199-205 (2000).

In the present invention, human-derived N-CoR is preferably used as N-CoR. Specifically, N-CoR refers to, for example, a protein that comprises the amino acid sequence shown by SEQ ID NO:22, or an analogue thereof that is capable of forming a complex with HDAC4 or HDAC3 (for example, protein encoded by the base sequence of GenBank accession number AF044209). N-CoR analogues include a variant having one or two or more amino acids substituted, deleted or added in the amino acid sequence shown by SEQ ID NO:22, a sugar chain added form and the like. As the N-CoR analogue, a protein that comprises an amino acid sequence having a homology of 70% or more, preferably 80% or more, more preferably 90% or more, still more preferably 95% or more, to the amino acid sequence shown by SEQ ID NO:22 can also be used. In the present invention, N-CoR may be any one, as long as it is capable of forming a complex with HDAC4 (both HDAC4 and HDAC3 as required). As such N-CoR, the N-CoR variants disclosed in, for example, Matthew G. Guenther et al., Molecular and Cellular Biology, 21(18): 6091-6101 (2001) and elsewhere, and the like can also be used, in addition to those mentioned above.

In the present invention, human-derived HDAC3 is preferably used as HDAC3. Specifically, HDAC3 refers to, for example, a protein that comprises the amino acid sequence shown by SEQ ID NO:16, or an analogue thereof that has HDAC enzyme activity or is capable of binding to HDAC4 (HDAC4 - N-CoR complex as required) (for example, protein encoded by the base sequence of GenBank accession number U66914). HDAC3 analogues include a variant having one or two or more amino acids substituted, deleted or added in the amino acid sequence shown by SEQ ID NO:16, a sugar chain added form and the like. As the HDAC3 analogue, a protein that comprises an amino acid sequence having a homology of 70% or more, preferably 80% or more, more preferably 90% or more, still more preferably 95% or more, to the amino acid sequence shown by SEQ ID NO:16 can also be used. In the analysis of a complex of HDAC3 and N-CoR (a higher complex of HDAC3 and HDAC4 - N-CoR complex as required), HDAC3 need not always have HDAC enzyme activity, as long as it retains the capability of binding to HDAC4 (HDAC4 - N-CoR complex as required). For details about HDAC3, see, for example, Molecular Cell, 9: 45-57 (January 2002), Matthew G. Guenther et al., Molecular and Cellular Biology, 21(18): 6091-6101 (2001) and the like.

Furthermore, in the present invention, human-derived HDAC1-2 and HDAC5-7 are preferably used as HDAC1-2 and HDAC5-7. Specifically, each of HDAC1-2 and HDAC5-7 is a protein encoded by the base sequence of GenBank accession number D50405 (HDAC1), the base sequence of GenBank accession number HSU31814 (HDAC2), the base sequence of GenBank accession number AF132608 (HDAC5), the base sequence of GenBank accession number AF132609 (HDAC6), or the base sequence of GenBank accession number AF239243 (HDAC7), or an analogue thereof that has HDAC enzyme activity. HDAC1-2 and HDAC5-7 analogues include a variant having one or two or more amino acids substituted, deleted or added in the amino acid sequence corresponding to each of the above-described HDAC1-2, HDAC5-7, a sugar chain added form and the like. As the HDAC1-2 and HDAC5-7 analogues, a protein that comprises an amino acid sequence having a homology of 70% or more, preferably 80% or more, more preferably 90% or more, still more preferably 95% or more, to the amino acid sequence corresponding to each of the above-described HDAC1-2 and HDAC5-7, can also be used

Furthermore, the present invention relates to a method of selecting a selective HDAC4 and/or HDAC8 inhibitor having low thrombocytopenic activity by further confirming the degree of GATA-1 production inhibitory activity of an immunosuppressant and/or an immunocyte proliferation inhibitor, each of which having low thrombocytopenic activity selected as described above. Although the degree of GATA-1 production inhibitory activity can be evaluated by, for example, measuring the expression level of GATA-1 mRNA, GATA-1 protein or GATA-1 reporter gene in a cell such as cell line or primary culture cells that are naturally expressing GATA-1, in the presence and/or absence of an immunosuppressant having low thrombocytopenic activity, it is preferable, from the viewpoint of assay simplicity, sensitivity and the like, that the expression level of the GATA-1 reporter gene be measured.

In the above-described method, as the test cells used to measure GATA-1 production inhibitory activity, a cell line, primary culture cells and the like that are naturally expressing GATA-1 can be used. Whether or not a particular cell line or primary culture cells are naturally expressing GATA-1 can be determined by confirming the expression of GATA-1 mRNA or protein in these cells by a method known in the art. Also, as the test cells used in the above-described method, cells that substantially reflect the state of megakaryocytic lineage cell are also preferred. The cell line that substantially reflects the state of megakaryocytic lineage cell is not subject to limitation, as long as it is derived from megakaryocytic lineage cell; for example, human-derived HEL cells (accession number JCRB0062, Japanese Collection of Research Bioresources) can be used.

When the GATA-1 production inhibitory activity of an HDAC4 and/or HDAC8 inhibitor is evaluated by the expression level of GATA-1 mRNA or GATA-1 protein, test cells can be allowed to coexist with the HDAC4 and/or HDAC8 inhibitor by adding the HDAC4 and/or HDAC8 inhibitor to a cell culture, and cultivating the cells.

When the GATA-1 production inhibitory activity of an HDAC4 and/or HDAC8 inhibitor is evaluated by a reporter assay using a GATA-1 reporter gene, test cells can be allowed to coexist with the HDAC4 and/or HDAC8 inhibitor by adding the HDAC4 and/or HDAC8 inhibitor to a cell culture, around the time of transferring the GATA-1 reporter gene to the cells by a transfection method such as electroporation. Preferably, the GATA-1 reporter gene is transferred, then the HDAC4 and/or HDAC8 inhibitor is added to the culture, and further cultivate the cells.

The expression level of GATA-1 mRNA can be confirmed by, for example, preparing each total RNA from test cells, and comparing the expression level of GATA-1 mRNA by quantitative RT-PCR and the like known in the art, such as competitive RT-PCR or real-time RT-PCR and the like.

The expression level of GATA-1 protein can be measured by, for example, preparing an extract from cells allowed to exist in the presence and absence of an HDAC4 and/or HDAC8 inhibitor, and using an anti-GATA-1 antibody and a labeled secondary antibody. As the method for this measurement, various methods in use for general immunochemical assays, such as radioisotope immunoassay (RIA), ELISA [Engvall, E., Methods in Enzymol., 70, 419-439 (1980)], fluorescent antibody method, plaque method, spot method, agglutination method, and Ouchterlony method ("Hybridoma Method and Monoclonal Antibodies", published by R&D Planning, pp. 30-53, March 5, 1982) can be used, and these methods can be easily performed by those skilled in the art. Although one of the above-described methods can be appropriately selected from various viewpoints, ELISA is preferred from the viewpoint of sensitivity, ease of operation and the like. As the labeling substance used to label the above-described secondary antibody, the same as those mentioned above can be used.

The expression level of a GATA-1 reporter gene can be evaluated by a reporter assay using the GATA-1 reporter gene. The "GATA-1 reporter gene" refers to a DNA construct prepared by artificially joining the transcription control region of the GATA-1 gene and a reporter gene. As the transcription control region of the GATA-1 gene, a DNA fragment comprising both the IE promoter region of the human GATA-1 gene (GenBank accession number: AF196971) and the HSI region (highly-sensitive-to-DNase I region) located at about 3.5 kb apart upstream thereof is used. Regarding the scope of transcription control region essential to the objects of the present invention, the region is desirably one that substantially reflects the mode of transcription control of the natural GATA-1 gene in megakaryocyte lineage cells, rather than simply retains transcriptional activity.

For promoters of the GATA-1 gene, at least two kinds, i.e., the IE promoter and the IT promoter, are known to exist. In relation to the thrombocytopenic activity of HDAC inhibitors, because the function of the IE promoter, which has been shown to be closely associated with transcription control in megakaryocyte lineage cells, is stipulated to be important, its control region is preferably used.

For the transcription control by the IE promoter, both a sequence located within about 0.7 kb just upstream of the transcription initiation site and the HSI region located further upstream thereof (highly-sensitive-to-DNase I region) are important. On the 5'-side of the HSI region, two CACC sites are present, and apart therefrom by about 50 base pairs, a GATA binding site and the E-box motif are adjacent to each other by a distance of about 10 base pairs. It is known that if a mutation that destroys the GATA-binding site is introduced, erythrocyte- or megakaryocyte-specific expression is no longer observed (P. Vyas et al., Development 126; 2799-2811 (1999)). Also, further on the 3'-side of this GATA-E-box motif, the CTGTGGCCACAG sequence (SEQ ID NO:86), which is a characteristic palindrome sequence, and a GC-rich region are present.
In the GC-rich region, the GGAA sequence, which is seen in the binding site of the transcription factor ETS, is present. Even if the CACC site on the 5'-side is deleted from the GATA-1 HSI region comprising these sequences, erythrocyte- or megakaryocyte-specific expression is seen, but if the GATA-E-box motif is also deleted, this expression disappears. Regarding the 3'-side, it has been reported that the GATA-1 HSI region can function as an enhancer, provided that up to about 250 base pairs downstream of the E-box are included. Therefore, provided that all the essential sequence sites described above are contained, the sequence may actually be of any length; for example, a sequence prepared by artificially joining a region comprising 819 base pairs in the vicinity of the transcription initiation site by the IE promoter (-789 to +30: transcription initiation site taken as +1) of the human the GATA-1 gene, and a region comprising 637 base pairs (-3769 to -3133) as the HIS region upstream of the transcription initiation site, can be used.

For the essential sequence sites such as the GATA-E-box motif, it is possible to prepare an artificial DNA construct wherein the essential sequences are multiplied tandem in a plurality of sets by an ordinary technique of recombinant DNA experiments. In some cases, the transcription induction activity of the transcription control region can be enhanced by using such an artificial construct in place of the natural sequence.

Note that a "reporter gene" refers to a gene that comprises a structural gene region that encodes a specific protein serving as an index of gene expression and a downstream untranslated region (3'-non-coding region), and that lacks all the transcription control region originally present on the upstream side of the structural gene. The structural gene region that encodes a specific protein serving as an index of gene expression may be any one, as long as the gene expression from the transcription control region in an exogenous gene fragment can easily be measured from the function of the index protein encoded by the reporter gene by artificially joining the structural gene region and the exogenous gene fragment, and transferring the structural gene region and exogenous gene fragment to cells. More preferably, the index protein is desirably one that stably occurs in ordinary mammalian cells, and that has no endogenous protein having similar activity in the cells, or has such an endogenous protein that can be measured with easy distinguishment. More preferably, the mRNA that encodes the index protein is desirably one that stably occurs in cells. Also, the substrate for the index protein is desirably one that need not be newly added, or can easily be incorporated in cells in a sufficient amount if need to be added, in measuring the activity. The assay system composed thereof desirably has both a broad range of linearity and high sensitivity. It is possible to use the firefly-derived luciferase gene, the Renilla-derived luciferase gene, the alkaline phosphatase gene, the β-galactosidase gene, the green fluorescent protein (GFP) gene, the enhanced green fluorescent protein (EGFP) gene, the β-glucuronidase gene, the chloramphenicol acetyltransferase (CAT) gene, the horseradish peroxidase (HRP) gene and the like. More preferably, the firefly-derived luciferase gene is used. For the firefly-derived luciferase gene, the detection sensitivity of the measurement system can be increased by using an improved type of the luciferase gene (luc+). For the untranslated region downstream of the structural gene, a sequence derived from the SV40 virus genome late gene, for example, can be used.

Each of the above-described GATA-1 reporter genes is joined to a cloning vector and amplified with *Escherichia coli* as a host. The cloning vector used is not subject to limitation, as long as it has a replication origin amplifiable in *Escherichia coli* and a selection marker; for example, using pGL3-Basic (Promega Corporation), which encodes an ampicillin resistance marker and an improved type of the luciferase structural gene (luc+) and the like, the above-described DNA construct can easily be prepared. Subsequently, the amplified vector is transiently transferred to cells and reporter activity is measured.

The present invention also relates to a kit for the selection of an immunosuppressant having low thrombocytopenic activity, or an IL-2 production inhibitor and/or an immunocyte proliferation inhibitor, each of which having low GATA-1 production inhibitory activity and the like, which includes all or part of the above-described components necessary to perform the above-described method. As an example of the assay kit for the selection of an immunosuppressant having low thrombocytopenic activity comprising a selective HDAC4 and/or HDAC8 inhibitor, a kit including (i) enzyme solutions obtained by expressing the HDAC1, HDAC2, HDAC3, HDAC5, HDAC6 and HDAC7 genes, respectively, or (ii) an enzyme solution obtained by expressing each of the HDAC4 and/or HDAC8 gene can be mentioned. Furthermore, the above-described kit may comprise other components, an instruction manual and the like.

The present invention encompasses a compound such as an immunosuppressant having low thrombocytopenic activity, or an IL-2 production inhibitor and/or an immunocyte proliferation inhibitor, each of which having low GATA-1 production inhibitory activity, obtained by the above-described method, or using the above-described kit.

Furthermore, the compound obtained by the above-described method, or using the above-described kit, is useful as an immunosuppressant having low thrombocytopenic activity, an IL-2 production inhibitor having low GATA-1 production inhibitory activity, and a selective HDAC4 and/or HDAC8 inhibitor having low thrombocytopenic activity, in the treatment or prophylaxis of diseases such as inflammatory diseases, diabetes, diabetic complications, homozygous thalassemia, fibrosis, liver cirrhosis, acute promyelocytic leukemia, protozoan infections, graft rejection during the transplantation of organs or tissues such as heart, kidney, liver, bone marrow, skin, cornea, lung, pancreas, small intestine, hand and foot, muscle, nerve, intervertebral disc, trachea, myeloblasts, and cartilage, graft-versus-host reactions in bone marrow transplantation, autoimmune diseases, cancers and the like. The present invention relates to a therapeutic and/or prophylactic pharmaceutical for the above-described diseases, which comprises such a compound as an active ingredient, and a therapeutic and/or prophylactic method for the above-described diseases, which comprises administering an effective amount of such a compound.

When the compound obtained by the above-described method, or using the above-described kit, is used as a pharmaceutical, it can be used as a pharmaceutical as is, and can also be used after being prepared as a formulation by a publicly known method of pharmaceutical making. For example, it can be used in the form of a pharmaceutical formulation which is appropriately combined with a publicly known pharmacologically acceptable carrier or medium, specifically distilled water, saline, vegetable oil, emulsifier, suspending agent and the like, for example, a solid, semi-solid or liquid formulation (for example, tablets, pills, troches, capsules, suppositories, creams, ointments, aerosols, powders, solutions, emulsions, suspensions and the like).

Regarding administration to patients, the pharmaceutical formulation of the present invention is suitable for nasal, ophthalmic, external (topical), rectal, pulmonary (nasal or intraoral injection), oral or non-oral (including subcutaneous, intravenous and intramuscular) administration or inhalation. Administration of an injection can be conducted by a publicly known method, for example, intra-arterial injection, intravenous injection, subcutaneous injection and the like. Dosage is variable depending on body weight, age and symptoms of patient, and the method of administration used and the like, and those skilled in the art can appropriately select the appropriate dosage.

The present invention also relates to a variant that lacks the function of normal HDAC4 enzyme (i.e., an HDAC4 dominant negative variant). As an example of the HDAC4 dominant negative variant, an HDAC4 variant that has (consists of) the amino acid sequence shown by SEQ ID NO:4 or SEQ ID NO:6 can be mentioned. Furthermore, the present invention also encompasses a variant that has one or more amino acids deleted, substituted or added in the amino acid sequence shown by SEQ ID NO:4 or SEQ ID NO:6, and that lacks the function of normal HDAC4 enzyme.

Furthermore, the present invention relates to a DNA that has (consists of) the base sequence that encodes the above-described HDAC4 dominant negative variant. More specifically, the present invention relates to a DNA that has (consists of) the base sequence shown by SEQ ID NO:3 or SEQ ID NO:5. The present invention also encompasses a DNA that has the base sequence that encodes a variant that has one or more amino acids deleted, substituted or added in the amino acid sequence shown by SEQ ID NO:4 or SEQ ID NO:6, and that lacks the function of normal HDAC4 enzyme.

The present invention also relates to a variant that lacks the function of normal HDAC1 enzyme (i.e., an HDAC1 dominant negative variant). As an example of the HDAC1 dominant negative variant, an HDAC1 variant that has (consists of) the amino acid sequence shown by SEQ ID NO:12 or SEQ ID NO:14 can be mentioned. Furthermore, the present invention also encompasses a variant that has one or more amino acids deleted, substituted or added in the amino acid sequence shown by SEQ ID NO:12 or SEQ ID NO:14, and that lacks the function of normal HDAC1 enzyme.

Furthermore, the present invention relates to a DNA that has (consists of) the base sequence that encodes the above-described HDAC1 dominant negative variant. More specifically, the present invention relates to a DNA that has (consists of) the base sequence shown by SEQ ID NO:11 or SEQ ID NO:13. The present invention also encompasses a DNA that has the base sequence that encodes a variant that has one or more amino acids deleted, substituted or added in the amino acid sequence shown by SEQ ID NO:12 or SEQ ID NO:14, and that lacks the function of normal HDAC1 enzyme.

The present invention also relates to a variant that lacks the function of normal HDAC3 enzyme (i.e., an HDAC3 dominant negative variant). As an example of the HDAC3 dominant negative variant, an HDAC3 variant that has (consists of) the amino acid sequence shown by SEQ ID NO:18 or SEQ ID NO:20, can be mentioned. Furthermore, the present invention also encompasses a variant that has one or more amino acids deleted, substituted or added in the amino acid sequence shown by SEQ ID NO:18 or SEQ ID NO:20, and that lacks the function of normal HDAC3 enzyme.

Furthermore, the present invention relates to a DNA that has (consists of) the base sequence that encodes the above-described HDAC3 dominant negative variant. More specifically, the present invention relates to a DNA that has (consists of) the base sequence shown by SEQ ID NO:17 or SEQ ID NO:19. The present invention also encompasses a DNA that has the base sequence that encodes a variant that has one or more amino acids deleted, substituted or added in the amino acid sequence shown by SEQ ID NO:18 or SEQ ID NO:20, and that lacks the function of normal HDAC3 enzyme.

HDAC1, HDAC3 and HDAC4 dominant negative variants, enzyme solutions containing these variants, and DNAs that encode these variants can, for example, be used as negative controls in the above-described screening. The kit for performing the screening method of the present invention can also include these HDAC1, HDAC3 and HDAC4 dominant negative variants, enzyme solutions containing these variants, or DNAs that encode these variants.

### EXAMPLES

The present invention is hereinafter described in more detail by means of the following Examples, which examples, however, are not to be construed as limiting the scope of the present invention.

### Example 1: Construction of reporter gene plasmids for IL-2 reporter gene assay

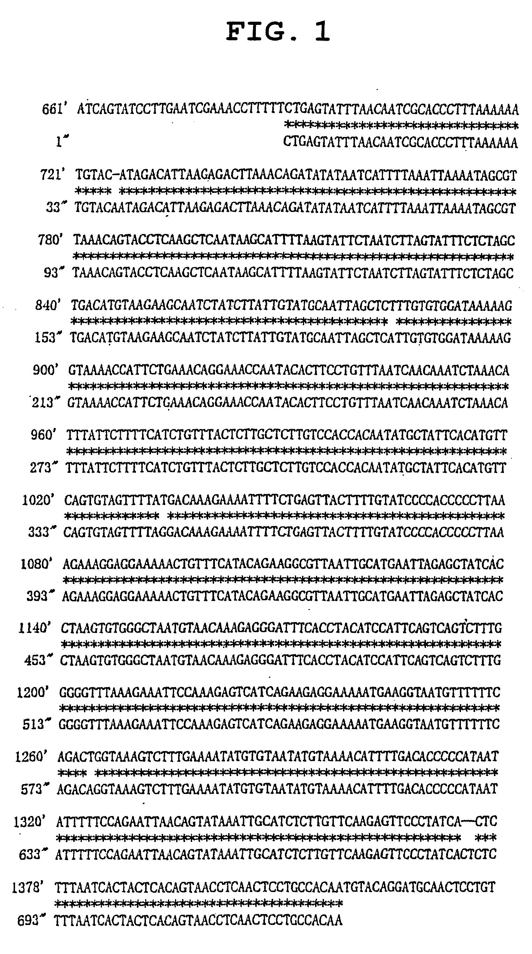

A 728-basepair fragment corresponding to the -674 to +54 region in the vicinity of the transcription initiation site of the human IL-2 gene was obtained by the PCR method with a genomic DNA isolated from Jurkat cells derived from human T cell (ATCC, TIB-152) as a template. PCR was performed using the primers shown by SEQ ID NO:27 and SEQ ID NO:28 below. These primers were designed on the basis of the human IL-2 gene sequence listed as Locus code: HSIL05 in the gene database GenBank. Additionally, a restriction endonuclease recognition site was added to the end of each primer to insert to a vector for reporter gene assay. The 728-basepair fragment obtained has an NheI recognition site upstream of the promoter, and a HindIII recognition site downstream of the promoter. The amplified 728-basepair fragment was inserted to the cloning vector pCR4 (manufactured by Invitrogen). Subsequently, the base sequence of the inserted region in the plasmid obtained (SEQ ID NO:23) was confirmed. As a result, the base sequence of the inserted region (SEQ ID NO:23) was confirmed as having base substitution at three sites, insertion of two bases at one site, and insertion of one base at one site, as shown in FIG. 1, compared to the promoter sequence of IL-2 listed as GenBank Locus code: HSIL05 (SEQ ID NO:29). Subsequently, the plasmid obtained was cleaved at the NheI recognition site and the HindIII recognition site. The fragment obtained was inserted to the NheI/HindIII site of pGL3 basic, a vector for reporter gene assay comprising the firefly luciferase gene (manufactured by Promega Company). Thus, the plasmid pGL3 IL2 Pro was obtained, which has a 731-basepair sequence (SEQ ID NO:23) corresponding to the -675 to +56 region in the vicinity of the transcription initiation site of the human IL-2 gene upstream of the firefly luciferase gene.

TCGCTAGCCTGAGTATTTAACAATCGCACCCT (SEQ ID NO:27)

CGAAGCTTGTGGCAGGAGTTGAGGTTACTG (SEQ ID NO:28)

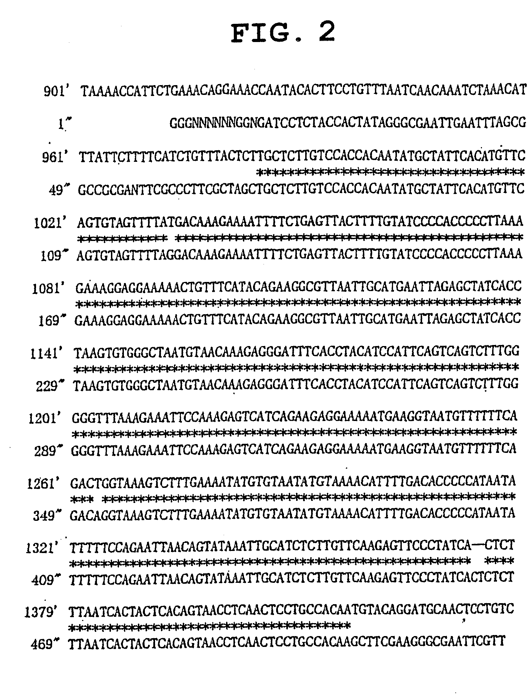

Subsequently, a 435-basepair fragment corresponding to the -379 to +56 region in the vicinity of the transcription initiation site of the human IL-2 gene was obtained by the PCR method with pGL3 IL2 Pro as a template. PCR was performed using the primer shown by SEQ ID NO:30 below and the primer shown by SEQ ID NO:28 above. The SEQ ID NO:30 primer was designed on the basis of the human IL-2 gene sequence inserted in pGL3 IL2 Pro. Additionally, a restriction endonuclease recognition site was added to the end of each primer to insert to a vector for reporter gene assay. The 435-basepair fragment obtained has an NheI recognition site upstream of the promoter, and a HindIII recognition site downstream of the promoter. The amplified 435-basepair fragment was inserted to the cloning vector pCR4. Subsequently, the base sequence of the inserted region in the plasmid obtained (SEQ ID NO:24) was confirmed. As a result, the base sequence of the inserted region (SEQ ID NO:24) was confirmed as having base substitution at two sites and insertion of two bases at one site as shown in FIG. 2 compared to the promoter sequence of IL-2 listed as GenBank Locus code: HSIL05 (SEQ ID NO:31). Subsequently, the plasmid obtained was cleaved at the NheI recognition site and the HindIII recognition site. The fragment obtained was inserted to the NheI/HindIII site of pGL3, a vector for reporter gene assay comprising the firefly luciferase gene. Thus, the plasmid pGL3 IL2 Pro43 was obtained, which has a 435-basepair promoter sequence corresponding to the -379 to +56 region in the vicinity of the transcription initiation site of the human IL-2 gene upstream of the firefly luciferase gene.

CGCTAGCTGCTCTTGTCCACCACAATATGC (SEQ ID NO:30)

### Example 2: Construction of reporter gene plasmids for GATA-1 reporter gene assay

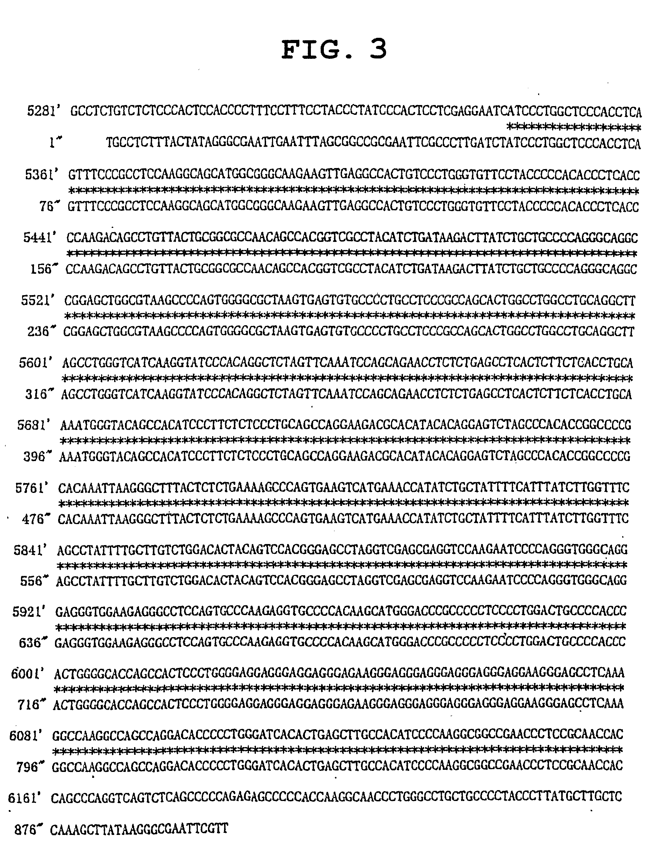

An 821-basepair fragment corresponding to the -789 to +32 region in the vicinity of the transcription initiation site of the human GATA-1 gene was obtained by the PCR method with human genomic DNA as a template. PCR was performed using the primers shown by SEQ ID NO:32 and SEQ ID NO:33 below. These primers were designed on the basis of the human GATA-1 gene sequence listed as gene database GenBank accession number AF196971. Additionally, a restriction endonuclease recognition site was added to the end of each primer to insert to a vector for reporter gene assay. The 821-basepair fragment obtained has a BglII recognition site upstream of the promoter, and a HindIII recognition site downstream of the promoter. The amplified 821-basepair fragment was inserted to the cloning vector pCR4. Subsequently, the base sequence of the inserted region in the plasmid obtained (SEQ ID NO:25) was confirmed. As a result, the base sequence of the inserted region (SEQ ID NO:25) was consistent with the promoter sequence of GATA-1 listed as GenBank accession number AF196971 (FIG. 3). Subsequently, the plasmid obtained was cleaved at the NheI recognition site and the HindIII recognition site. The fragment obtained was inserted to the NheI/HindIII site of pGL3 basic, a vector for reporter gene assay comprising the firefly luciferase gene. Thus, the plasmid pGL3-IE was obtained, which has an 821-basepair sequence corresponding to the -789 to +32 region of the promoter region of the GATA-1 gene upstream of the firefly luciferase gene.

ATAGATCTATCCCTGGCTCCCACCTCAG (SEQ ID NO:32)

ATAAGCTTTGGTGGTTGCGGAGGGTTCG (SEQ ID NO:33)

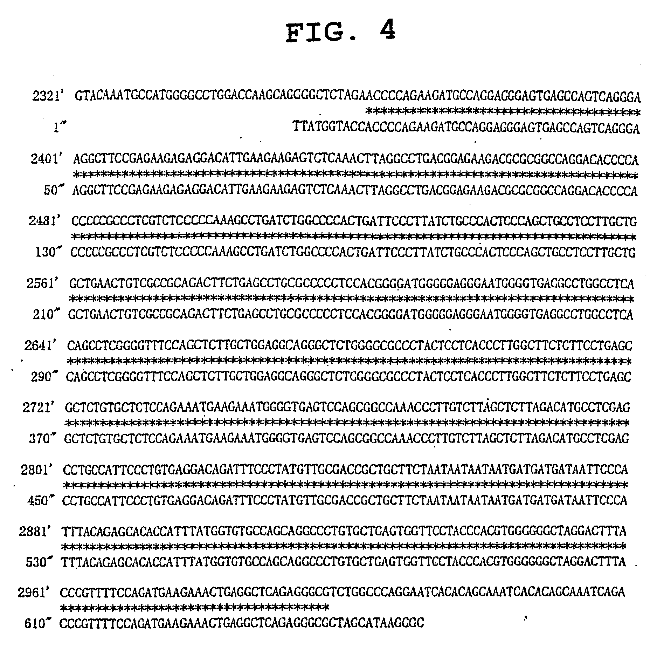

Subsequently, a 637-basepair fragment corresponding to the -3769 to -3133 region upstream of the transcription initiation site of human GATA-1 gene was obtained by the PCR method with human genomic DNA as a template. PCR was performed using the primers shown by SEQ ID NO:34 and SEQ ID NO:35 below. These primers were designed on the basis of the human GATA-1 gene sequence listed as gene database GenBank accession number AF196971. Additionally, a restriction endonuclease recognition site was added to the end of each primer to insert to a vector for reporter gene assay. The 637-basepair fragment obtained has a KpnI recognition site upstream of the promoter, and an NheI recognition site downstream of the promoter. The amplified 637-basepair fragment was inserted to the cloning vector pCR4. Subsequently, the base sequence of the inserted region in the plasmid obtained (SEQ ID NO:26) was confirmed. As a result, the base sequence of the inserted region (SEQ ID NO:26) was consistent with the promoter sequence of GATA-1 listed as GenBank accession number AF196971 (FIG. 4). Furthermore, the plasmid obtained was cleaved at the KpnI recognition site and the NheI recognition site. The fragment obtained was inserted to the KpnI/NheI site of the pGL3-IE promoter. Thus, the plasmid pGL3-HSI-IE Pro was obtained, which has a 637-basepair sequence corresponding to the -3769 to -3133 region upstream of the transcription initiation site of the GATA-1 gene, and an 821-basepair sequence corresponding to the -789 to +32 region, upstream of the firefly luciferase gene.

ATGGTACCACCCCAGAAGATGCCAGGAG (SEQ ID NO:34)

ATGCTAGCGCCCTCTGAGCCTCAGTTTC (SEQ ID NO:35)

### Example 3:

### 3.1. Construction of plasmids for subcloning of HDAC1 to 8

Using the templates and primers shown in Table 1, various full-length HDAC isozymes were amplified by PCR and once subcloned into pGEM-T (Promega). Subsequently, each of full-length HDAC1 to 6 was inserted to pBluescriptII KS(+) (TOYOBO) via treatment with a restriction endonuclease (EcoRI/NotI for HDAC1, BamHI/NotI for HDAC2, EcoRI/NotI for HDAC3, EcoRI/NotI for HDAC4, HindIII/NotI for HDAC5, HindIII/NotI for HDAC6). Each of full-length HDAC7 and 8 was subcloned into pUC18 (TaKaRa) (SmaI treatment). For details, see Table 1.

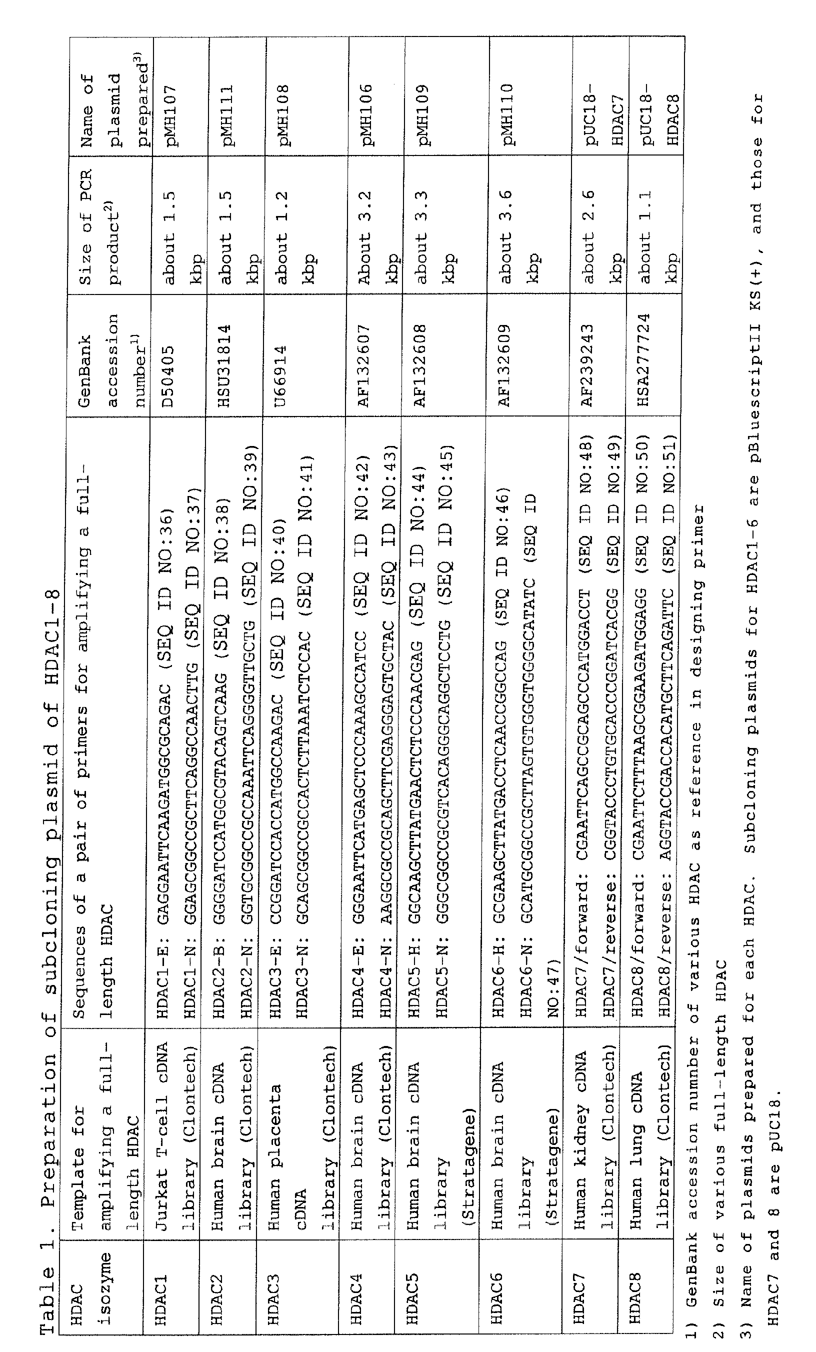

### 3.2. Construction of plasmids for expression of HDAC1 to 8

Subsequently, plasmids for expression having a Flag sequence added to the C-terminal of each of HDAC1 to 8 were prepared. Details are shown below.

### HDAC3

Full-length HDAC3 was amplified by PCR with pMH108 as a template using the following primers 108E (SEQ ID NO : 52) and 108KFN (SEQ ID NO : 53). Meanwhile, the primers were designed to add a Flag sequence (DYKDDDDK) (SEQ ID NO:54) to the C-terminal. The PCR fragment obtained was subcloned into pGEM-T (Promega) to yield pGEM-HDAC3-Flag (E108KFN). E108KFN was inserted to pEAK10 (Edge BioSystems) via EcoRI/NotI (about 1.2 kbp fragment) treatment to yield pEAK-HDAC3 (pMH118).

108E: CTGAATTCACCATGGCCAAGACCG (SEQ ID NO:52)

108KFN:
GGGCGGCCGCCTACTTGTCATCGTCGTCCTTGTAATCGGTACCAATCTCCACATCG
CTTTC (SEQ ID NO:53)

### HDAC1

Full-length HDAC1 was amplified by PCR with pMH107 as a template using the primers HDAC1-E (SEQ ID NO:36) and 107K (SEQ ID NO:55). Meanwhile, the primers were designed by modifying the C-terminal stop codon to insert a KpnI site. The PCR fragment obtained was subcloned into pGEM-T (Promega) to yield pGEM-HDAC1 (E107K). E107K was inserted to pMH118 (EcoRI/KpnI, about 6 kbp) via EcoRI/KpnI (about 1.5 kbp fragment) treatment to yield pEAK-HDAC1 (pMH119).

107K: ATCGGTACCGGCCAACTTGACCTCCTCCTTG (SEQ ID
NO:55)

### HDAC2

Full-length HDAC2 was amplified by PCR with pMH111 as a template using the primers HDAC2-B (SEQ ID NO:38) and 111K (SEQ ID NO:56). Meanwhile, the primers were designed by modifying the C-terminal stop codon to insert a KpnI site. The PCR fragment obtained was subcloned into pGEM-T (Promega) to yield pGEM-HDAC2. pGEM-HDAC2 was inserted to pFLAG-CMV-5a (SIGMA) (BamHI/KpnI) via BamHI/KpnI treatment to yield pFLAG-CMV-5a-HDAC2 (pMH113). pMH113 was inserted to pMH118 (EcoRI/KpnI, about 6 kbp) via EcoRI/KpnI (about 1.5 kbp fragment) treatment to yield pEAK-HDAC2 (pMH121).

111K: ATCGGTACCGGGGTTGCTGAGCTGTTCTG (SEQ ID NO:56)

### HDAC4

Full-length HDAC4 was amplified by PCR with pMH106 as a template using the primers HDAC4-E (SEQ ID NO:42) and 106B (SEQ ID NO:57). Meanwhile, the primers were designed by modifying the C-terminal stop codon to insert a BglII site. The PCR fragment obtained was subcloned into pGEM-T (Promega) to yield pGEM-HDAC4 (E106B). A linker oligo having a BglII site and a FLAG sequence (DYKDDDDK) (FLAG-E (SEQ ID NO:58), prepared by annealing FLAG-N (SEQ ID NO:59)) was inserted to an EcoRI/NotI-treated fragment (about 3 kbp) of pBluescriptII KS(+) to yield pBlue-Flag. E106B was inserted to pBlue-Flag via EcoRI/BglII treatment to yield pBKS-HDAC4 (E106BN). E106BN was inserted to about 6 kbp pEAK10 (Edge Byo Systems) (EcoRI/NotI) via EcoRI/NotI (about 3.2 kbp fragment) treatment to yield pEAK-HDAC4 (pMH122).

106B: GATAGATCTCAGGGGCGGCTCCTCTTC (SEQ ID NO:57)

FLAG-E:
AATTCCTGCAGAGATCTGATTACAAGGACGACGATGACAAGTAGGC (SEQ ID
NO:58)

FLAG-N:
GGCCGCCTACTTGTCATCGTCGTCCTTGTAATCAGATCTCTGCAGG (SEQ ID
NO:59)

### HDAC5

A HindIII/NotI fragment (about 3.3 kbp) of pMH109 was inserted to p3XFLAG-CMV-10 (SIGMA) (HindIII/NotI, about 6.3 kbp) to yield p3XFLAG-CMV-10-HDACS (pMH144).

### HDAC6

A HindIII/NotI fragment (about 3.6 kbp) of pMH110 was inserted to p3XFLAG-CMV-10 (SIGMA) (HindIII/NotI, about 6.3 kbp) to yield p3XFLAG-CMV-10-HDAC6 (pMH145).

### HDAC7

A EcoRI/KpnI fragment (about 2.6 kbp) of pUC18-HDAC7 was inserted to PMH118 (EcoRI/KpnI, about 6 kbp) to yield pEAK-HDAC7 (pMH141).

### HDAC8

A EcoRI/KpnI fragment (about 1.1 kbp) of pUC18-HDAC8 was inserted to PMH118 (EcoRI/KpnI, about 6 kbp) to yield pEAK-HDAC8 (pMH140).

For details about the individual HDAC expression vectors prepared, see Table 2.

**Table 2.**

| Preparation of HDAC1-8 expression plasmid | | | |
|---|---|---|---|
| HDAC | Expression plasmid used¹⁾ | Position of Flag tag (DYKDDDK) for Each HDAC | Name of plasmid prepared |
| HDAC1 | pEAK10 (Edge Bio Systems) | C-terminal | pMH119 |
| HDAC2 | pEAK10 (Edge Bio Systems) | C-terminal | pMH121 |
| HDAC3 | pEAK10 (Edge Bio Systems) | C-terminal | pMH118 |
| HDAC4 | pEAK10 (Edge Bio Systems) | C-terminal | pMH122 |
| HDAC5 | p3XFLAG-CMV-10 (SIGMA) | N-terminal | pMH144 |
| HDAC6 | p3XFLAG-CMV-10 (SIGMA) | N-terminal | pMH145 |
| HDAC7 | pEAK10 (Edge Bio Systems) | C-terminal | pMH141 |
| HDAC8 | pEAK10 (Edge Bio Systems) | C-terminal | pMH140 |

| | | | |
|---|---|---|---|
| 1) pEAK10 (Edge Bio Systems) is regulated by EF1α (elongation factor 1α) promoter. p3XFLAG-CMV-10 (SIGMA) is regulated by CMV (cytomegarovirus) promoter. In addition, HDAC1, 2, 3, 4, 7 and 8 are inserted to each expression plasmid via EcoRI/NotI, and HDAC5 and 6 are inserted to each expression plasmid via HindIII/NotI. | | | |

### Example 4: Effects of overexpression of various HDAC isozymes on IL-2 transcriptional activity

1 µg of the plasmid pGL3 IL2 Pro43, which has a human IL-2 promoter sequence (the -674 to +54 region in the vicinity of the transcription initiation site) upstream of the firefly luciferase gene, 6 µg of pRL-TK (Promega), and 10 µg of each of various HDAC isozyme expression plasmids or a mock plasmid (the mock plasmid was pEAK10, and the various HDAC expression plasmids were pMH119 for HDAC1, pMH121 for HDAC2, pMH118 for HDAC3, pMH122 for HDAC4, pMH144 for HDAC5, pMH145 for HDAC6, pMH141 for HDAC7, and pMH140 for HDAC8, respectively) were mixed, and 1 x 10⁷ Jurkat cells (ATCC, TIB-152) were transformed by the electroporation method (BIO-RAD, Gene Pulser II, voltage 300 V, charge 975 µF, 400 µL).

After transformation, 2.5 mL of RPMI 1640 (SIGMA) containing 10% FBS (MOREGATE) (10% FBS RPMI 1640) was added, and this mixture was dispensed to a 96-well white plate in a ratio of 50 µL/well. After cultivation under conditions of 37°C, 5% CO₂ and saturated humidity for 10 hours, a 10% FBS RPMI 1640 solution was added at 50 µL/well, and a liquid mixture of Phorbol 12-Myristate 13-Acetate (PMA, SIGMA), Ionomycin (SIGMA) and Anti-CD28 antibody (Pharmingen) in 10% FBS RPMI 1640 was added at 50 µL/well (the final concentrations were 50 ng/mL, 1 µg/mL and 75 ng/mL, respectively) to stimulate the Jurkat cells. After cultivation under conditions of 37°C, 5% CO₂ and saturated humidity for 12 hours, luciferase activity in the cells was measured according to the manual of the Dual Luciferase Assay System (Promega) using a multi-label counter (1420 MULTILABEL COUNTER ARVO SX, WALLAC).

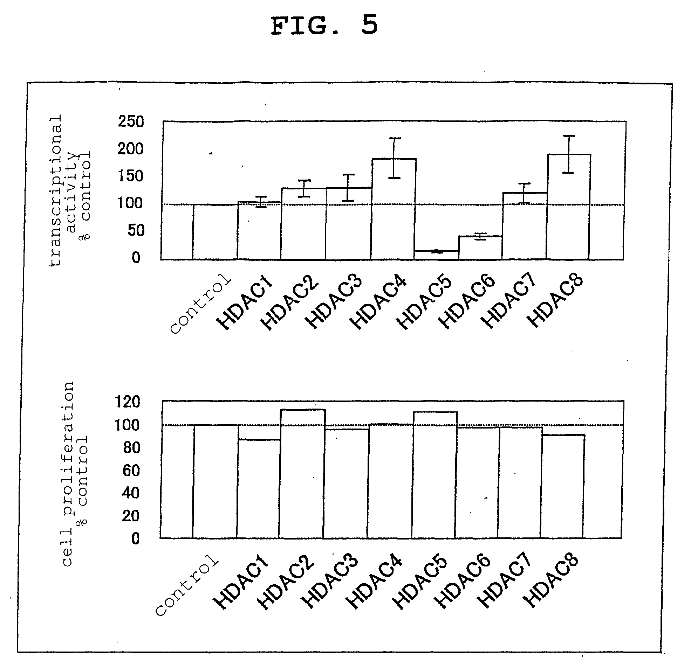

As a result, it was found that luciferase was induced from pGL3 IL2 Pro43 in response to the stimulation of the Jarkat cells by PMA, Ionomycin, and Anti-CD28 antibody, and that the expression level thereof increased with the overexpression of HDAC4 and 8 (FIG. 5). It was also confirmed using the Cell Counting Kit-8 (DOJIN) that the overexpression of various HDAC isozymes did not significantly affect the degree of cell proliferation (FIG. 5).

These results demonstrate that HDAC4 and 8 are HDAC isozymes involved in the IL-2 transcription system.

### Example 5: Effects of overexpression of various HDAC isozymes on GATA-1 transcriptional activity

5 µg of the plasmid pGL3-HSI-IE Pro, which has a human GATA-1 promoter sequence (the -3769 to -3133 and -789 to +32 regions in the vicinity of the transcription initiation site) upstream of the firefly luciferase gene, and 10 µg of each of various HDAC isozyme expression plasmids or a mock plasmid (the mock plasmid was pEAK10, and HDAC1 to 4, 7 and 8 were under the control of the EF-1α promoter, HDAC5 and 6 were under the control of the CMV promoter, and the various HDAC isozymes had a Flag tag at the C-terminal or N-terminal thereof) were mixed, and 8.75 x 10⁶ HEL cells (accession number JCRB0062, JCRB) were transformed by the electroporation method (BIO-RAD, Gene Pulser II, voltage 1750 V, charge 10 µF, 365 µL). After transformation, 2 mL of 10% FBS RPMI 1640 was added, and this mixture was dispensed to a 96-well white plate in a ratio of 50 µL/well. After cultivation under conditions of 37°C, 5% CO₂ and saturated humidity for 3 hours, a 10% FBS RPMI 1640 medium was added at 100 µL/well. After cultivation under conditions of 37°C, 5% CO₂ and saturated humidity for 8 hours, luciferase activity in the cells was measured according to the manual of the Dual Luciferase Assay System using a multi-label counter.

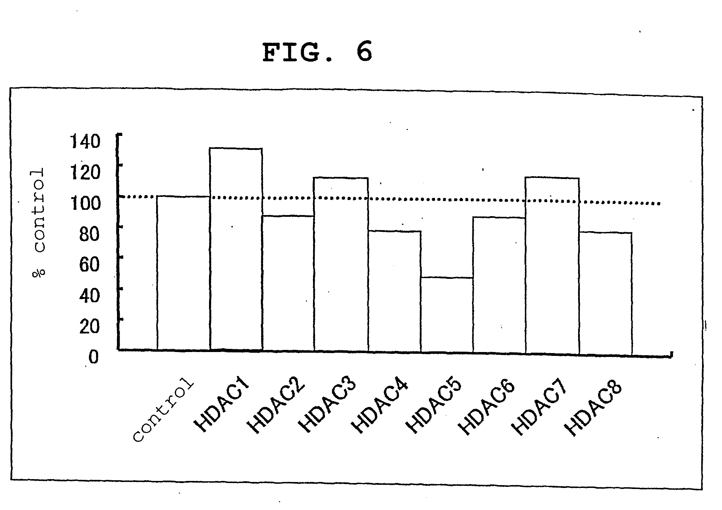

As a result, it was found that luciferase depending on the GATA-1 promoter was induced from pGL3-HSI-IE Pro, and that the expression level of the induced luciferase increased slightly with the overexpression of HDAC1, 3 and 7 (FIG. 6).

These results demonstrate that HDAC1, 3 and 7 are involved in the GATA-1 transcription system, and that HDAC4 and 8, which are involved in the IL-2 transcription system, are not significantly involved in the GATA-1 transcription system.

### Example 6: Preparation of HDAC4 dominant negative variants

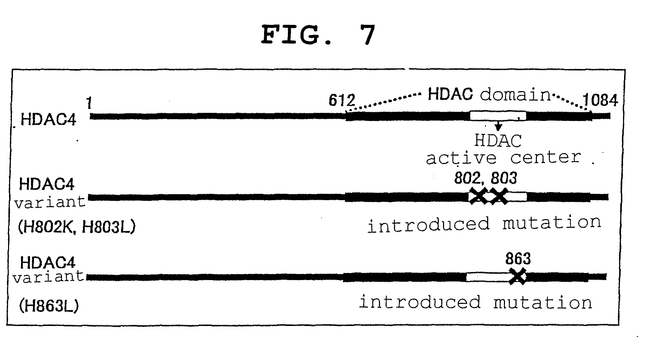

For HDAC4, which seems to be largely involved in IL-2 transcription, HDAC4 dominant negative variants were prepared for the purpose of losing the function of endogenous HDAC4. Hence, from the literature information in PNAS, 1998, Vol. 95, p.3519-3524 & Mol. Cell. Biol., 1999, Vol. 19 (11), p.7816-7827, the sites for mutagenesis to reduce HDAC activity were determined (H802K, H803L, H863L) (FIG. 7). The human HDAC4 CDS base sequence was obtained from GenBank accession number AF132607, and primers for mutagenesis were designed (for the base sequences of the individual primers, see Table 3; for the HDAC4 CDS base sequence, see SEQ ID NO:1; for the amino acid sequence encoded by the HDAC4 CDS base sequence, see SEQ ID NO:2).

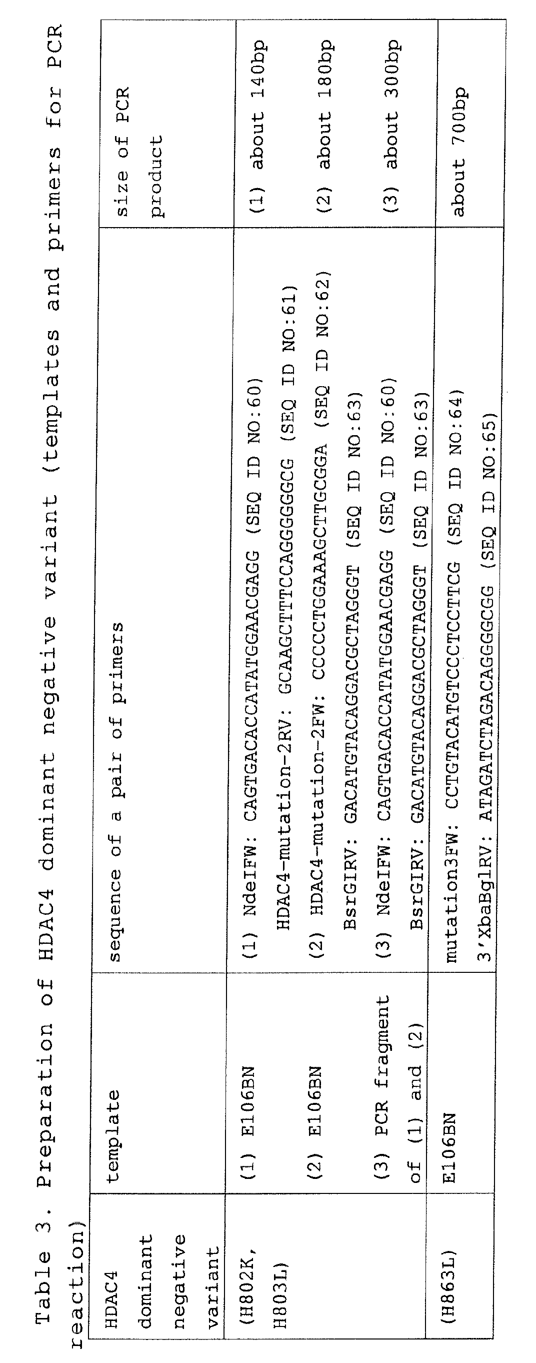

A PCR reaction was conducted according to the protocol of Expand (High fidelity PCR System (Roche)). E106BN, which was used as a template, is a plasmid prepared by inserting full-length HDAC4 CDS to pBluescriptII KS(+) (TOYOBO) (EcoRI/NotI, the initiation codon was located on the EcoRI side).

Using the Topo TA cloning kit for sequence (Invitrogen Company), each of the PCR products obtained ((3) for H802K and H803L variants) was subcloned into the TOPO cloning vector for sequence (pCR4-HDAC4 (H802K, H803L) and pCR4-HDAC4 (H863L)). Subsequently, a PCR fragment confirmed as being mutated by sequencing was inserted to an HDAC4 expression plasmid (pMH122) by the procedures shown below.

### HDAC4 dominant negative variants (H802K, H803L)

An NdeI/BsrGI-treated fragment (about 300 bp) of pCR4-HDAC4 (H802K, H803L) was inserted to E106BN (NdeI/BsrGI) to yield pBKS-HDAC4 (H802K, H803L). Subsequently, an EcoRI/NotI-treated fragment (about 3.3 kbp) of pBKS-HDAC4 (H802K, H803L) was inserted to pMH122 (EcoRI/NotI, about 6 kbp) to yield pEAK-HDAC4 (H802K, H803L). The base sequence in the coding region of HDAC4 dominant negative variants (H802K, H803L) is shown by SEQ ID NO:3, and the amino acid sequence thereof is shown by SEQ ID NO:4.

### HDAC4 dominant negative variant (H863L)

A BsrGI/BglII-treated fragment (about 750 bp) of pCR4-HDAC4 (H863L) was inserted to E106BN (BsrGI/BglII, about 3 kbp) to yield pBKS-HDAC4 (H863L). Subsequently, an EcoRI/NotI-treated fragment (about 3.3 kbp) of pBKS-HDAC4 (H863L) was inserted to pMH122 (EcoRI/NotI, about 6 kbp) to yield pEAK-HDAC4 (H863L). The base sequence in the coding region of HDAC4 dominant negative variant (H863L) is shown by SEQ ID NO:5, and the amino acid sequence thereof is shown by SEQ ID NO:6.

### Example 7: Preparation of HDAC1 dominant negative variants

HDAC1 dominant negative variants were prepared in the same manner as the preparation of the HDAC4 dominant negative variants. The human HDAC1 CDS base sequence was obtained from GenBank accession number D50405, and primers for mutagenesis were designed (for the base sequences of the individual primers, see Table 4; for the base sequence of HDAC1 CDS, see SEQ ID NO:9; for the amino acid sequence encoded by the HDAC1 CDS base sequence, see SEQ ID NO:10).

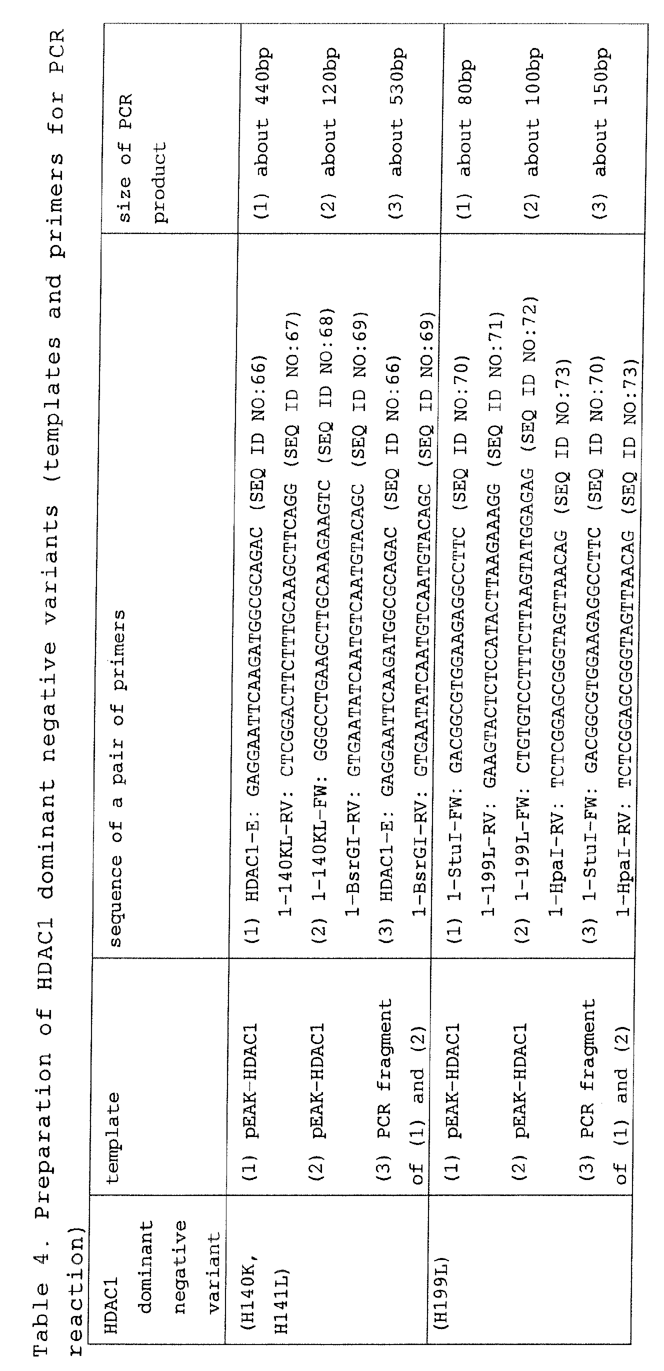

A PCR reaction was conducted according to the protocol of Expand (High fidelity PCR System (Roche)). pEAK-HDAC1 (pMH119), which was used as a template, is a plasmid prepared by inserting full-length HDAC1 CDS to pEAK10.

Using the Topo TA cloning kit for sequence (Invitrogen Company), each of the PCR products obtained (3) was subcloned into the TOPO cloning vector for sequence (pCR4-HDAC1 (H140K, H141L) and pCR4-HDAC1 (H199L)). A PCR fragment confirmed as being mutated by sequencing was inserted to an HDAC1 expression plasmid (pMH119) using the procedures shown below. E107K is a plasmid prepared by inserting full-length HDAC1 CDS to pGEM-T (Promega) (EcoRI/KpnI, the initiation codon is located on the EcoRI side).

### HDAC1 dominant negative variants (H140K, H141L)

An EcoRI/BsrGI-treated fragment (about 530 bp) of pCR4-HDAC1 (H140K, H141L) was inserted to E107K (EcoRI/BsrGI, about 3 kbp) to yield pGEM-HDAC1 (H140K, H141L). Subsequently, pGEM-HDAC1 (H140K, H141L) (EcoRI/KpnI, about 1.5 kbp) was inserted to an EcoRI/KpnI-treated fragment (about 5.9 kbp) of pMH119 to yield pEAK-HDAC1 (H140K, H141L). The base sequence in the coding region of HDAC1 dominant negative variants (H140K, H141L) is shown by SEQ ID NO:11, and the amino acid sequence thereof is shown by SEQ ID NO:12.

### HDAC1 dominant negative variant (H199L)

An StuI/HpaI-treated fragment (about 150 bp) of pCR4-HDAC1 (H199L) was inserted to E107K (StuI/HpaI) (about 3 kbp) to yield pGEM-HDAC1 (H199L). Subsequently, pGEM-HDAC1 (H199L) (EcoRI/KpnI, about 1.5 kbp) was inserted to an EcoRI/KpnI-treated fragment (about 5.9 kbp) of pMH119 to yield pEAK-HDAC1 (H199L). The base sequence in the coding region of HDAC1 dominant negative variant (H199L) is shown by SEQ ID NO:13, and the amino acid sequence thereof is shown by SEQ ID NO:14.

### Example 8: Preparation of HDAC3 dominant negative variants

HDAC3 dominant negative variants were prepared in the same manner as the preparation of the HDAC4 dominant negative variants. The human HDAC3 CDS base sequence was obtained from GenBank accession number U66914, and primers for mutagenesis were designed (for the base sequences of the individual primers, see Table 5; for the HDAC3 CDS base sequence, see SEQ ID NO:15; for the amino acid sequence encoded by the HDAC3 CDS base sequence, see SEQ ID NO:16).

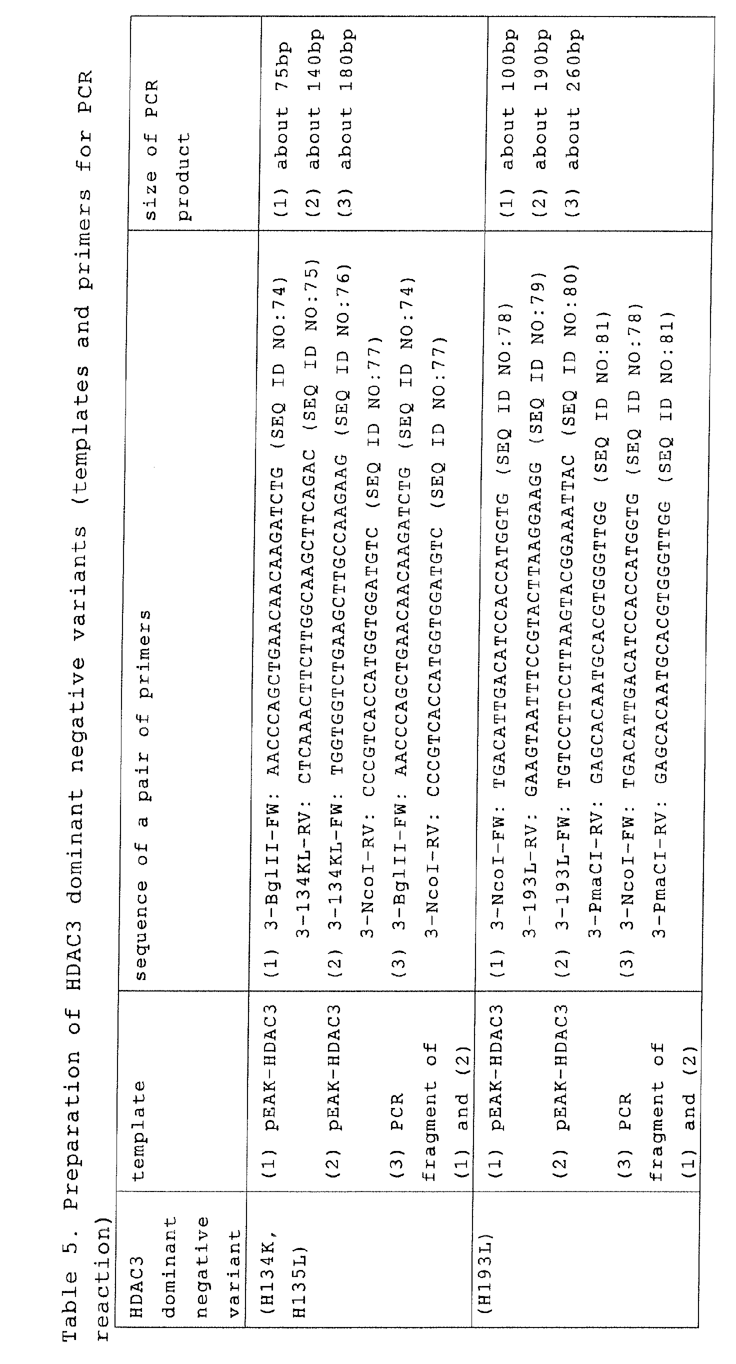

A PCR reaction was conducted according to the protocol of Expand (High fidelity PCR System (Roche)). pEAK-HDAC3 (pMH118), which was used as a template, is a plasmid prepared by inserting full-length HDAC3 CDS to pEAK10.

Using the Topo TA cloning kit for sequence (Invitrogen Company), each of the PCR products (3) obtained was subcloned into the TOPO cloning vector for sequence (pCR4-HDAC3 (H134K, H135L) and pCR4-HDAC3 (H193L)). A PCR fragment confirmed as being mutated by sequencing was inserted to an HDAC3 expression plasmid (pMH118) by the procedures shown below.

### HDAC3 dominant negative variants (H134K, H135L)

An EcoRI/KpnI-treated fragment (about 1.3 kbp) of pMH118 was inserted to pBluescriptII KS(-) (TOYOBO) (EcoRI/KpnI) (about 3 kbp) to yield pBKS-HDAC3. Subsequently, pCR4-HDAC3 (H134K, H135L) (BglII/NcoI, about 180 bp) was inserted to pBKS-HDAC3 (BgIII/NcoI, about 3 kbp) to yield pBKS-HDAC3 (H134K, H135L). Finally, pBKS-HDAC3 (H134K, H135L) (EcoRI/KpnI, about 1.3 kbp) was inserted to an EcoRI/KpnI-treated fragment (about 5.7 kbp) of pMH118 to yield pEAK-HDAC3 (H134K, H135L). The base sequence in the coding region of HDAC3 dominant negative variants (H134K, H135L) is shown by SEQ ID NO:17, and the amino acid sequence thereof is shown by SEQ ID NO:18.

### HDAC3 dominant negative variant (H193L)

A pCR4-HDAC3 (H134K, H135L) (NcoI/PmaCI, About 260 bp) was inserted to pMH118 (NcoI/PmaCI, about 7 kbp) to yield pEAK-HDAC3 (H193L). The base sequence in the coding region of HDAC3 dominant negative variant (H193L) is shown by SEQ ID NO:19, and the amino acid sequence thereof is shown by SEQ ID NO:20.

### Example 9: IL-2 transcriptional activity suppression by expression of HDAC4 dominant negative variants

To 1 µg of the plasmid pGL3 IL2 Pro43, which has a human IL-2 promoter sequence (the -674 to +54 region in the vicinity of the transcription initiation site) upstream of the firefly luciferase gene, and 6 µg of pRL-TK (Promega), the plasmids for the expression of wild-type HDAC4 or each of HDAC4 dominant negative variants (H802K, H803L, H863L) prepared in Example 3 was mixed in various ratios (1, 3 and 10 µg), and 1 x 10⁷ Jurkat cells (ATCC, TIB-152) were transformed by the electroporation method (BIO-RAD, Gene Pulser II, voltage 300 V, charge 975 µF, 400 µL).

After transformation, 2.5 mL of RPMI 1640 containing 10% FBS (MOREGATE) (10% FBS RPMI 1640) was added, and this mixture was dispensed to a 96-well white plate in a ratio of 50 µL/well. After cultivation under conditions of 37°C, 5% CO₂ and saturated humidity for 10 hours, a liquid mixture of Phorbol 12-Myristate 13-Acetate (PMA, SIGMA), Ionomycin (SIGMA) and Anti-CD28 antibody (Pharmingen) in 10% FBS RPMI 1640 was added at 50 µL/well (the final concentrations were 50 ng/mL, 1 µg/mL and 75 ng/mL, respectively) to stimulate the Jurkat cells. After cultivation under conditions of 37°C, 5% CO₂ and saturated humidity for 12 hours, luciferase activity in the cells was measured according to the manual of the Bright-Glo Luciferase Assay System (Promega) using a multi-label counter (1420 MULTILABEL COUNTER ARVO SX, WALLAC).

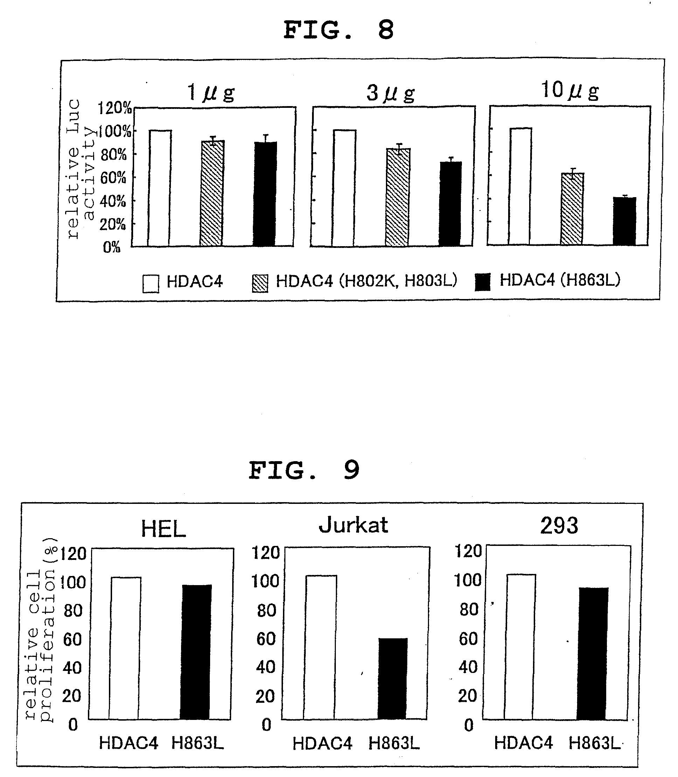

As a result, IL-2 transcription suppression depending on the expression level of HDAC4 dominant negative variants (H802K, H803L, H863L) was observed (FIG. 8). The expression level of the transferred HDAC was confirmed by Western blotting using an anti-Flag antibody (SIGMA, anti-Flag M2).

These results suggest that IL-2 transcription may be suppressed by selectively suppressing the function of HDAC4.

### Example 10: Cell proliferation suppression by expression of HDAC4 dominant negative variants

10 µg of a plasmid for the expression of wild-type HDAC4 and the HDAC4 dominant negative variant (H863L) prepared in Example 6 was transfected to Jurkat cells, HEL cells and 293 cells.

1 x 10⁷ Jurkat cells (ATCC, TIB-152) were transformed by the electroporation method (BIO-RAD, Gene Pulser II, voltage 300 V, charge 975 µF, 400 µL). After transformation, 2.5 mL of RPMI 1640 containing 10% FBS (MOREGATE) (10% FBS RPMI 1640) was added, and this mixture was dispensed to a 96-well white plate in a ratio of 50 µL/well. After cultivation under conditions of 37°C, 5% CO₂ and saturated humidity for 5 hours, a liquid mixture of Phorbol 12-Myristate 13-Acetate (PMA, SIGMA), Ionomycin (SIGMA) and Anti-CD28 antibody (Pharmingen) in 10% FBS RPMI 1640 was added at 50 µL/well (the final concentrations were 50 ng/mL, 1 µg/mL and 75 ng/mL, respectively) to stimulate the Jurkat cells. After cultivation under conditions of 37°C, 5% CO₂ and saturated humidity for 4 hours, the number of cell was confirmed using the Cell Counting Kit-8 (DOJIN).

8.75 x 10⁶ HEL cells (accession number JCRB0062, JCRB) were transformed by the electroporation method (BIO-RAD, Gene Pulser II, voltage 1750 V, charge 10 µF, 365 µL). After transformation, 2 mL of 10% FBS RPMI 1640 was added, and this mixture was dispensed to a 96-well white plate in a ratio of 50 µL/well. After cultivation under conditions of 37°C, 5% CO₂ and saturated humidity for 10 hours, 10% FBS RPMI 1640 medium was added at 50 µL/well, further cultivation was conducted for 12 hours, and the number of cell was confirmed using the Cell Counting Kit-8 (DOJIN).

293 cells (ATCC, CRL-1573) were transfected by the calcium phosphate method (a mixture of DNA and calcium phosphate was added to 293 cells in culture using a DMEM (SIGMA) medium containing 10% FBS, and the medium was replaced with a fresh one 6 hours later); after cultivation under conditions of 37°C, 5% CO₂ and saturated humidity for 48 hours, the number of cell was confirmed using the Cell Counting Kit-8 (DOJIN).

As a result, cell proliferation inhibition specific for Jurkat cells due to the expression of HDAC4 dominant negative variant (H863L) was observed (FIG. 9). For each type of cells, the expression level of the transferred HDAC was confirmed by Western blotting using an anti-Flag antibody (SIGMA, anti-Flag M2).

These results demonstrate that the proliferation of T cell lineage is selectively suppressed by selectively inhibiting the function of HDAC4.

### Example 11: Effects of expression of HDAC1, 3 and 4 dominant negative variants on IL-2 and GATA-1 transcription activities

IL-2 and GATA-1 transcription activities were measured with the expression of the HDAC1, 3 and 4 dominant negative variants prepared in Examples 6, 7 and 8.

1 x 10⁷ Jurkat cells (ATCC, TIB-152) were transformed with 10 µg of a plasmid for the expression of various HDACs or HDAC variants by the electroporation method (BIO-RAD, Gene Pulser II, voltage 300 V, charge 975 µF, 400 µL). After transformation, 2.5 mL of RPMI 1640 (SIGMA) containing 10% FBS (MOREGATE) (10% FBS RPMI 1640) was added, and this mixture was dispensed to a 96-well white plate in a ratio of 50 µL/well. After cultivation under conditions of 37°C, 5% CO₂ and saturated humidity for 10 hours, a liquid mixture of Phorbol 12-Myristate 13-Acetate (PMA, SIGMA), Ionomycin (SIGMA) and Anti-CD28 antibody (Pharmingen) in 10% FBS RPMI 1640 was added at 50 µL/well (the final concentrations were 50 ng/mL, 1 µg/mL and 75 ng/mL, respectively) to stimulate the Jurkat cells. After cultivation under conditions of 37°C, 5% CO₂ and saturated humidity for 12 hours, luciferase activity in cells were measured according to the manual of the Bright-Glo Luciferase Assay System (Promega), and IL-2 transcriptional activity was measured using a multi-label counter (1420 MULTILABEL COUNTER ARVO SX, WALLAC).

Using 15 µg of pGL3-HSI-IE Pro and 10 µg of a plasmid for the expression of various HDACs or HDAC variants, 8.75 x 10⁶ HEL cells (accession number JCRB0062, JCRB) were transformed by the electroporation method (BIO-RAD, Gene Pulser II, voltage 1750 V, charge 10 µF, 365 µL). After transformation, 2 mL of 10% FBS RPMI 1640 was added, and this mixture was dispensed to a 96-well white plate in a ratio of 50 µL/well. After cultivation under conditions of 37°C, 5% CO₂ and saturated humidity for 3 hours, 10% FBS RPMI 1640 medium was added at 50 µL/well. After cultivation under conditions of 37°C, 5% CO₂ and saturated humidity for 8 hours, luciferase activity in cells were measured according to the manual of the Bright-Glo Luciferase Assay System, and GATA-1 transcriptional activity was measured using a multi-label counter.

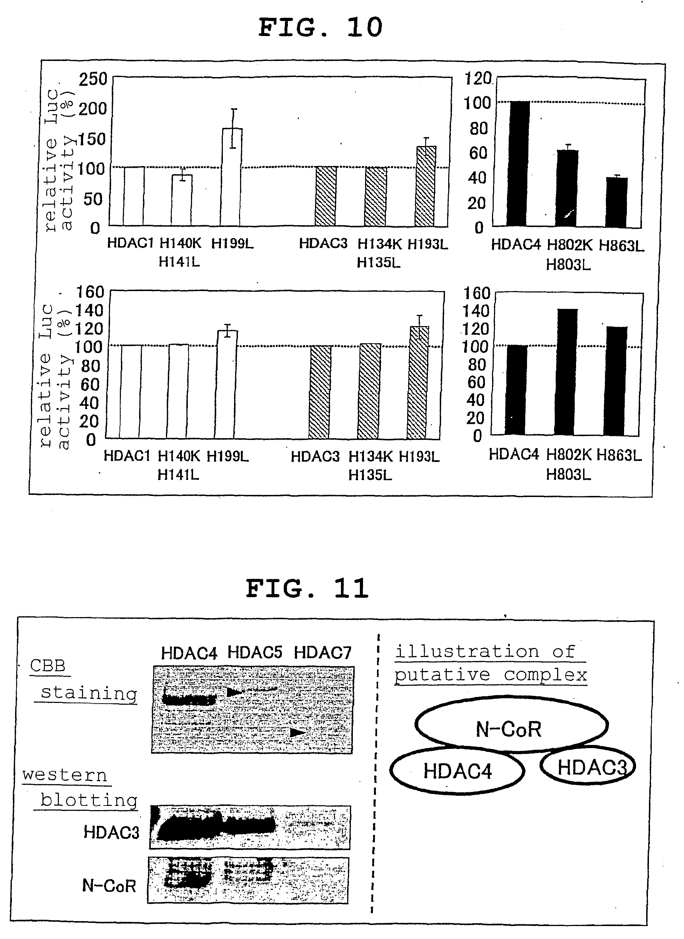

As a result, it was found that IL-2 transcription suppression was observed only with the expression of HDAC4 dominant negative variants (H802K, H803L, H863L), and that GATA-1 transcription suppression was not observed at that time (FIG. 10). For each type of cells, the expression level of the transferred HDAC was confirmed by Western blotting using an anti-Flag antibody (SIGMA, anti-Flag M2).

These results suggest that immune function can be suppressed, without suffering thrombocytopenia, by selectively inhibiting the function of HDAC4.

### Example 12: Complex formation of HDAC4

In Mol. Cell, Vol. 9, p.45-57, 2002, it is reported that Class II HDACs have formed a complex with N-CoR and HDAC3, and that HDAC activity with histone as a substrate is much more portent for HDAC3 than for Class II HDACs. Hence, the formation of complexes of crudely purified Class II HDACs was confirmed.

7 µg of each of HDAC4, 5 and 7 expression plasmids was transfected to PEAK Rapid cells (Edge BioSystems) by the calcium phosphate method; 48 hours later, whole cell lysate (cells were disrupted in 25mM Tris, pH 7.4, 150mM NaCl, 1mM CaCl₂, 1% Triton X-100, and cell debris was removed by centrifugation) was crudely purified using an anti-Flag antibody column (SIGMA, anti-Flag M2-agarose) (washed with TBS, then eluted with 0.1M Glysine-HCl, pH 3.5). Subsequently, the crudely purified fraction was subjected to SDS-PAGE and then stained with CBB.

As a result, the expression of the transferred HDAC4, 5 and 7 was confirmed (FIG. 11). It was also confirmed by Western blotting that each of the crudely purified fractions of HDAC4, 5 and 7 contained HDAC3 and N-CoR (using an anti-HDAC3 antibody of SIGMA Company and an anti-N-CoR antibody of Santa Cruz Company).

These results confirm that the crudely purified HDAC4, 5 and 7 have formed a complex with N-CoR and HDAC3.

### Example 13: Complex forming ability of HDAC4 dominant negative variants

In Mol. Cell, Vol. 9, p.45-57, 2002, it is reported that some HDAC4 variants do not form a complex with N-CoR and HDAC3. Hence, the complex forming ability of HDAC4 dominant negative variant (H863L) was examined.

7 µg of a plasmid for the expression of wild-type HDAC4 and HDAC4 dominant negative variant (H863L) was transfected to PEAK Rapid cells (Edge BioSystems) by the calcium phosphate method; 48 hours later, whole cell lysate (cells were disrupted in 25mM Tris, pH 7.4, 150mM NaCl, 1mM CaCl₂, 1% Triton X-100, and cell debris was removed by centrifugation) was crudely purified using an anti-Flag antibody column (SIGMA, anti-Flag M2-agarose) (washed with TBS, then eluted with 0.1M Glysine-HCl, pH 3.5). Subsequently, the crudely purified fraction was subjected to SDS-PAGE and then stained with CBB.

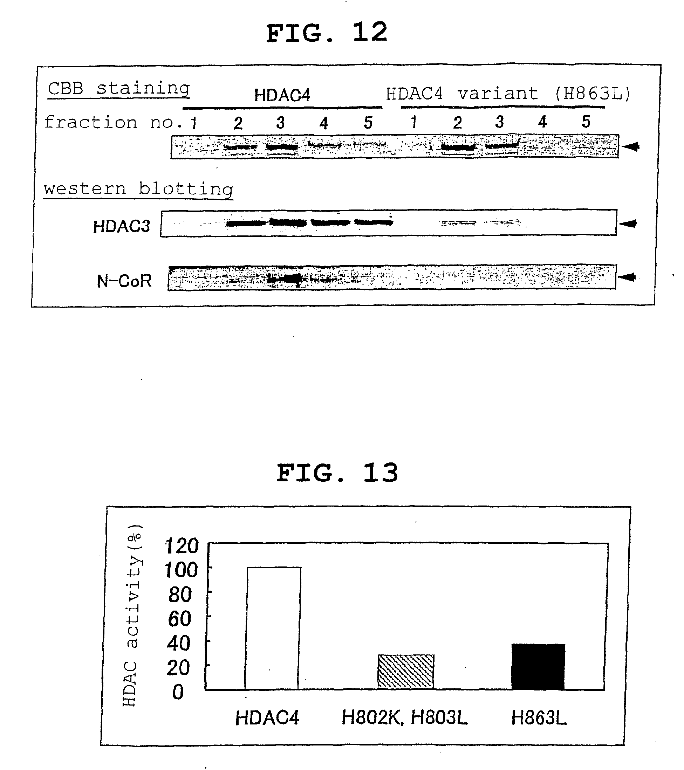

As a result, the expression of the transferred HDAC4 and HDAC4 dominant negative variant (H863L) was confirmed (FIG. 12). It was also confirmed by Western blotting (using an anti-HDAC3 antibody of SIGMA Company and an anti-N-CoR antibody of Santa Cruz Company) that the crudely purified fraction of HDAC4 contained HDAC3 and N-CoR, and that the crudely purified fraction of HDAC4 dominant negative variant (H863L) contained almost no HDAC3 or N-CoR.

These results demonstrate that HDAC4 dominant negative variant (H863L) has a decreased complex forming ability with N-CoR and HDAC3 compared to wild-type HDAC4.

### Example 14: Measurement of HDAC activity

In Mol. Cell, Vol. 9, p.45-57, 2002, it is reported that HDAC4 variants which decreased complex forming ability with N-CoR and HDAC3 shows decrease of HDAC activity with histone as a substrate. Hence, the HDAC activities of HDAC4 dominant negative variants (H802K, H803L, H863L) with histone as a substrate were measured.

7 µg of a plasmid for the expression of wild-type HDAC4 and HDAC4 dominant negative variants (H802K, H803L, H863L) was transfected to 293T cells by the calcium phosphate method; 48 hours later, whole cell lysate (cells were disrupted in 25mM Tris, pH 7.4, 150mM NaCl, 1mM CaCl₂, 1% Triton X-100, and cell debris was removed by centrifugation) was crudely purified using an anti-Flag antibody column (SIGMA, anti-Flag M2-agarose) (washed with TBS, then eluted with 0.1M Glysine-HCl, pH 3.5), and used for the measurement of HDAC activity. The reaction substrate used was a [³H]-labeled acetylated histone prepared by cultivating Jurkat cells (2×10⁸ cells) in 40 mL of an RPMI (SIGMA) medium containing 300 MBq [³H] sodium acetate (NENTM Life Science Products, Inc.) for 2 hours, disrupting the cells in Lysis buffer, pH 6.5 (10mM Tris-HCl, 50mM Sodium Hydrogen sulfate, 1% Triton X-100, 10mM MgCl₂-6H₂O, 8.6% Sucrose), washing the cells with Wash buffer (10mM Tris-HCl, 13mM EDTA), and then adding 0.4N H₂SO₄.

As a result, the HDAC4 dominant negative variants (H802K, H803L, H863L) had decreased HDAC activity compared to wild-type HDAC4 (FIG. 13).

These results confirm that the HDAC4 dominant negative variants (H802K, H803L, H863L) have decreased complex forming ability with N-CoR and HDAC3 (Example 13), and hence decreased HDAC activity with histone as a substrate.

### Example 15: Construction of detection system for interaction of HDAC4 and N-CoR

Since it was found that an HDAC4 dominant negative variant (H863L) had decreased complex forming ability with N-CoR compared to wild-type HDAC4, it was considered that the same IL-2 transcription suppressive action as that obtained with the expression of HDAC4 dominant negative variant is exhibited by inhibiting the interaction of HDAC4 and N-CoR.

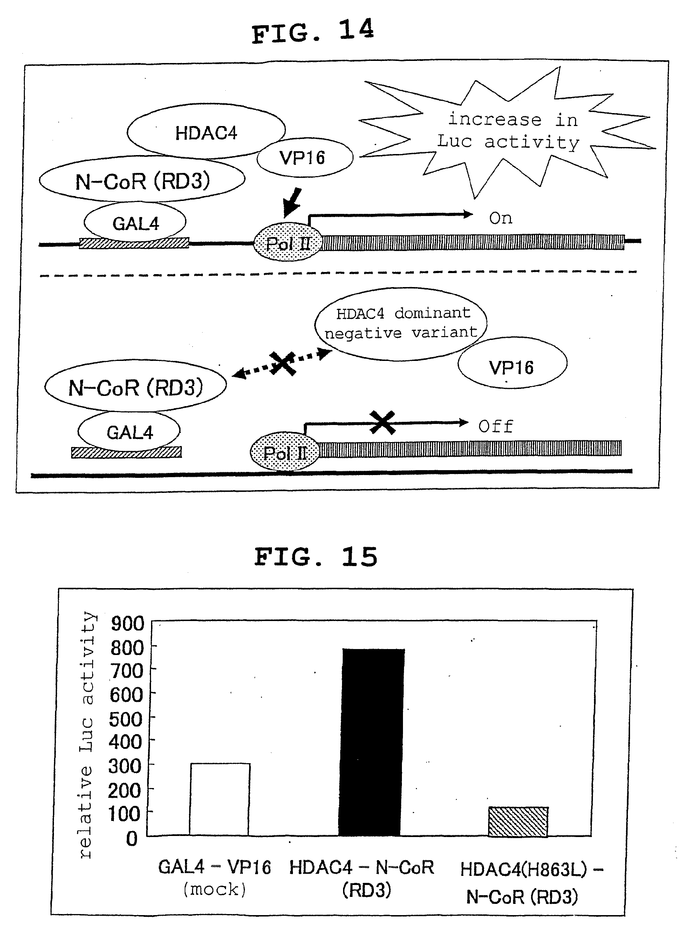

Hence, to construct a screening system for a drug that inhibits the interaction of HDAC4 and N-CoR, the CheckMate Mammalian Two-Hybrid System (Promega) was used (FIG. 14).

First, from the literature information in Mol. Cell, 2002, Vol. 9, p.45-57, the site corresponding to the RD3 sequence of human N-CoR was determined (1005-1498a.a.). The human N-CoR CDS base sequence was obtained from GenBank accession number AF044209, and human N-CoR(RD3) cDNA was amplified from human-lung-derived cDNA by the PCR method. The primers used were RD3-Bam-FW (SEQ ID NO:82) and RD3-Not-RV (SEQ ID NO:83) below. For the base sequence of human N-CoR(RD3), see SEQ ID NO:21; for the amino acid sequence encoded by the base sequence of human N-CoR(RD3), see SEQ ID NO:22. The PCR fragment obtained was confirmed by sequencing (for N-CoR(RD3), a variation of L1014V was observed, but this was adopted since it was reported as a variant in SWISS-PROT), and it was inserted to pBIND (Promega) via treatment with restriction endonuclease (BamHI/NotI) to yield a GAL4-N-CoR(RD3) expression plasmid. Also, HDAC4 or an HDAC4 dominant negative variant (H863L) in full length was inserted to pACT (Promega) via restriction endonuclease treatment (XbaI/NotI) to yield HDAC4-VP16 and HDAC4(H863L)-VP16 expression plasmids.

RD3-Bam-FW: GGGATCCGTGAAGTCCTTCAGCCTGCTCC (SEQ ID
NO:82)

RD3-Not-RV: TGCGGCCGCCATGGGTGAGCCTCTGGACA (SEQ ID
NO:83)

Subsequently, 10 µg of pG5-Luc (Promega), 3 µg of pBND and 3 µg of pACT, or 10 µg of pG5-Luc, 3 µg of pBND-N-CoR(RD3) and 3 µg of pACT-HDAC4, or 10 µg of pG5-Luc, 3 µg of pBND-N-CoR(RD3) and 3 µg of pACT-HDAC4 (H863L), were mixed respectively, and each mixture was transfected to 1 x 10⁷ Jurkat cells (ATCC, TIB-152) by the electroporation method (BIO-RAD, Gene Pulser II, voltage 300 V, charge 975 µF, 400 µL). Twenty-two hours after transfection, luciferase activity in the cells was measured according to the manual of the Bright-Glo Luciferase Assay System (Promega) using a multi-label counter (1420 MULTILABEL COUNTER ARVO SX, WALLAC).

As a result, GAL4 transcription activation due to the interaction of N-CoR (RD3) and HDAC4 was detected, and it was confirmed that the HDAC4 dominant negative variant (H863L) did not interact with N-CoR(RD3) (FIG. 15).

From these results, it was evaluated that a detection system for the interaction of HDAC4 and N-CoR(RD3) was constructed.

### Example 16: Effects of HDAC4-selective gene expression suppression using siRNA on IL-2 and GATA-1 transcription activities

A mixture of four kinds of HDAC4-targeted siRNA, i.e., siGENE HDAC4 SMARTpool (manufactured by Dharmacon Inc., marketed by B-Bridge international Inc., code number M-003497-00-05), or Non-specific Control Duplex IX (47% GC content) (manufactured by Dharmacon Inc., marketed by B-Bride international Inc., code number D-001206-09-20), and pGL3 IL2 Pro43 were transfected to Jurkat cells by the electroporation method using the same conditions as those mentioned above, and IL-2 transcriptional activity was measured. Likewise, siGENE HDAC4 SMARTpool or Non-specific Control Duplex IX, and pGL3-HSI-IE Pro were transfected to HEL cells, and GATA-1 transcriptional activity was measured. The conditions of siRNA transfection and their effects in Jurkat cells and HEL cells were previously confirmed using pGL3-control (Promega) and Luciferase GL3 Duplex (B-Bride: D-001400-01-20).

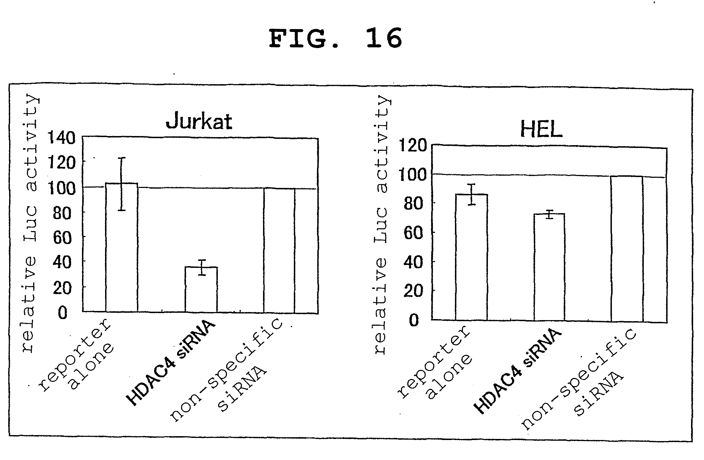

As a result, IL-2 transcription suppression due to the selective suppression of the expression of the HDAC4 gene was observed, but GATA-1 transcription suppression was not observed (FIG. 16).

These results again suggest that immune function can be suppressed, without suffering thrombocytopenia, by selectively inhibiting the function of HDAC4.

### Industrial Applicability

The present invention has shown that immunity can be suppressed, IL-2 production can be inhibited, or immunocyte proliferation can be suppressed, without exhibiting a noticeable thrombocytopenic activity and/or GATA-1 production inhibitory activity, by selectively suppressing HDAC4/HDAC8, out of various HDACs. Therefore, by selecting a compound that selectively suppresses HDAC4/HDAC8, it is possible to select an immunosuppressant having low thrombocytopenic activity or an IL-2 production inhibitor or an immunocyte proliferation inhibitor having low GATA-1 production inhibitory activity more conveniently and in a shorter time than conventional. Hence, the method of the present invention is very useful for research for developing new drug.

The present appliation is base on Japanese patent application no. 2002-378800 filed in Japan on December 27, 2002 and its content is herein incorporated by reference.

## Claims

1. An agent for inhibiting IL-2 production and/or an agent for inhibiting immunocyte proliferation, each of which having low GATA-1 production inhibitory activity, comprising a selective HDAC4 and/or HDAC8 inhibitor.

2. An agent for suppressing immunity having low thrombocytopenic activity, comprising a selective HDAC4 and/or HDAC8 inhibitor.

3. A method of selecting an IL-2 production inhibitor and/or an immunocyte proliferation inhibitor which have low GATA-1 production inhibitory activity, which comprises measuring the HDAC4 and/or HDAC8 enzyme inhibitory activity of a test substance.

4. A method of selecting an immunosuppressant having low thrombocytopenic activity, which comprises measuring the HDAC4 and/or HDAC8 enzyme inhibitory activity of a test substance.

5. A method of selecting an immunosuppressant having low thrombocytopenic activity, which comprises steps (i) and (ii) below:
(i) measuring the HDAC4 and/or HDAC8 enzyme inhibitory activity of a test substance, and
(ii) measuring the HDAC enzyme inhibitory activity of the test substance, wherein the HDAC enzyme inhibitory activity is one or more HDAC enzyme inhibitory activities selected from a group consisting of HDAC1, HDAC2, HDAC3, HDAC5, HDAC6 and HDAC7 enzyme inhibitory activities.

6. The method of claim 3, which is a method of selecting an IL-2 production inhibitor and/or an immunocyte proliferation inhibitor which have low GATA-1 production inhibitory activity, and which comprises steps (i) to (iii) below, wherein the IL-2 production inhibitor and/or the immunocyte proliferation inhibitor selectively inhibits HDAC4 and/or HDAC8 enzyme activity:
(i) expressing each of the HDAC1, HDAC2, HDAC3, HDAC4, HDAC5, HDAC6, HDAC7 and HDAC8 genes to obtain respective enzyme solutions;
(ii) measuring HDAC enzyme activity for each of the enzyme solutions in the presence of a test substance;
(iii) selecting a test substance that selectively suppresses HDAC enzyme activity only when using an enzyme solution obtained by expressing the HDAC4 gene or the HDAC8 gene.

7. The method of claim 4, which is a method of selecting an immunosuppressant having low thrombocytopenic activity, and which comprises steps (i) to (iii) below, wherein the immunosuppressant selectively inhibits HDAC4 and/or HDAC8 enzyme activity:
(i) expressing each of the HDAC1, HDAC2, HDAC3, HDAC4, HDAC5, HDAC6, HDAC7 and HDAC8 genes to obtain respective enzyme solutions;
(ii) measuring HDAC enzyme activity for each of the enzyme solutions in the presence of a test substance;
(iii) selecting a test substance that selectively suppresses HDAC enzyme activity only when using an enzyme solution obtained by expressing the HDAC4 gene or the HDAC8 gene.

8. The method of claim 4, which is a method of selecting an immunosuppressant having low thrombocytopenic activity, and which comprises steps (i) to (iii) below, wherein the immunosuppressant selectively inhibits HDAC4 and/or HDAC8 enzyme activity:
(i) measuring HDAC enzyme activity for an enzyme solution partially purified from human cells, in the presence of a test substance;
(ii) measuring HDAC enzyme activity for an enzyme solution obtained by expressing the HDAC4 gene and/or the HDAC8 gene, in the presence of a test substance;
(iii) selecting a test substance that selectively suppresses HDAC enzyme activity only when using an enzyme solution obtained by expressing the HDAC4 gene and/or the HDAC8 gene, by comparing the enzyme activities measured in (i) and (ii).

9. A method of selecting an immunosuppressant having low thrombocytopenic activity, which comprises steps (i) to (iii) below, wherein an HDAC inhibitor that specifically inhibits the formation of a complex of HDAC4 and N-CoR is selected from among test HDAC inhibitors:
(i) expressing the HDAC4 gene and the N-CoR gene to obtain an enzyme solution;
(ii) allowing the enzyme solution to coexist with a test HDAC inhibitor;
(iii) determining whether or not a complex of HDAC4 and N-CoR is formed in the mixture of enzyme solution and test HDAC inhibitor obtained in (ii).

10. A method of selecting an immunosuppressant having low thrombocytopenic activity, which comprises steps (i) and (ii) below, wherein an HDAC inhibitor that specifically inhibits the formation of a complex of the HDAC4 fusion protein comprising at least a portion of the HDAC4 protein, and the N-CoR fusion protein comprising at least a portion of the N-CoR protein, is selected from among test HDAC inhibitors:
(i) expressing the gene that encodes the HDAC4 fusion protein and the gene that encodes the N-CoR fusion protein in cells;
(ii) determining whether or not the test HDAC inhibitor inhibits the formation of a complex of the HDAC4 fusion protein and the N-CoR fusion protein.

11. A method of selecting a compound having low thrombocytopenic activity and having immunosuppressive activity, which comprises steps (i) and (ii) below, wherein an HDAC inhibitor that suppresses the expression level of HDAC4 and/or HDAC8 is selected from among test HDAC inhibitors:
(i) allowing cells that express HDAC4 and/or HDAC8 to coexist with a test HDAC inhibitors;
(ii) measuring the HDAC4 and/or HDAC8 expression level in the cells.

12. A method of selecting a compound having low thrombocytopenic activity and having immunosuppressive activity, which comprises steps (i) and (ii) below, wherein an HDAC inhibitor that selectively inhibits the binding of HDAC4 and/or HDAC8 and an HDAC4- and/or HDAC8-specific ligand is selected from among test HDAC inhibitors:
(i) expressing the HDAC4 and/or HDAC8 gene to obtain an enzyme solution;
(ii) measuring the binding activity of the HDAC4 and/or HDAC8 enzyme and the HDAC4- and/or HDAC8-specific ligand for the enzyme solution in the presence of an HDAC4- and/or HDAC8-specific ligand and a test HDAC inhibitor.

13. An assay kit for the selection of an immunosuppressant having low thrombocytopenic activity including a selective HDAC4 and/or HDAC8 inhibitor, which includes at least (i) and (ii) below:
(i) enzyme solutions prepared by expressing each of the HDAC1, HDAC2, HDAC3, HDAC5, HDAC6 and HDAC7 genes;
(ii) an enzyme solution prepared by expressing each of the HDAC4 and/or HDAC8 gene;

14. A DNA having the base sequence that encodes the amino acid sequence shown by SEQ ID NO:4 or SEQ ID NO:6.

15. An HDAC4 variant that is the amino acid sequence shown by SEQ ID NO:4 or SEQ ID NO:6.

16. A method of inhibiting IL-2 production and/or immunocyte proliferation with low GATA-1 production inhibitory activity, which comprises administering an effective amount of selective HDAC4 and/or HDAC8 inhibitor to a subject.

17. A method of suppressing immunity with low thrombocytopenic activity, which comprises administering an effective amount of selective HDAC4 and/or HDAC8 inhibitor to a subject.

18. Use of a selective HDAC4 and/or HDAC8 inhibitor for the production of an agent for inhibiting IL-2 production and/or an agent for inhibiting immunocyte proliferation which have low GATA-1 production inhibitory activity.

19. Use of a selective HDAC4 and/or HDAC8 inhibitor for the production of an agent for suppressing immunity having low thrombocytopenic activity.


SEQUENCE LISTING

<110> Fujisawa Pharmaceutical Co., Ltd.

<120> New methods for selecting an immunosuppressive agent

<130> 09598

<150> JP2002-378800
<151> 2002-12-27

<160> 86

<170> PatentIn version 3.1

<210> 1
<211> 3255
<212> DNA
<213> Homo sapiens

<220>
<221> CDS
<222> (1)..(3255)
<223> human histone deacetylase-4 (HDAC4) gene

<400> 1
atg agc tcc caa agc cat cca gat gga ctt tct ggc cga gac cag cca 48
Met Ser Ser Gln Ser His Pro Asp Gly Leu Ser Gly Arg Asp Gln Pro
1               5                   10                  15

gtg gag ctg ctg aat cct gcc cgc gtg aac cac atg ccc agc acg gtg 96
Val Glu Leu Leu Asn Pro Ala Arg Val Asn His Met Pro Ser Thr Val
            20                  25                  30

gat gtg gcc acg gcg ctg cct ctg caa gtg gcc ccc tcg gca gtg ccc 144
Asp Val Ala Thr Ala Leu Pro Leu Gln Val Ala Pro Ser Ala Val Pro
        35                  40                  45

atg gac ctg cgc ctg gac cac cag ttc tca ctg cct gtg gca gag ccg 192
Met Asp Leu Arg Leu Asp His Gln Phe Ser Leu Pro Val Ala Glu Pro
    50                  55                  60

gcc ctg cgg gag cag cag ctg cag cag gag ctc ctg gcg ctc aag cag 240
Ala Leu Arg Glu Gln Gln Leu Gln Gln Glu Leu Leu Ala Leu Lys Gln
65                  70                  75                  80

aag cag cag atc cag agg cag atc ctc atc gct gag ttc cag agg cag 288
Lys Gln Gln Ile Gln Arg Gln Ile Leu Ile Ala Glu Phe Gln Arg Gln
                85                  90                  95

cac gag cag ctc tcc cgg cag cac gag gcg cag ctc cac gag cac atc 336
His Glu Gln Leu Ser Arg Gln His Glu Ala Gln Leu His Glu His Ile
            100                 105                 110

aag caa caa cag gag atg ctg gcc atg aag cac cag cag gag ctg ctg 384
Lys Gln Gln Gln Glu Met Leu Ala Met Lys His Gln Gln Glu Leu Leu
        115                 120                 125

gaa cac cag cgg aag ctg gag agg cac cgc cag gag cag gag ctg gag 432
Glu His Gln Arg Lys Leu Glu Arg His Arg Gln Glu Gln Glu Leu Glu
    130                 135                 140

aag cag cac cgg gag cag aag ctg cag cag ctc aag aac aag gag aag 480
Lys Gln His Arg Glu Gln Lys Leu Gln Gln Leu Lys Asn Lys Glu Lys
145                 150                 155                 160

ggc aaa gag agt gcc gtg gcc agc aca gaa gtg aag atg aag tta caa 528
Gly Lys Glu Ser Ala Val Ala Ser Thr Glu Val Lys Met Lys Leu Gln
                165                 170                 175

gaa ttt gtc ctc aat aaa aag aag gcg ctg gcc cac cgg aat ctg aac 576
Glu Phe Val Leu Asn Lys Lys Lys Ala Leu Ala His Arg Asn Leu Asn
            180                 185                 190

cac tgc att tcc agc gac cct cgc tac tgg tac ggg aaa acg cag cac 624

His Cys Ile Ser Ser Asp Pro Arg Tyr Trp Tyr Gly Lys Thr Gln His
        195                 200                 205

agt tcc ctt gac cag agt tct cca ccc cag agc gga gtg tcg acc tcc      672
Ser Ser Leu Asp Gln Ser Ser Pro Pro Gln Ser Gly Val Ser Thr Ser
    210                 215                 220

tat aac cac ccg gtc ctg gga atg tac gac gcc aaa gat gac ttc cct      720
Tyr Asn His Pro Val Leu Gly Met Tyr Asp Ala Lys Asp Asp Phe Pro
225                 230                 235                 240

ctt agg aaa aca gct tct gaa ccg aat ctg aaa tta cgg tcc agg cta      768
Leu Arg Lys Thr Ala Ser Glu Pro Asn Leu Lys Leu Arg Ser Arg Leu
                245                 250                 255

aag cag aaa gtg gcc gaa aga cgg agc agc ccc ctg tta cgc agg aaa      816
Lys Gln Lys Val Ala Glu Arg Arg Ser Ser Pro Leu Leu Arg Arg Lys
            260                 265                 270

gac ggg cca gtg gtc act gct cta aaa aag cgt ccg ttg gat gtc aca      864
Asp Gly Pro Val Val Thr Ala Leu Lys Lys Arg Pro Leu Asp Val Thr
        275                 280                 285

gac tcc gcg tgc agc agc gcc cca ggc tcc gga ccc agc tca ccc aac      912
Asp Ser Ala Cys Ser Ser Ala Pro Gly Ser Gly Pro Ser Ser Pro Asn
    290                 295                 300

aac agc tcc ggg agc gtc agc gcg gag aac ggt atc gcg ccc gcc gtc      960
Asn Ser Ser Gly Ser Val Ser Ala Glu Asn Gly Ile Ala Pro Ala Val
305                 310                 315                 320

ccc agc atc ccg gcg gag acg agt ttg gcg cac aga ctt gtg gca cga     1008
Pro Ser Ile Pro Ala Glu Thr Ser Leu Ala His Arg Leu Val Ala Arg
                325                 330                 335

gaa ggc tcg gcc gct cca ctt ccc ctc tac aca tcg cca tcc ttg ccc     1056
Glu Gly Ser Ala Ala Pro Leu Pro Leu Tyr Thr Ser Pro Ser Leu Pro
            340                 345                 350

aac atc acg ctg ggc ctg cct gcc acc ggc ccc tct gcg ggc acg gcg     1104
Asn Ile Thr Leu Gly Leu Pro Ala Thr Gly Pro Ser Ala Gly Thr Ala
        355                 360                 365

ggc cag cag gac acc gag aga ctc acc ctt ccc gcc ctc cag cag agg     1152
Gly Gln Gln Asp Thr Glu Arg Leu Thr Leu Pro Ala Leu Gln Gln Arg
    370                 375                 380

ctc tcc ctt ttc ccc ggc acc cac ctc act ccc tac ctg agc acc tcg     1200
Leu Ser Leu Phe Pro Gly Thr His Leu Thr Pro Tyr Leu Ser Thr Ser
385                 390                 395                 400

ccc ttg gag cgg gac gga ggg gca gcg cac agc cct ctt ctg cag cac     1248
Pro Leu Glu Arg Asp Gly Gly Ala Ala His Ser Pro Leu Leu Gln His
                405                 410                 415

atg gtc tta ctg gag cag cca ccg gca caa gca ccc ctc gtc aca ggc     1296
Met Val Leu Leu Glu Gln Pro Pro Ala Gln Ala Pro Leu Val Thr Gly
            420                 425                 430

ctg gga gca ctg ccc ctc cac gca cag tcc ttg gtt ggt gca gac cgg     1344
Leu Gly Ala Leu Pro Leu His Ala Gln Ser Leu Val Gly Ala Asp Arg
        435                 440                 445

gtg tcc ccc tcc atc cac aag ctg cgg cag cac cgc cca ctg ggg cgg     1392
Val Ser Pro Ser Ile His Lys Leu Arg Gln His Arg Pro Leu Gly Arg
    450                 455                 460

acc cag tcg gcc ccg ctg ccc cag aac gcc cag gct ctg cag cac ctg     1440
Thr Gln Ser Ala Pro Leu Pro Gln Asn Ala Gln Ala Leu Gln His Leu
465                 470                 475                 480

gtc atc cag cag cag cat cag cag ttt ctg gag aaa cac aag cag cag     1488
Val Ile Gln Gln Gln His Gln Gln Phe Leu Glu Lys His Lys Gln Gln
                485                 490                 495

ttc cag cag cag caa ctg cag atg aac aag atc atc ccc aag cca agc 1536
Phe Gln Gln Gln Gln Leu Gln Met Asn Lys Ile Ile Pro Lys Pro Ser
            500                 505                 510

gag cca gcc cgg cag ccg gag agc cac ccg gag gag acg gag gag gag 1584
Glu Pro Ala Arg Gln Pro Glu Ser His Pro Glu Glu Thr Glu Glu Glu
        515                 520                 525

ctc cgt gag cac cag gct ctg ctg gac gag ccc tac ctg gac cgg ctg 1632
Leu Arg Glu His Gln Ala Leu Leu Asp Glu Pro Tyr Leu Asp Arg Leu
    530                 535                 540

ccg ggg cag aag gag gcg cac gca cag gcc ggc gtg cag gtg aag cag 1680
Pro Gly Gln Lys Glu Ala His Ala Gln Ala Gly Val Gln Val Lys Gln
545                 550                 555                 560

gag ccc att gag agc gat gag gaa gag gca gag ccc cca cgg gag gtg 1728
Glu Pro Ile Glu Ser Asp Glu Glu Glu Ala Glu Pro Pro Arg Glu Val
                565                 570                 575

gag ccg ggc cag cgc cag ccc agt gag cag gag ctg ctc ttc aga cag 1776
Glu Pro Gly Gln Arg Gln Pro Ser Glu Gln Glu Leu Leu Phe Arg Gln
            580                 585                 590

caa gcc ctc ctg ctg gag cag cag cgg atc cac cag ctg agg aac tac 1824
Gln Ala Leu Leu Leu Glu Gln Gln Arg Ile His Gln Leu Arg Asn Tyr
        595                 600                 605

cag gcg tcc atg gag gcc gcc ggc atc ccc gtg tcc ttc ggc ggc cac 1872
Gln Ala Ser Met Glu Ala Ala Gly Ile Pro Val Ser Phe Gly Gly His
    610                 615                 620

agg cct ctg tcc cgg gcg cag tcc tca ccc gcg tct gcc acc ttc ccc 1920
Arg Pro Leu Ser Arg Ala Gln Ser Ser Pro Ala Ser Ala Thr Phe Pro
625                 630                 635                 640

gtg tct gtg cag gag ccc ccc acc aag ccg agg ttc acg aca ggc ctc 1968
Val Ser Val Gln Glu Pro Pro Thr Lys Pro Arg Phe Thr Thr Gly Leu
                645                 650                 655

gtg tat gac acg ctg atg ctg aag cac cag tgc acc tgc ggg agt agc 2016
Val Tyr Asp Thr Leu Met Leu Lys His Gln Cys Thr Cys Gly Ser Ser
            660                 665                 670

agc agc cac ccc gag cac gcc ggg agg atc cag agc atc tgg tcc cgc 2064
Ser Ser His Pro Glu His Ala Gly Arg Ile Gln Ser Ile Trp Ser Arg
        675                 680                 685

ctg cag gag acg ggc ctc cgg ggc aaa tgc gag tgc atc cgc gga cgc 2112
Leu Gln Glu Thr Gly Leu Arg Gly Lys Cys Glu Cys Ile Arg Gly Arg
    690                 695                 700

aag gcc acc ctg gag gag cta cag acg gtg cac tcg gaa gcc cac acc 2160
Lys Ala Thr Leu Glu Glu Leu Gln Thr Val His Ser Glu Ala His Thr
705                 710                 715                 720

ctc ctg tat ggc acg aac ccc ctc aac cgg cag aaa ctg gac agt aag 2208
Leu Leu Tyr Gly Thr Asn Pro Leu Asn Arg Gln Lys Leu Asp Ser Lys
                725                 730                 735

aaa ctt cta ggc tcg ctc gcc tcc gtg ttc gtc cgg ctc cct tgc ggt 2256
Lys Leu Leu Gly Ser Leu Ala Ser Val Phe Val Arg Leu Pro Cys Gly
            740                 745                 750

ggt gtt ggg gtg gac agt gac acc ata tgg aac gag gtg cac tcg gcg 2304
Gly Val Gly Val Asp Ser Asp Thr Ile Trp Asn Glu Val His Ser Ala
        755                 760                 765

ggg gca gcc cgc ctg gct gtg ggc tgc gtg gta gag ctg gtc ttc aag 2352
Gly Ala Ala Arg Leu Ala Val Gly Cys Val Val Glu Leu Val Phe Lys
    770                 775                 780

gtg gcc aca ggg gag ctg aag aat ggc ttt gct gtg gtc cgc ccc cct 2400
Val Ala Thr Gly Glu Leu Lys Asn Gly Phe Ala Val Val Arg Pro Pro
785                 790                 795                 800

gga cac cat gcg gag gag agc acg ccc atg ggc ttt tgc tac ttc aac 2448
Gly His His Ala Glu Glu Ser Thr Pro Met Gly Phe Cys Tyr Phe Asn
                805                 810                 815

tcc gtg gcc gtg gca gcc aag ctt ctg cag cag agg ttg agc gtg agc 2496
Ser Val Ala Val Ala Ala Lys Leu Leu Gln Gln Arg Leu Ser Val Ser
            820                 825                 830

aag atc ctc atc gtg gac tgg gac gtg cac cat gga aac ggg acc cag 2544
Lys Ile Leu Ile Val Asp Trp Asp Val His His Gly Asn Gly Thr Gln
        835                 840                 845

cag gct ttc tac agc gac cct agc gtc ctg tac atg tcc ctc cac cgc 2592
Gln Ala Phe Tyr Ser Asp Pro Ser Val Leu Tyr Met Ser Leu His Arg
    850                 855                 860

tac gac gat ggg aac ttc ttc cca ggc agc ggg gct cct gat gag gtg 2640
Tyr Asp Asp Gly Asn Phe Phe Pro Gly Ser Gly Ala Pro Asp Glu Val
865                 870                 875                 880

ggc aca ggg ccc ggc gtg ggt ttc aac gtc aac atg gct ttc acc ggc 2688
Gly Thr Gly Pro Gly Val Gly Phe Asn Val Asn Met Ala Phe Thr Gly
                885                 890                 895

ggc ctg gac ccc ccc atg gga gac gct gag tac ttg gcg gcc ttc aga 2736
Gly Leu Asp Pro Pro Met Gly Asp Ala Glu Tyr Leu Ala Ala Phe Arg
            900                 905                 910

acg gtg gtc atg ccg atc gcc agc gag ttt gcc ccg gat gtg gtg ctg 2784
Thr Val Val Met Pro Ile Ala Ser Glu Phe Ala Pro Asp Val Val Leu
        915                 920                 925

gtg tca tca ggc ttc gat gcc gtg gag ggc cac ccc acc cct ctt ggg 2832
Val Ser Ser Gly Phe Asp Ala Val Glu Gly His Pro Thr Pro Leu Gly
    930                 935                 940

ggc tac aac ctc tcc gcc aga tgc ttc ggg tac ctg acg aag cag ctg 2880
Gly Tyr Asn Leu Ser Ala Arg Cys Phe Gly Tyr Leu Thr Lys Gln Leu
945                 950                 955                 960

atg ggc ctg gct ggc ggc cgg att gtc ctg gcc ctc gag gga ggc cac 2928
Met Gly Leu Ala Gly Gly Arg Ile Val Leu Ala Leu Glu Gly Gly His
                965                 970                 975

gac ctg acc gcc att tgc gac gcc tcg gaa gca tgt gtt tct gcc ttg 2976
Asp Leu Thr Ala Ile Cys Asp Ala Ser Glu Ala Cys Val Ser Ala Leu
            980                 985                 990

ctg gga aac gag ctt gat cct ctc cca gaa aag gtt tta cag caa aga 3024
Leu Gly Asn Glu Leu Asp Pro Leu Pro Glu Lys Val Leu Gln Gln Arg
        995                 1000                1005

ccc aat gca aac gct gtc cgt tcc atg gag aaa gtc atg gag atc cac 3072
Pro Asn Ala Asn Ala Val Arg Ser Met Glu Lys Val Met Glu Ile His
    1010                1015                1020

agc aag tac tgg cgc tgc ctg cag cgc aca acc tcc aca gcg ggg cgt 3120
Ser Lys Tyr Trp Arg Cys Leu Gln Arg Thr Thr Ser Thr Ala Gly Arg
1025                1030                1035                1040

tct ctg atc gag gct cag act tgc gag aac gaa gaa gcc gag acg gtc 3168
Ser Leu Ile Glu Ala Gln Thr Cys Glu Asn Glu Glu Ala Glu Thr Val
                1045                1050                1055

acc gcc atg gcc tcg ctg tcc gtg ggc gtg aag ccc gcc gaa aag aga 3216
Thr Ala Met Ala Ser Leu Ser Val Gly Val Lys Pro Ala Glu Lys Arg
            1060                1065                1070

cca gat gag gag ccc atg gaa gag gag ccg ccc ctg tag 3255
Pro Asp Glu Glu Pro Met Glu Glu Glu Pro Pro Leu
        1075                1080

<210> 2

<211> 1084
<212> PRT
<213> Homo sapiens

<400> 2
Met Ser Ser Gln Ser His Pro Asp Gly Leu Ser Gly Arg Asp Gln Pro
1               5                   10                  15

Val Glu Leu Leu Asn Pro Ala Arg Val Asn His Met Pro Ser Thr Val
            20                  25                  30

Asp Val Ala Thr Ala Leu Pro Leu Gln Val Ala Pro Ser Ala Val Pro
        35                  40                  45

Met Asp Leu Arg Leu Asp His Gln Phe Ser Leu Pro Val Ala Glu Pro
    50                  55                  60

Ala Leu Arg Glu Gln Gln Leu Gln Gln Glu Leu Leu Ala Leu Lys Gln
65                  70                  75                  80

Lys Gln Gln Ile Gln Arg Gln Ile Leu Ile Ala Glu Phe Gln Arg Gln
                85                  90                  95

His Glu Gln Leu Ser Arg Gln His Glu Ala Gln Leu His Glu His Ile
            100                 105                 110

Lys Gln Gln Gln Glu Met Leu Ala Met Lys His Gln Gln Glu Leu Leu
        115                 120                 125

Glu His Gln Arg Lys Leu Glu Arg His Arg Gln Glu Gln Glu Leu Glu
    130                 135                 140

Lys Gln His Arg Glu Gln Lys Leu Gln Gln Leu Lys Asn Lys Glu Lys
145                 150                 155                 160

Gly Lys Glu Ser Ala Val Ala Ser Thr Glu Val Lys Met Lys Leu Gln
                165                 170                 175

Glu Phe Val Leu Asn Lys Lys Lys Ala Leu Ala His Arg Asn Leu Asn
            180                 185                 190

His Cys Ile Ser Ser Asp Pro Arg Tyr Trp Tyr Gly Lys Thr Gln His
        195                 200                 205

Ser Ser Leu Asp Gln Ser Ser Pro Pro Gln Ser Gly Val Ser Thr Ser
    210                 215                 220

Tyr Asn His Pro Val Leu Gly Met Tyr Asp Ala Lys Asp Asp Phe Pro
225                 230                 235                 240

Leu Arg Lys Thr Ala Ser Glu Pro Asn Leu Lys Leu Arg Ser Arg Leu
                245                 250                 255

Lys Gln Lys Val Ala Glu Arg Arg Ser Ser Pro Leu Leu Arg Arg Lys
            260                 265                 270

Asp Gly Pro Val Val Thr Ala Leu Lys Lys Arg Pro Leu Asp Val Thr
        275                 280                 285

Asp Ser Ala Cys Ser Ser Ala Pro Gly Ser Gly Pro Ser Ser Pro Asn
    290                 295                 300

Asn Ser Ser Gly Ser Val Ser Ala Glu Asn Gly Ile Ala Pro Ala Val
305                 310                 315                 320

Pro Ser Ile Pro Ala Glu Thr Ser Leu Ala His Arg Leu Val Ala Arg
                325                 330                 335

Glu Gly Ser Ala Ala Pro Leu Pro Leu Tyr Thr Ser Pro Ser Leu Pro
            340                 345                 350

Asn Ile Thr Leu Gly Leu Pro Ala Thr Gly Pro Ser Ala Gly Thr Ala
        355                 360                 365

Gly Gln Gln Asp Thr Glu Arg Leu Thr Leu Pro Ala Leu Gln Gln Arg

370                 375                 380

Leu Ser Leu Phe Pro Gly Thr His Leu Thr Pro Tyr Leu Ser Thr Ser
385                 390                 395                 400

Pro Leu Glu Arg Asp Gly Gly Ala Ala His Ser Pro Leu Leu Gln His
                405                 410                 415

Met Val Leu Leu Glu Gln Pro Pro Ala Gln Ala Pro Leu Val Thr Gly
            420                 425                 430

Leu Gly Ala Leu Pro Leu His Ala Gln Ser Leu Val Gly Ala Asp Arg
        435                 440                 445

Val Ser Pro Ser Ile His Lys Leu Arg Gln His Arg Pro Leu Gly Arg
    450                 455                 460

Thr Gln Ser Ala Pro Leu Pro Gln Asn Ala Gln Ala Leu Gln His Leu
465                 470                 475                 480

Val Ile Gln Gln Gln His Gln Gln Phe Leu Glu Lys His Lys Gln Gln
                485                 490                 495

Phe Gln Gln Gln Gln Leu Gln Met Asn Lys Ile Ile Pro Lys Pro Ser
            500                 505                 510

Glu Pro Ala Arg Gln Pro Glu Ser His Pro Glu Glu Thr Glu Glu Glu
        515                 520                 525

Leu Arg Glu His Gln Ala Leu Leu Asp Glu Pro Tyr Leu Asp Arg Leu
    530                 535                 540

Pro Gly Gln Lys Glu Ala His Ala Gln Ala Gly Val Gln Val Lys Gln
545                 550                 555                 560

Glu Pro Ile Glu Ser Asp Glu Glu Glu Ala Glu Pro Pro Arg Glu Val
                565                 570                 575

Glu Pro Gly Gln Arg Gln Pro Ser Glu Gln Glu Leu Leu Phe Arg Gln
            580                 585                 590

Gln Ala Leu Leu Leu Glu Gln Gln Arg Ile His Gln Leu Arg Asn Tyr
        595                 600                 605

Gln Ala Ser Met Glu Ala Ala Gly Ile Pro Val Ser Phe Gly Gly His
    610                 615                 620

Arg Pro Leu Ser Arg Ala Gln Ser Ser Pro Ala Ser Ala Thr Phe Pro
625                 630                 635                 640

Val Ser Val Gln Glu Pro Pro Thr Lys Pro Arg Phe Thr Thr Gly Leu
                645                 650                 655

Val Tyr Asp Thr Leu Met Leu Lys His Gln Cys Thr Cys Gly Ser Ser
            660                 665                 670

Ser Ser His Pro Glu His Ala Gly Arg Ile Gln Ser Ile Trp Ser Arg
        675                 680                 685

Leu Gln Glu Thr Gly Leu Arg Gly Lys Cys Glu Cys Ile Arg Gly Arg
    690                 695                 700

Lys Ala Thr Leu Glu Glu Leu Gln Thr Val His Ser Glu Ala His Thr
705                 710                 715                 720

Leu Leu Tyr Gly Thr Asn Pro Leu Asn Arg Gln Lys Leu Asp Ser Lys
                725                 730                 735

Lys Leu Leu Gly Ser Leu Ala Ser Val Phe Val Arg Leu Pro Cys Gly
            740                 745                 750

Gly Val Gly Val Asp Ser Asp Thr Ile Trp Asn Glu Val His Ser Ala
        755                 760                 765

Gly Ala Ala Arg Leu Ala Val Gly Cys Val Val Glu Leu Val Phe Lys

770                 775                 780

Val Ala Thr Gly Glu Leu Lys Asn Gly Phe Ala Val Val Arg Pro Pro
785                 790                 795                 800

Gly His His Ala Glu Glu Ser Thr Pro Met Gly Phe Cys Tyr Phe Asn
                805                 810                 815

Ser Val Ala Val Ala Ala Lys Leu Leu Gln Gln Arg Leu Ser Val Ser
            820                 825                 830

Lys Ile Leu Ile Val Asp Trp Asp Val His His Gly Asn Gly Thr Gln
        835                 840                 845

Gln Ala Phe Tyr Ser Asp Pro Ser Val Leu Tyr Met Ser Leu His Arg
    850                 855                 860

Tyr Asp Asp Gly Asn Phe Phe Pro Gly Ser Gly Ala Pro Asp Glu Val
865                 870                 875                 880

Gly Thr Gly Pro Gly Val Gly Phe Asn Val Asn Met Ala Phe Thr Gly
                885                 890                 895

Gly Leu Asp Pro Pro Met Gly Asp Ala Glu Tyr Leu Ala Ala Phe Arg
            900                 905                 910

Thr Val Val Met Pro Ile Ala Ser Glu Phe Ala Pro Asp Val Val Leu
        915                 920                 925

Val Ser Ser Gly Phe Asp Ala Val Glu Gly His Pro Thr Pro Leu Gly
    930                 935                 940

Gly Tyr Asn Leu Ser Ala Arg Cys Phe Gly Tyr Leu Thr Lys Gln Leu
945                 950                 955                 960

Met Gly Leu Ala Gly Gly Arg Ile Val Leu Ala Leu Glu Gly Gly His
                965                 970                 975

Asp Leu Thr Ala Ile Cys Asp Ala Ser Glu Ala Cys Val Ser Ala Leu
            980                 985                 990

Leu Gly Asn Glu Leu Asp Pro Leu Pro Glu Lys Val Leu Gln Gln Arg
        995                 1000                1005

Pro Asn Ala Asn Ala Val Arg Ser Met Glu Lys Val Met Glu Ile His
    1010                1015                1020

Ser Lys Tyr Trp Arg Cys Leu Gln Arg Thr Thr Ser Thr Ala Gly Arg
1025                1030                1035                1040

Ser Leu Ile Glu Ala Gln Thr Cys Glu Asn Glu Glu Ala Glu Thr Val
                1045                1050                1055

Thr Ala Met Ala Ser Leu Ser Val Gly Val Lys Pro Ala Glu Lys Arg
            1060                1065                1070

Pro Asp Glu Glu Pro Met Glu Glu Glu Pro Pro Leu
        1075                1080


<210>  3
<211>  3255
<212>  DNA
<213>  Artificial

<220>
<221>  CDS
<222>  (1)..(3255)
<223>  Dominant negative mutant (H802K, H803L) of human histone deacetylase-4
        (HDAC4) gene

<400>  3
atg agc tcc caa agc cat cca gat gga ctt tct ggc cga gac cag cca        48
Met Ser Ser Gln Ser His Pro Asp Gly Leu Ser Gly Arg Asp Gln Pro
1               5                   10                  15

gtg gag ctg ctg aat cct gcc cgc gtg aac cac atg ccc agc acg gtg     96
Val Glu Leu Leu Asn Pro Ala Arg Val Asn His Met Pro Ser Thr Val
            20                  25                  30

gat gtg gcc acg gcg ctg cct ctg caa gtg gcc ccc tcg gca gtg ccc     144
Asp Val Ala Thr Ala Leu Pro Leu Gln Val Ala Pro Ser Ala Val Pro
        35                  40                  45

atg gac ctg cgc ctg gac cac cag ttc tca ctg cct gtg gca gag ccg     192
Met Asp Leu Arg Leu Asp His Gln Phe Ser Leu Pro Val Ala Glu Pro
    50                  55                  60

gcc ctg cgg gag cag cag ctg cag cag gag ctc ctg gcg ctc aag cag     240
Ala Leu Arg Glu Gln Gln Leu Gln Gln Glu Leu Leu Ala Leu Lys Gln
65                  70                  75                  80

aag cag cag atc cag agg cag atc ctc atc gct gag ttc cag agg cag     288
Lys Gln Gln Ile Gln Arg Gln Ile Leu Ile Ala Glu Phe Gln Arg Gln
                85                  90                  95

cac gag cag ctc tcc cgg cag cac gag gcg cag ctc cac gag cac atc     336
His Glu Gln Leu Ser Arg Gln His Glu Ala Gln Leu His Glu His Ile
            100                 105                 110

aag caa caa cag gag atg ctg gcc atg aag cac cag cag gag ctg ctg     384
Lys Gln Gln Gln Glu Met Leu Ala Met Lys His Gln Gln Glu Leu Leu
        115                 120                 125

gaa cac cag cgg aag ctg gag agg cac cgc cag gag cag gag ctg gag     432
Glu His Gln Arg Lys Leu Glu Arg His Arg Gln Glu Gln Glu Leu Glu
    130                 135                 140

aag cag cac cgg gag cag aag ctg cag cag ctc aag aac aag gag aag     480
Lys Gln His Arg Glu Gln Lys Leu Gln Gln Leu Lys Asn Lys Glu Lys
145                 150                 155                 160

ggc aaa gag agt gcc gtg gcc agc aca gaa gtg aag atg aag tta caa     528
Gly Lys Glu Ser Ala Val Ala Ser Thr Glu Val Lys Met Lys Leu Gln
                165                 170                 175

gaa ttt gtc ctc aat aaa aag aag gcg ctg gcc cac cgg aat ctg aac     576
Glu Phe Val Leu Asn Lys Lys Lys Ala Leu Ala His Arg Asn Leu Asn
            180                 185                 190

cac tgc att tcc agc gac cct cgc tac tgg tac ggg aaa acg cag cac     624
His Cys Ile Ser Ser Asp Pro Arg Tyr Trp Tyr Gly Lys Thr Gln His
        195                 200                 205

agt tcc ctt gac cag agt tct cca ccc cag agc gga gtg tcg acc tcc     672
Ser Ser Leu Asp Gln Ser Ser Pro Pro Gln Ser Gly Val Ser Thr Ser
    210                 215                 220

tat aac cac ccg gtc ctg gga atg tac gac gcc aaa gat gac ttc cct     720
Tyr Asn His Pro Val Leu Gly Met Tyr Asp Ala Lys Asp Asp Phe Pro
225                 230                 235                 240

ctt agg aaa aca gct tct gaa ccg aat ctg aaa tta cgg tcc agg cta     768
Leu Arg Lys Thr Ala Ser Glu Pro Asn Leu Lys Leu Arg Ser Arg Leu
                245                 250                 255

aag cag aaa gtg gcc gaa aga cgg agc agc ccc ctg tta cgc agg aaa     816
Lys Gln Lys Val Ala Glu Arg Arg Ser Ser Pro Leu Leu Arg Arg Lys
            260                 265                 270

gac ggg cca gtg gtc act gct cta aaa aag cgt ccg ttg gat gtc aca     864
Asp Gly Pro Val Val Thr Ala Leu Lys Lys Arg Pro Leu Asp Val Thr
        275                 280                 285

gac tcc gcg tgc agc agc gcc cca ggc tcc gga ccc agc tca ccc aac     912
Asp Ser Ala Cys Ser Ser Ala Pro Gly Ser Gly Pro Ser Ser Pro Asn
    290                 295                 300

aac agc tcc ggg agc gtc agc gcg gag aac ggt atc gcg ccc gcc gtc     960
Asn Ser Ser Gly Ser Val Ser Ala Glu Asn Gly Ile Ala Pro Ala Val

305                 310                 315                 320

ccc agc atc ccg gcg gag acg agt ttg gcg cac aga ctt gtg gca cga     1008
Pro Ser Ile Pro Ala Glu Thr Ser Leu Ala His Arg Leu Val Ala Arg
                325                 330                 335

gaa ggc tcg gcc gct cca ctt ccc ctc tac aca tcg cca tcc ttg ccc     1056
Glu Gly Ser Ala Ala Pro Leu Pro Leu Tyr Thr Ser Pro Ser Leu Pro
            340                 345                 350

aac atc acg ctg ggc ctg cct gcc acc ggc ccc tct gcg ggc acg gcg     1104
Asn Ile Thr Leu Gly Leu Pro Ala Thr Gly Pro Ser Ala Gly Thr Ala
        355                 360                 365

ggc cag cag gac acc gag aga ctc acc ctt ccc gcc ctc cag cag agg     1152
Gly Gln Gln Asp Thr Glu Arg Leu Thr Leu Pro Ala Leu Gln Gln Arg
    370                 375                 380

ctc tcc ctt ttc ccc ggc acc cac ctc act ccc tac ctg agc acc tcg     1200
Leu Ser Leu Phe Pro Gly Thr His Leu Thr Pro Tyr Leu Ser Thr Ser
385                 390                 395                 400

ccc ttg gag cgg gac gga ggg gca gcg cac agc cct ctt ctg cag cac     1248
Pro Leu Glu Arg Asp Gly Gly Ala Ala His Ser Pro Leu Leu Gln His
                405                 410                 415

atg gtc tta ctg gag cag cca ccg gca caa gca ccc ctc gtc aca ggc     1296
Met Val Leu Leu Glu Gln Pro Pro Ala Gln Ala Pro Leu Val Thr Gly
            420                 425                 430

ctg gga gca ctg ccc ctc cac gca cag tcc ttg gtt ggt gca gac cgg     1344
Leu Gly Ala Leu Pro Leu His Ala Gln Ser Leu Val Gly Ala Asp Arg
        435                 440                 445

gtg tcc ccc tcc atc cac aag ctg cgg cag cac cgc cca ctg ggg cgg     1392
Val Ser Pro Ser Ile His Lys Leu Arg Gln His Arg Pro Leu Gly Arg
    450                 455                 460

acc cag tcg gcc ccg ctg ccc cag aac gcc cag gct ctg cag cac ctg     1440
Thr Gln Ser Ala Pro Leu Pro Gln Asn Ala Gln Ala Leu Gln His Leu
465                 470                 475                 480

gtc atc cag cag cag cat cag cag ttt ctg gag aaa cac aag cag cag     1488
Val Ile Gln Gln Gln His Gln Gln Phe Leu Glu Lys His Lys Gln Gln
                485                 490                 495

ttc cag cag cag caa ctg cag atg aac aag atc atc ccc aag cca agc     1536
Phe Gln Gln Gln Gln Leu Gln Met Asn Lys Ile Ile Pro Lys Pro Ser
            500                 505                 510

gag cca gcc cgg cag ccg gag agc cac ccg gag gag acg gag gag gag     1584
Glu Pro Ala Arg Gln Pro Glu Ser His Pro Glu Glu Thr Glu Glu Glu
        515                 520                 525

ctc cgt gag cac cag gct ctg ctg gac gag ccc tac ctg gac cgg ctg     1632
Leu Arg Glu His Gln Ala Leu Leu Asp Glu Pro Tyr Leu Asp Arg Leu
    530                 535                 540

ccg ggg cag aag gag gcg cac gca cag gcc ggc gtg cag gtg aag cag     1680
Pro Gly Gln Lys Glu Ala His Ala Gln Ala Gly Val Gln Val Lys Gln
545                 550                 555                 560

gag ccc att gag agc gat gag gaa gag gca gag ccc cca cgg gag gtg     1728
Glu Pro Ile Glu Ser Asp Glu Glu Glu Ala Glu Pro Pro Arg Glu Val
                565                 570                 575

gag ccg ggc cag cgc cag ccc agt gag cag gag ctg ctc ttc aga cag     1776
Glu Pro Gly Gln Arg Gln Pro Ser Glu Gln Glu Leu Leu Phe Arg Gln
            580                 585                 590

caa gcc ctc ctg ctg gag cag cag cgg atc cac cag ctg agg aac tac     1824
Gln Ala Leu Leu Leu Glu Gln Gln Arg Ile His Gln Leu Arg Asn Tyr
        595                 600                 605

cag gcg tcc atg gag gcc gcc ggc atc ccc gtg tcc ttc ggc ggc cac     1872

Gln Ala Ser Met Glu Ala Ala Gly Ile Pro Val Ser Phe Gly Gly His
    610                 615                 620

agg cct ctg tcc cgg gcg cag tcc tca ccc gcg tct gcc acc ttc ccc 1920
Arg Pro Leu Ser Arg Ala Gln Ser Ser Pro Ala Ser Ala Thr Phe Pro
625                 630                 635                 640

gtg tct gtg cag gag ccc ccc acc aag ccg agg ttc acg aca ggc ctc 1968
Val Ser Val Gln Glu Pro Pro Thr Lys Pro Arg Phe Thr Thr Gly Leu
                645                 650                 655

gtg tat gac acg ctg atg ctg aag cac cag tgc acc tgc ggg agt agc 2016
Val Tyr Asp Thr Leu Met Leu Lys His Gln Cys Thr Cys Gly Ser Ser
            660                 665                 670

agc agc cac ccc gag cac gcc ggg agg atc cag agc atc tgg tcc cgc 2064
Ser Ser His Pro Glu His Ala Gly Arg Ile Gln Ser Ile Trp Ser Arg
        675                 680                 685

ctg cag gag acg ggc ctc cgg ggc aaa tgc gag tgc atc cgc gga cgc 2112
Leu Gln Glu Thr Gly Leu Arg Gly Lys Cys Glu Cys Ile Arg Gly Arg
    690                 695                 700

aag gcc acc ctg gag gag cta cag acg gtg cac tcg gaa gcc cac acc 2160
Lys Ala Thr Leu Glu Glu Leu Gln Thr Val His Ser Glu Ala His Thr
705                 710                 715                 720

ctc ctg tat ggc acg aac ccc ctc aac cgg cag aaa ctg gac agt aag 2208
Leu Leu Tyr Gly Thr Asn Pro Leu Asn Arg Gln Lys Leu Asp Ser Lys
                725                 730                 735

aaa ctt cta ggc tcg ctc gcc tcc gtg ttc gtc cgg ctc cct tgc ggt 2256
Lys Leu Leu Gly Ser Leu Ala Ser Val Phe Val Arg Leu Pro Cys Gly
            740                 745                 750

ggt gtt ggg gtg gac agt gac acc ata tgg aac gag gtg cac tcg gcg 2304
Gly Val Gly Val Asp Ser Asp Thr Ile Trp Asn Glu Val His Ser Ala
        755                 760                 765

ggg gca gcc cgc ctg gct gtg ggc tgc gtg gta gag ctg gtc ttc aag 2352
Gly Ala Ala Arg Leu Ala Val Gly Cys Val Val Glu Leu Val Phe Lys
    770                 775                 780

gtg gcc aca ggg gag ctg aag aat ggc ttt gct gtg gtc cgc ccc cct 2400
Val Ala Thr Gly Glu Leu Lys Asn Gly Phe Ala Val Val Arg Pro Pro
785                 790                 795                 800

gga aag ctt gcg gag gag agc acg ccc atg ggc ttt tgc tac ttc aac 2448
Gly Lys Leu Ala Glu Glu Ser Thr Pro Met Gly Phe Cys Tyr Phe Asn
                805                 810                 815

tcc gtg gcc gtg gca gcc aag ctt ctg cag cag agg ttg agc gtg agc 2496
Ser Val Ala Val Ala Ala Lys Leu Leu Gln Gln Arg Leu Ser Val Ser
            820                 825                 830

aag atc ctc atc gtg gac tgg gac gtg cac cat gga aac ggg acc cag 2544
Lys Ile Leu Ile Val Asp Trp Asp Val His His Gly Asn Gly Thr Gln
        835                 840                 845

cag gct ttc tac agc gac cct agc gtc ctg tac atg tcc ctc cac cgc 2592
Gln Ala Phe Tyr Ser Asp Pro Ser Val Leu Tyr Met Ser Leu His Arg
    850                 855                 860

tac gac gat ggg aac ttc ttc cca ggc agc ggg gct cct gat gag gtg 2640
Tyr Asp Asp Gly Asn Phe Phe Pro Gly Ser Gly Ala Pro Asp Glu Val
865                 870                 875                 880

ggc aca ggg ccc ggc gtg ggt ttc aac gtc aac atg gct ttc acc ggc 2688
Gly Thr Gly Pro Gly Val Gly Phe Asn Val Asn Met Ala Phe Thr Gly
                885                 890                 895

ggc ctg gac ccc ccc atg gga gac gct gag tac ttg gcg gcc ttc aga 2736
Gly Leu Asp Pro Pro Met Gly Asp Ala Glu Tyr Leu Ala Ala Phe Arg
            900                 905                 910

acg gtg gtc atg ccg atc gcc agc gag ttt gcc ccg gat gtg gtg ctg 2784
Thr Val Val Met Pro Ile Ala Ser Glu Phe Ala Pro Asp Val Val Leu
        915                 920                 925

gtg tca tca ggc ttc gat gcc gtg gag ggc cac ccc acc cct ctt ggg 2832
Val Ser Ser Gly Phe Asp Ala Val Glu Gly His Pro Thr Pro Leu Gly
    930                 935                 940

ggc tac aac ctc tcc gcc aga tgc ttc ggg tac ctg acg aag cag ctg 2880
Gly Tyr Asn Leu Ser Ala Arg Cys Phe Gly Tyr Leu Thr Lys Gln Leu
945                 950                 955                 960

atg ggc ctg gct ggc ggc cgg att gtc ctg gcc ctc gag gga ggc cac 2928
Met Gly Leu Ala Gly Gly Arg Ile Val Leu Ala Leu Glu Gly Gly His
                965                 970                 975

gac ctg acc gcc att tgc gac gcc tcg gaa gca tgt gtt tct gcc ttg 2976
Asp Leu Thr Ala Ile Cys Asp Ala Ser Glu Ala Cys Val Ser Ala Leu
            980                 985                 990

ctg gga aac gag ctt gat cct ctc cca gaa aag gtt tta cag caa aga 3024
Leu Gly Asn Glu Leu Asp Pro Leu Pro Glu Lys Val Leu Gln Gln Arg
        995                 1000                1005

ccc aat gca aac gct gtc cgt tcc atg gag aaa gtc atg gag atc cac 3072
Pro Asn Ala Asn Ala Val Arg Ser Met Glu Lys Val Met Glu Ile His
    1010                1015                1020

agc aag tac tgg cgc tgc ctg cag cgc aca acc tcc aca gcg tct ctg 3120
Ser Lys Tyr Trp Arg Cys Leu Gln Arg Thr Thr Ser Thr Ala Gly Arg
1025                1030                1035                1040

tct ctg atc gag gct cag act tgc gag aac gaa gaa gcc gag acg gtc 3168
Ser Leu Ile Glu Ala Gln Thr Cys Glu Asn Glu Glu Ala Glu Thr Val
                1045                1050                1055

acc gcc atg gcc tcg ctg tcc gtg ggc gtg aag ccc gcc gaa aag aga 3216
Thr Ala Met Ala Ser Leu Ser Val Gly Val Lys Pro Ala Glu Lys Arg
            1060                1065                1070

cca gat gag gag ccc atg gaa gag gag ccg ccc ctg tag 3255
Pro Asp Glu Glu Pro Met Glu Glu Glu Pro Pro Leu
        1075                1080

<210> 4
<211> 1084
<212> PRT
<213> Artificial

<220>
<223> Dominant negative mutant (H802K, H803L) of human histone deacetylase-4
(HDAC4) gene

<400> 4
Met Ser Ser Gln Ser His Pro Asp Gly Leu Ser Gly Arg Asp Gln Pro
1               5                   10                  15

Val Glu Leu Leu Asn Pro Ala Arg Val Asn His Met Pro Ser Thr Val
            20                  25                  30

Asp Val Ala Thr Ala Leu Pro Leu Gln Val Ala Pro Ser Ala Val Pro
        35                  40                  45

Met Asp Leu Arg Leu Asp His Gln Phe Ser Leu Pro Val Ala Glu Pro
    50                  55                  60

Ala Leu Arg Glu Gln Gln Leu Gln Gln Glu Leu Leu Ala Leu Lys Gln
65                  70                  75                  80

Lys Gln Gln Ile Gln Arg Gln Ile Leu Ile Ala Glu Phe Gln Arg Gln
                85                  90                  95

His Glu Gln Leu Ser Arg Gln His Glu Ala Gln Leu His Glu His Ile
            100                 105                 110

Lys Gln Gln Gln Glu Met Leu Ala Met Lys His Gln Gln Glu Leu Leu
        115                 120                 125

Glu His Gln Arg Lys Leu Glu Arg His Arg Gln Glu Gln Glu Leu Glu
    130                 135                 140

Lys Gln His Arg Glu Gln Lys Leu Gln Gln Leu Lys Asn Lys Glu Lys
145                 150                 155                 160

Gly Lys Glu Ser Ala Val Ala Ser Thr Glu Val Lys Met Lys Leu Gln
                165                 170                 175

Glu Phe Val Leu Asn Lys Lys Lys Ala Leu Ala His Arg Asn Leu Asn
            180                 185                 190

His Cys Ile Ser Ser Asp Pro Arg Tyr Trp Tyr Gly Lys Thr Gln His
        195                 200                 205

Ser Ser Leu Asp Gln Ser Ser Pro Pro Gln Ser Gly Val Ser Thr Ser
    210                 215                 220

Tyr Asn His Pro Val Leu Gly Met Tyr Asp Ala Lys Asp Asp Phe Pro
225                 230                 235                 240

Leu Arg Lys Thr Ala Ser Glu Pro Asn Leu Lys Leu Arg Ser Arg Leu
                245                 250                 255

Lys Gln Lys Val Ala Glu Arg Arg Ser Ser Pro Leu Leu Arg Arg Lys
            260                 265                 270

Asp Gly Pro Val Val Thr Ala Leu Lys Lys Arg Pro Leu Asp Val Thr
        275                 280                 285

Asp Ser Ala Cys Ser Ser Ala Pro Gly Ser Gly Pro Ser Ser Pro Asn
    290                 295                 300

Asn Ser Ser Gly Ser Val Ser Ala Glu Asn Gly Ile Ala Pro Ala Val
305                 310                 315                 320

Pro Ser Ile Pro Ala Glu Thr Ser Leu Ala His Arg Leu Val Ala Arg
                325                 330                 335

Glu Gly Ser Ala Ala Pro Leu Pro Leu Tyr Thr Ser Pro Ser Leu Pro
            340                 345                 350

Asn Ile Thr Leu Gly Leu Pro Ala Thr Gly Pro Ser Ala Gly Thr Ala
        355                 360                 365

Gly Gln Gln Asp Thr Glu Arg Leu Thr Leu Pro Ala Leu Gln Gln Arg
    370                 375                 380

Leu Ser Leu Phe Pro Gly Thr His Leu Thr Pro Tyr Leu Ser Thr Ser
385                 390                 395                 400

Pro Leu Glu Arg Asp Gly Gly Ala Ala His Ser Pro Leu Leu Gln His
                405                 410                 415

Met Val Leu Leu Glu Gln Pro Pro Ala Gln Ala Pro Leu Val Thr Gly
            420                 425                 430

Leu Gly Ala Leu Pro Leu His Ala Gln Ser Leu Val Gly Ala Asp Arg
        435                 440                 445

Val Ser Pro Ser Ile His Lys Leu Arg Gln His Arg Pro Leu Gly Arg
    450                 455                 460

Thr Gln Ser Ala Pro Leu Pro Gln Asn Ala Gln Ala Leu Gln His Leu
465                 470                 475                 480

Val Ile Gln Gln Gln His Gln Gln Phe Leu Glu Lys His Lys Gln Gln
                485                 490                 495

Phe Gln Gln Gln Gln Leu Gln Met Asn Lys Ile Ile Pro Lys Pro Ser
            500                 505                 510

Glu Pro Ala Arg Gln Pro Glu Ser His Pro Glu Glu Thr Glu Glu Glu
        515                 520                 525

Leu Arg Glu His Gln Ala Leu Leu Asp Glu Pro Tyr Leu Asp Arg Leu
    530                 535                 540

Pro Gly Gln Lys Glu Ala His Ala Gln Ala Gly Val Gln Val Lys Gln
545                 550                 555                 560

Glu Pro Ile Glu Ser Asp Glu Glu Glu Ala Glu Pro Pro Arg Glu Val
                565                 570                 575

Glu Pro Gly Gln Arg Gln Pro Ser Glu Gln Glu Leu Leu Phe Arg Gln
            580                 585                 590

Gln Ala Leu Leu Leu Glu Gln Gln Arg Ile His Gln Leu Arg Asn Tyr
        595                 600                 605

Gln Ala Ser Met Glu Ala Ala Gly Ile Pro Val Ser Phe Gly Gly His
    610                 615                 620

Arg Pro Leu Ser Arg Ala Gln Ser Ser Pro Ala Ser Ala Thr Phe Pro
625                 630                 635                 640

Val Ser Val Gln Glu Pro Pro Thr Lys Pro Arg Phe Thr Thr Gly Leu
                645                 650                 655

Val Tyr Asp Thr Leu Met Leu Lys His Gln Cys Thr Cys Gly Ser Ser
            660                 665                 670

Ser Ser His Pro Glu His Ala Gly Arg Ile Gln Ser Ile Trp Ser Arg
        675                 680                 685

Leu Gln Glu Thr Gly Leu Arg Gly Lys Cys Glu Cys Ile Arg Gly Arg
    690                 695                 700

Lys Ala Thr Leu Glu Glu Leu Gln Thr Val His Ser Glu Ala His Thr
705                 710                 715                 720

Leu Leu Tyr Gly Thr Asn Pro Leu Asn Arg Gln Lys Leu Asp Ser Lys
                725                 730                 735

Lys Leu Leu Gly Ser Leu Ala Ser Val Phe Val Arg Leu Pro Cys Gly
            740                 745                 750

Gly Val Gly Val Asp Ser Asp Thr Ile Trp Asn Glu Val His Ser Ala
        755                 760                 765

Gly Ala Ala Arg Leu Ala Val Gly Cys Val Val Glu Leu Val Phe Lys
    770                 775                 780

Val Ala Thr Gly Glu Leu Lys Asn Gly Phe Ala Val Val Arg Pro Pro
785                 790                 795                 800

Gly Lys Leu Ala Glu Glu Ser Thr Pro Met Gly Phe Cys Tyr Phe Asn
                805                 810                 815

Ser Val Ala Val Ala Ala Lys Leu Leu Gln Gln Arg Leu Ser Val Ser
            820                 825                 830

Lys Ile Leu Ile Val Asp Trp Asp Val His His Gly Asn Gly Thr Gln
        835                 840                 845

Gln Ala Phe Tyr Ser Asp Pro Ser Val Leu Tyr Met Ser Leu His Arg
    850                 855                 860

Tyr Asp Asp Gly Asn Phe Phe Pro Gly Ser Gly Ala Pro Asp Glu Val
865                 870                 875                 880

Gly Thr Gly Pro Gly Val Gly Phe Asn Val Asn Met Ala Phe Thr Gly
                885                 890                 895

Gly Leu Asp Pro Pro Met Gly Asp Ala Glu Tyr Leu Ala Ala Phe Arg
            900                 905                 910

Thr Val Val Met Pro Ile Ala Ser Glu Phe Ala Pro Asp Val Val Leu
        915                 920                 925

Val Ser Ser Gly Phe Asp Ala Val Glu Gly His Pro Thr Pro Leu Gly
    930                 935                 940

Gly Tyr Asn Leu Ser Ala Arg Cys Phe Gly Tyr Leu Thr Lys Gln Leu
945                 950                 955                 960

Met Gly Leu Ala Gly Gly Arg Ile Val Leu Ala Leu Glu Gly Gly His
                965                 970                 975

Asp Leu Thr Ala Ile Cys Asp Ala Ser Glu Ala Cys Val Ser Ala Leu
            980                 985                 990

Leu Gly Asn Glu Leu Asp Pro Leu Pro Glu Lys Val Leu Gln Gln Arg
        995                 1000                1005

Pro Asn Ala Asn Ala Val Arg Ser Met Glu Lys Val Met Glu Ile His
    1010                1015                1020

Ser Lys Tyr Trp Arg Cys Leu Gln Arg Thr Thr Ser Thr Ala Gly Arg
1025                1030                1035                1040

Ser Leu Ile Glu Ala Gln Thr Cys Glu Asn Glu Glu Ala Glu Thr Val
                1045                1050                1055

Thr Ala Met Ala Ser Leu Ser Val Gly Val Lys Pro Ala Glu Lys Arg
            1060                1065                1070

Pro Asp Glu Glu Pro Met Glu Glu Glu Pro Pro Leu
        1075                1080


<210>  5
<211>  3255
<212>  DNA
<213>  Artificial

<220>
<221>  CDS
<222>  (1)..(3255)
<223>  Dominant negative mutant (H863L) of human histone deacetylase-4 (HDAC4)
       gene

<400>  5
atg agc tcc caa agc cat cca gat gga ctt tct ggc cga gac cag cca       48
Met Ser Ser Gln Ser His Pro Asp Gly Leu Ser Gly Arg Asp Gln Pro
1               5                   10                  15

gtg gag ctg ctg aat cct gcc cgc gtg aac cac atg ccc agc acg gtg       96
Val Glu Leu Leu Asn Pro Ala Arg Val Asn His Met Pro Ser Thr Val
            20                  25                  30

gat gtg gcc acg gcg ctg cct ctg caa gtg gcc ccc tcg gca gtg ccc      144
Asp Val Ala Thr Ala Leu Pro Leu Gln Val Ala Pro Ser Ala Val Pro
        35                  40                  45

atg gac ctg cgc ctg gac cac cag ttc tca ctg cct gtg gca gag ccg      192
Met Asp Leu Arg Leu Asp His Gln Phe Ser Leu Pro Val Ala Glu Pro
    50                  55                  60

gcc ctg cgg gag cag cag ctg cag cag gag ctc ctg gcg ctc aag cag      240
Ala Leu Arg Glu Gln Gln Leu Gln Gln Glu Leu Leu Ala Leu Lys Gln
65                  70                  75                  80

aag cag cag atc cag agg cag atc ctc atc gct gag ttc cag agg cag      288
Lys Gln Gln Ile Gln Arg Gln Ile Leu Ile Ala Glu Phe Gln Arg Gln
                85                  90                  95

cac gag cag ctc tcc cgg cag cac gag gcg cag ctc cac gag cac atc      336
His Glu Gln Leu Ser Arg Gln His Glu Ala Gln Leu His Glu His Ile
            100                 105                 110

aag caa caa cag gag atg ctg gcc atg aag cac cag cag gag ctg ctg 384
Lys Gln Gln Gln Glu Met Leu Ala Met Lys His Gln Gln Glu Leu Leu
        115                 120                 125

gaa cac cag cgg aag ctg gag agg cac cgc cag gag cag gag ctg gag 432
Glu His Gln Arg Lys Leu Glu Arg His Arg Gln Glu Gln Glu Leu Glu
    130                 135                 140

aag cag cac cgg gag cag aag ctg cag cag ctc aag aac aag gag aag 480
Lys Gln His Arg Glu Gln Lys Leu Gln Gln Leu Lys Asn Lys Glu Lys
145                 150                 155                 160

ggc aaa gag agt gcc gtg gcc agc aca gaa gtg aag atg aag tta caa 528
Gly Lys Glu Ser Ala Val Ala Ser Thr Glu Val Lys Met Lys Leu Gln
                165                 170                 175

gaa ttt gtc ctc aat aaa aag aag gcg ctg gcc cac cgg aat ctg aac 576
Glu Phe Val Leu Asn Lys Lys Lys Ala Leu Ala His Arg Asn Leu Asn
            180                 185                 190

cac tgc att tcc agc gac cct cgc tac tgg tac ggg aaa acg cag cac 624
His Cys Ile Ser Ser Asp Pro Arg Tyr Trp Tyr Gly Lys Thr Gln His
        195                 200                 205

agt tcc ctt gac cag agt tct cca ccc cag agc gga gtg tcg acc tcc 672
Ser Ser Leu Asp Gln Ser Ser Pro Pro Gln Ser Gly Val Ser Thr Ser
    210                 215                 220

tat aac cac ccg gtc ctg gga atg tac gac gcc aaa gat gac ttc cct 720
Tyr Asn His Pro Val Leu Gly Met Tyr Asp Ala Lys Asp Asp Phe Pro
225                 230                 235                 240

ctt agg aaa aca gct tct gaa ccg aat ctg aaa tta cgg tcc agg cta 768
Leu Arg Lys Thr Ala Ser Glu Pro Asn Leu Lys Leu Arg Ser Arg Leu
                245                 250                 255

aag cag aaa gtg gcc gaa aga cgg agc agc ccc ctg tta cgc agg aaa 816
Lys Gln Lys Val Ala Glu Arg Arg Ser Ser Pro Leu Leu Arg Arg Lys
            260                 265                 270

gac ggg cca gtg gtc act gct cta aaa aag cgt ccg ttg gat gtc aca 864
Asp Gly Pro Val Val Thr Ala Leu Lys Lys Arg Pro Leu Asp Val Thr
        275                 280                 285

gac tcc gcg tgc agc agc gcc cca ggc tcc gga ccc agc tca ccc aac 912
Asp Ser Ala Cys Ser Ser Ala Pro Gly Ser Gly Pro Ser Ser Pro Asn
    290                 295                 300

aac agc tcc ggg agc gtc agc gcg gag aac ggt atc gcg ccc gcc gtc 960
Asn Ser Ser Gly Ser Val Ser Ala Glu Asn Gly Ile Ala Pro Ala Val
305                 310                 315                 320

ccc agc atc ccg gcg gag acg agt ttg gcg cac aga ctt gtg gca cga 1008
Pro Ser Ile Pro Ala Glu Thr Ser Leu Ala His Arg Leu Val Ala Arg
                325                 330                 335

gaa ggc tcg gcc gct cca ctt ccc ctc tac aca tcg cca tcc ttg ccc 1056
Glu Gly Ser Ala Ala Pro Leu Pro Leu Tyr Thr Ser Pro Ser Leu Pro
            340                 345                 350

aac atc acg ctg ggc ctg cct gcc acc ggc ccc tct gcg ggc acg gcg 1104
Asn Ile Thr Leu Gly Leu Pro Ala Thr Gly Pro Ser Ala Gly Thr Ala
        355                 360                 365

ggc cag cag gac acc gag aga ctc acc ctt ccc gcc ctc cag cag agg 1152
Gly Gln Gln Asp Thr Glu Arg Leu Thr Leu Pro Ala Leu Gln Gln Arg
    370                 375                 380

ctc tcc ctt ttc ccc ggc acc cac ctc act ccc tac ctg agc acc tcg 1200
Leu Ser Leu Phe Pro Gly Thr His Leu Thr Pro Tyr Leu Ser Thr Ser
385                 390                 395                 400

ccc ttg gag cgg gac gga ggg gca gcg cac agc cct ctt ctg cag cac 1248
Pro Leu Glu Arg Asp Gly Gly Ala Ala His Ser Pro Leu Leu Gln His
                405                 410                 415

atg gtc tta ctg gag cag cca ccg gca caa gca ccc ctc gtc aca ggc 1296
Met Val Leu Leu Glu Gln Pro Pro Ala Gln Ala Pro Leu Val Thr Gly
            420                 425                 430

ctg gga gca ctg ccc ctc cac gca cag tcc ttg gtt ggt gca gac cgg 1344
Leu Gly Ala Leu Pro Leu His Ala Gln Ser Leu Val Gly Ala Asp Arg
        435                 440                 445

gtg tcc ccc tcc atc cac aag ctg cgg cag cac cgc cca ctg ggg cgg 1392
Val Ser Pro Ser Ile His Lys Leu Arg Gln His Arg Pro Leu Gly Arg
    450                 455                 460

acc cag tcg gcc ccg ctg ccc cag aac gcc cag gct ctg cag cac ctg 1440
Thr Gln Ser Ala Pro Leu Pro Gln Asn Ala Gln Ala Leu Gln His Leu
465                 470                 475                 480

gtc atc cag cag cag cat cag cag ttt ctg gag aaa cac aag cag cag 1488
Val Ile Gln Gln Gln His Gln Gln Phe Leu Glu Lys His Lys Gln Gln
                485                 490                 495

ttc cag cag cag caa ctg cag atg aac aag atc atc ccc aag cca agc 1536
Phe Gln Gln Gln Gln Leu Gln Met Asn Lys Ile Ile Pro Lys Pro Ser
            500                 505                 510

gag cca gcc cgg cag ccg gag agc cac ccg gag gag acg gag gag gag 1584
Glu Pro Ala Arg Gln Pro Glu Ser His Pro Glu Glu Thr Glu Glu Glu
        515                 520                 525

ctc cgt gag cac cag gct ctg ctg gac gag ccc tac ctg gac cgg ctg 1632
Leu Arg Glu His Gln Ala Leu Leu Asp Glu Pro Tyr Leu Asp Arg Leu
    530                 535                 540

ccg ggg cag aag gag gcg cac gca cag gcc ggc gtg cag gtg aag cag 1680
Pro Gly Gln Lys Glu Ala His Ala Gln Ala Gly Val Gln Val Lys Gln
545                 550                 555                 560

gag ccc att gag agc gat gag gaa gag gca gag ccc cca cgg gag gtg 1728
Glu Pro Ile Glu Ser Asp Glu Glu Glu Ala Glu Pro Pro Arg Glu Val
                565                 570                 575

gag ccg ggc cag cgc cag ccc agt gag cag gag ctg ctc ttc aga cag 1776
Glu Pro Gly Gln Arg Gln Pro Ser Glu Gln Glu Leu Leu Phe Arg Gln
            580                 585                 590

caa gcc ctc ctg ctg gag cag cag cgg atc cac cag ctg agg aac tac 1824
Gln Ala Leu Leu Leu Glu Gln Gln Arg Ile His Gln Leu Arg Asn Tyr
        595                 600                 605

cag gcg tcc atg gag gcc gcc ggc atc ccc gtg tcc ttc ggc ggc cac 1872
Gln Ala Ser Met Glu Ala Ala Gly Ile Pro Val Ser Phe Gly Gly His
    610                 615                 620

agg cct ctg tcc cgg gcg cag tcc tca ccc gcg tct gcc acc ttc ccc 1920
Arg Pro Leu Ser Arg Ala Gln Ser Ser Pro Ala Ser Ala Thr Phe Pro
625                 630                 635                 640

gtg tct gtg cag gag ccc ccc acc aag ccg agg ttc acg aca ggc ctc 1968
Val Ser Val Gln Glu Pro Pro Thr Lys Pro Arg Phe Thr Thr Gly Leu
                645                 650                 655

gtg tat gac acg ctg atg ctg aag cac cag tgc acc tgc ggg agt agc 2016
Val Tyr Asp Thr Leu Met Leu Lys His Gln Cys Thr Cys Gly Ser Ser
            660                 665                 670

agc agc cac ccc gag cac gcc ggg agg atc cag agc atc tgg tcc cgc 2064
Ser Ser His Pro Glu His Ala Gly Arg Ile Gln Ser Ile Trp Ser Arg
        675                 680                 685

ctg cag gag acg ggc ctc cgg ggc aaa tgc gag tgc atc cgc gga cgc 2112
Leu Gln Glu Thr Gly Leu Arg Gly Lys Cys Glu Cys Ile Arg Gly Arg
    690                 695                 700

aag gcc acc ctg gag gag cta cag acg gtg cac tcg gaa gcc cac acc 2160
Lys Ala Thr Leu Glu Glu Leu Gln Thr Val His Ser Glu Ala His Thr

705                 710                 715                 720

ctc ctg tat ggc acg aac ccc ctc aac cgg cag aaa ctg gac agt aag     2208
Leu Leu Tyr Gly Thr Asn Pro Leu Asn Arg Gln Lys Leu Asp Ser Lys
                725                 730                 735

aaa ctt cta ggc tcg ctc gcc tcc gtg ttc gtc cgg ctc cct tgc ggt     2256
Lys Leu Leu Gly Ser Leu Ala Ser Val Phe Val Arg Leu Pro Cys Gly
            740                 745                 750

ggt gtt ggg gtg gac agt gac acc ata tgg aac gag gtg cac tcg gcg     2304
Gly Val Gly Val Asp Ser Asp Thr Ile Trp Asn Glu Val His Ser Ala
        755                 760                 765

ggg gca gcc cgc ctg gct gtg ggc tgc gtg gta gag ctg gtc ttc aag     2352
Gly Ala Ala Arg Leu Ala Val Gly Cys Val Val Glu Leu Val Phe Lys
    770                 775                 780

gtg gcc aca ggg gag ctg aag aat ggc ttt gct gtg gtc cgc ccc cct     2400
Val Ala Thr Gly Glu Leu Lys Asn Gly Phe Ala Val Val Arg Pro Pro
785                 790                 795                 800

gga cac cat gcg gag gag agc acg ccc atg ggc ttt tgc tac ttc aac     2448
Gly His His Ala Glu Glu Ser Thr Pro Met Gly Phe Cys Tyr Phe Asn
                805                 810                 815

tcc gtg gcc gtg gca gcc aag ctt ctg cag cag agg ttg agc gtg agc     2496
Ser Val Ala Val Ala Ala Lys Leu Leu Gln Gln Arg Leu Ser Val Ser
            820                 825                 830

aag atc ctc atc gtg gac tgg gac gtg cac cat gga aac ggg acc cag     2544
Lys Ile Leu Ile Val Asp Trp Asp Val His His Gly Asn Gly Thr Gln
        835                 840                 845

cag gct ttc tac agc gac cct agc gtc ctg tac atg tcc ctc ctt cgc     2592
Gln Ala Phe Tyr Ser Asp Pro Ser Val Leu Tyr Met Ser Leu Leu Arg
    850                 855                 860

tac gac gat ggg aac ttc ttc cca ggc agc ggg gct cct gat gag gtg     2640
Tyr Asp Asp Gly Asn Phe Phe Pro Gly Ser Gly Ala Pro Asp Glu Val
865                 870                 875                 880

ggc aca ggg ccc ggc gtg ggt ttc aac gtc aac atg gct ttc acc ggc     2688
Gly Thr Gly Pro Gly Val Gly Phe Asn Val Asn Met Ala Phe Thr Gly
                885                 890                 895

ggc ctg gac ccc ccc atg gga gac gct gag tac ttg gcg gcc ttc aga     2736
Gly Leu Asp Pro Pro Met Gly Asp Ala Glu Tyr Leu Ala Ala Phe Arg
            900                 905                 910

acg gtg gtc atg ccg atc gcc agc gag ttt gcc ccg gat gtg gtg ctg     2784
Thr Val Val Met Pro Ile Ala Ser Glu Phe Ala Pro Asp Val Val Leu
        915                 920                 925

gtg tca tca ggc ttc gat gcc gtg gag ggc cac ccc acc cct ctt ggg     2832
Val Ser Ser Gly Phe Asp Ala Val Glu Gly His Pro Thr Pro Leu Gly
    930                 935                 940

ggc tac aac ctc tcc gcc aga tgc ttc ggg tac ctg acg aag cag ctg     2880
Gly Tyr Asn Leu Ser Ala Arg Cys Phe Gly Tyr Leu Thr Lys Gln Leu
945                 950                 955                 960

atg ggc ctg gct ggc ggc cgg att gtc ctg gcc ctc gag gga ggc cac     2928
Met Gly Leu Ala Gly Gly Arg Ile Val Leu Ala Leu Glu Gly Gly His
                965                 970                 975

gac ctg acc gcc att tgc gac gcc tcg gaa gca tgt gtt tct gcc ttg     2976
Asp Leu Thr Ala Ile Cys Asp Ala Ser Glu Ala Cys Val Ser Ala Leu
            980                 985                 990

ctg gga aac gag ctt gat cct ctc cca gaa aag gtt tta cag caa aga     3024
Leu Gly Asn Glu Leu Asp Pro Leu Pro Glu Lys Val Leu Gln Gln Arg
        995                 1000                1005

ccc aat gca aac gct gtc cgt tcc atg gag aaa gtc atg gag atc cac     3072

Pro Asn Ala Asn Ala Val Arg Ser Met Glu Lys Val Met Glu Ile His
    1010                1015                1020

agc aag tac tgg cgc tgc ctg cag cgc aca acc tcc aca gcg ggg cgt 3120
Ser Lys Tyr Trp Arg Cys Leu Gln Arg Thr Thr Ser Thr Ala Gly Arg
1025                1030                1035                1040

tct ctg atc gag gct cag act tgc gag aac gaa gaa gcc gag acg gtc 3168
Ser Leu Ile Glu Ala Gln Thr Cys Glu Asn Glu Glu Ala Glu Thr Val
                1045                1050                1055

acc gcc atg gcc tcg ctg tcc gtg ggc gtg aag ccc gcc gaa aag aga 3216
Thr Ala Met Ala Ser Leu Ser Val Gly Val Lys Pro Ala Glu Lys Arg
            1060                1065                1070

cca gat gag gag ccc atg gaa gag gag ccg ccc ctg tag 3255
Pro Asp Glu Glu Pro Met Glu Glu Glu Pro Pro Leu
        1075                1080

<210> 6
<211> 1084
<212> PRT
<213> Homo sapiens

<220>
<223> Dominant negative mutant (H863L) of human histone deacetylase-4 (HDAC4)
gene

<400> 6
Met Ser Ser Gln Ser His Pro Asp Gly Leu Ser Gly Arg Asp Gln Pro
1               5                   10                  15

Val Glu Leu Leu Asn Pro Ala Arg Val Asn His Met Pro Ser Thr Val
            20                  25                  30

Asp Val Ala Thr Ala Leu Pro Leu Gln Val Ala Pro Ser Ala Val Pro
        35                  40                  45

Met Asp Leu Arg Leu Asp His Gln Phe Ser Leu Pro Val Ala Glu Pro
    50                  55                  60

Ala Leu Arg Glu Gln Gln Leu Gln Gln Glu Leu Leu Ala Leu Lys Gln
65                  70                  75                  80

Lys Gln Gln Ile Gln Arg Gln Ile Leu Ile Ala Glu Phe Gln Arg Gln
                85                  90                  95

His Glu Gln Leu Ser Arg Gln His Glu Ala Gln Leu His Glu His Ile
            100                 105                 110

Lys Gln Gln Gln Glu Met Leu Ala Met Lys His Gln Gln Glu Leu Leu
        115                 120                 125

Glu His Gln Arg Lys Leu Glu Arg His Arg Gln Glu Gln Glu Leu Glu
    130                 135                 140

Lys Gln His Arg Glu Gln Lys Leu Gln Gln Leu Lys Asn Lys Glu Lys
145                 150                 155                 160

Gly Lys Glu Ser Ala Val Ala Ser Thr Glu Val Lys Met Lys Leu Gln
                165                 170                 175

Glu Phe Val Leu Asn Lys Lys Lys Ala Leu Ala His Arg Asn Leu Asn
            180                 185                 190

His Cys Ile Ser Ser Asp Pro Arg Tyr Trp Tyr Gly Lys Thr Gln His
        195                 200                 205

Ser Ser Leu Asp Gln Ser Ser Pro Pro Gln Ser Gly Val Ser Thr Ser
    210                 215                 220

Tyr Asn His Pro Val Leu Gly Met Tyr Asp Ala Lys Asp Asp Phe Pro
225                 230                 235                 240

Leu Arg Lys Thr Ala Ser Glu Pro Asn Leu Lys Leu Arg Ser Arg Leu
                245                 250                 255

Lys Gln Lys Val Ala Glu Arg Arg Ser Ser Pro Leu Leu Arg Arg Lys
            260                 265                 270

Asp Gly Pro Val Val Thr Ala Leu Lys Lys Arg Pro Leu Asp Val Thr
        275                 280                 285

Asp Ser Ala Cys Ser Ser Ala Pro Gly Ser Gly Pro Ser Ser Pro Asn
    290                 295                 300

Asn Ser Ser Gly Ser Val Ser Ala Glu Asn Gly Ile Ala Pro Ala Val
305                 310                 315                 320

Pro Ser Ile Pro Ala Glu Thr Ser Leu Ala His Arg Leu Val Ala Arg
                325                 330                 335

Glu Gly Ser Ala Ala Pro Leu Pro Leu Tyr Thr Ser Pro Ser Leu Pro
            340                 345                 350

Asn Ile Thr Leu Gly Leu Pro Ala Thr Gly Pro Ser Ala Gly Thr Ala
        355                 360                 365

Gly Gln Gln Asp Thr Glu Arg Leu Thr Leu Pro Ala Leu Gln Gln Arg
    370                 375                 380

Leu Ser Leu Phe Pro Gly Thr His Leu Thr Pro Tyr Leu Ser Thr Ser
385                 390                 395                 400

Pro Leu Glu Arg Asp Gly Gly Ala Ala His Ser Pro Leu Leu Gln His
                405                 410                 415

Met Val Leu Leu Glu Gln Pro Pro Ala Gln Ala Pro Leu Val Thr Gly
            420                 425                 430

Leu Gly Ala Leu Pro Leu His Ala Gln Ser Leu Val Gly Ala Asp Arg
        435                 440                 445

Val Ser Pro Ser Ile His Lys Leu Arg Gln His Arg Pro Leu Gly Arg
    450                 455                 460

Thr Gln Ser Ala Pro Leu Pro Gln Asn Ala Gln Ala Leu Gln His Leu
465                 470                 475                 480

Val Ile Gln Gln Gln His Gln Gln Phe Leu Glu Lys His Lys Gln Gln
                485                 490                 495

Phe Gln Gln Gln Gln Leu Gln Met Asn Lys Ile Ile Pro Lys Pro Ser
            500                 505                 510

Glu Pro Ala Arg Gln Pro Glu Ser His Pro Glu Glu Thr Glu Glu Glu
        515                 520                 525

Leu Arg Glu His Gln Ala Leu Leu Asp Glu Pro Tyr Leu Asp Arg Leu
    530                 535                 540

Pro Gly Gln Lys Glu Ala His Ala Gln Ala Gly Val Gln Val Lys Gln
545                 550                 555                 560

Glu Pro Ile Glu Ser Asp Glu Glu Glu Ala Glu Pro Pro Arg Glu Val
                565                 570                 575

Glu Pro Gly Gln Arg Gln Pro Ser Glu Gln Glu Leu Leu Phe Arg Gln
            580                 585                 590

Gln Ala Leu Leu Leu Glu Gln Gln Arg Ile His Gln Leu Arg Asn Tyr
        595                 600                 605

Gln Ala Ser Met Glu Ala Ala Gly Ile Pro Val Ser Phe Gly Gly His
    610                 615                 620

Arg Pro Leu Ser Arg Ala Gln Ser Ser Pro Ala Ser Ala Thr Phe Pro
625                 630                 635                 640

Val Ser Val Gln Glu Pro Pro Thr Lys Pro Arg Phe Thr Thr Gly Leu
                645                 650                 655

Val Tyr Asp Thr Leu Met Leu Lys His Gln Cys Thr Cys Gly Ser Ser
            660                 665                 670

Ser Ser His Pro Glu His Ala Gly Arg Ile Gln Ser Ile Trp Ser Arg
        675                 680                 685

Leu Gln Glu Thr Gly Leu Arg Gly Lys Cys Glu Cys Ile Arg Gly Arg
    690                 695                 700

Lys Ala Thr Leu Glu Glu Leu Gln Thr Val His Ser Glu Ala His Thr
705                 710                 715                 720

Leu Leu Tyr Gly Thr Asn Pro Leu Asn Arg Gln Lys Leu Asp Ser Lys
                725                 730                 735

Lys Leu Leu Gly Ser Leu Ala Ser Val Phe Val Arg Leu Pro Cys Gly
            740                 745                 750

Gly Val Gly Val Asp Ser Asp Thr Ile Trp Asn Glu Val His Ser Ala
        755                 760                 765

Gly Ala Ala Arg Leu Ala Val Gly Cys Val Val Glu Leu Val Phe Lys
    770                 775                 780

Val Ala Thr Gly Glu Leu Lys Asn Gly Phe Ala Val Val Arg Pro Pro
785                 790                 795                 800

Gly His His Ala Glu Glu Ser Thr Pro Met Gly Phe Cys Tyr Phe Asn
                805                 810                 815

Ser Val Ala Val Ala Ala Lys Leu Leu Gln Gln Arg Leu Ser Val Ser
            820                 825                 830

Lys Ile Leu Ile Val Asp Trp Asp Val His His Gly Asn Gly Thr Gln
        835                 840                 845

Gln Ala Phe Tyr Ser Asp Pro Ser Val Leu Tyr Met Ser Leu Leu Arg
    850                 855                 860

Tyr Asp Asp Gly Asn Phe Phe Pro Gly Ser Gly Ala Pro Asp Glu Val
865                 870                 875                 880

Gly Thr Gly Pro Gly Val Gly Phe Asn Val Asn Met Ala Phe Thr Gly
                885                 890                 895

Gly Leu Asp Pro Pro Met Gly Asp Ala Glu Tyr Leu Ala Ala Phe Arg
            900                 905                 910

Thr Val Val Met Pro Ile Ala Ser Glu Phe Ala Pro Asp Val Val Leu
        915                 920                 925

Val Ser Ser Gly Phe Asp Ala Val Glu Gly His Pro Thr Pro Leu Gly
    930                 935                 940

Gly Tyr Asn Leu Ser Ala Arg Cys Phe Gly Tyr Leu Thr Lys Gln Leu
945                 950                 955                 960

Met Gly Leu Ala Gly Gly Arg Ile Val Leu Ala Leu Glu Gly Gly His
                965                 970                 975

Asp Leu Thr Ala Ile Cys Asp Ala Ser Glu Ala Cys Val Ser Ala Leu
            980                 985                 990

Leu Gly Asn Glu Leu Asp Pro Leu Pro Glu Lys Val Leu Gln Gln Arg
        995                 1000                1005

Pro Asn Ala Asn Ala Val Arg Ser Met Glu Lys Val Met Glu Ile His
    1010                1015                1020

Ser Lys Tyr Trp Arg Cys Leu Gln Arg Thr Thr Ser Thr Ala Gly Arg
1025                1030                1035                1040

Ser Leu Ile Glu Ala Gln Thr Cys Glu Asn Glu Glu Ala Glu Thr Val
                1045                1050                1055

Thr Ala Met Ala Ser Leu Ser Val Gly Val Lys Pro Ala Glu Lys Arg
            1060                1065                1070

Pro Asp Glu Glu Pro Met Glu Glu Glu Pro Pro Leu
        1075                1080

<210> 7
<211> 1743
<212> DNA
<213> Homo sapiens

<220>
<221> CDS
<222> (43)..(1176)
<223> human histone deacetylase-8 (HDAC8) gene

<400> 7
gccagatctg gaaggtggct gcggaacggt tttaagcgga ag atg gag gag ccg 54
gag gaa ccg gcg gac agt ggg cag tcg ctg gtc ccg gtt tat atc tat 102
agt ccc gag tat gtc agt atg tgt gac tcc ctg gcc aag atc ccc aaa 150
cgg gcc agt atg gtg cat tct ttg att gaa gca tat gca ctg cat aag 198
cag atg agg ata gtt aag cct aaa gtg gcc tcc atg gag gag atg gcc 246
acc ttc cac act gat gct tat ctg cag cat ctc cag aag gtc agc caa 294
gag ggc gat gat gat cat ccg gac tcc ata gaa tat ggg cta ggt tat 342
gac tgc cca gcc act gaa ggg ata ttt gac tat gca gca gct ata gga 390
ggg gct acg atc aca gct gcc caa tgc ctg att gac gga atg tgc aaa 438
gta gca att aac tgg tct gga ggg tgg cat cat gca aag aaa gat gaa 486
gca tct ggt ttt tgt tat ctc aat gat gct gtc ctg gga ata tta cga 534
ttg cga cgg aaa ttt gag cgt att ctc tac gtg gat ttg gat ctg cac 582
cat gga gat ggt gta gaa gac gca ttc agt ttc acc tcc aaa gtc atg 630
acc gtg tcc ctg cac aaa ttc tcc cca gga ttt ttc cca gga aca ggt 678
gac gtg tct gat gtt ggc cta ggg aag gga cgg tac tac agt gta aat        726
gtg ccc att cag gat ggc ata caa gat gaa aaa tat tac cag atc tgt        774
gaa agt gta cta aag gaa gta tac caa gcc ttt aat ccc aaa gca gtg        822
gtc tta cag ctg gga gct gac aca ata gct ggg gat ccc atg tgc tcc        870
ttt aac atg act cca gtg gga att ggc aag tgt ctt aag tac atc ctt        918
caa tgg cag ttg gca aca ctc att ttg gga gga gga ggc tat aac ctt        966
gcc aac acg gct cga tgc tgg aca tac ttg acc ggg gtc atc cta ggg       1014
aaa aca cta tcc tct gag atc cca gat cat gag ttt ttc aca gca tat       1062
ggt cct gat tat gtg ctg gaa atc acg cca agc tgc cgg cca gac cgc       1110
aat gag ccc cac cga atc caa caa atc ctc aac tac atc aaa ggg aat       1158
ctg aag cat gtg gtc tag ttgacagaaa gagatcaggt ttccagagct              1206
gaggagtggt gcctataatg aagacagcgt gtttatgcaa gcagtttgtg gaatttgtga     1266
ctgcagggaa aatttgaaag aaattacttc ctgaaaattt ccaaggggca tcaagtggca     1326
gctggcttcc tggggtgaag aggcaggcac cccagagtcc tcaactggac ctaggggaag     1386
aaggagatat cccacattta aagttcttat ttaaaaaaac acacacacac aaatgaaatt     1446
tttaatcttt gaaaattatt tttaagcgaa ttggggaggg gagtatttta atcatcttaa     1506
atgaaacaga tcagaagctg gatgagagca gtcaccagtt tgtagggcag gaggcagctg     1566
agaggcaggg tttgggcctc aggaccatcc aggtggagcc ctgggagaga gggtactgat     1626
cagcagactg ggaggtgggg agaagtccgc tggtgttgtt ttagtgttat atatctttgg     1686
tttttttaat aaaatctttg aaaacctaaa aaaaaaaaaa aaaaaaaaaa aaaaaaa        1743

<210>  8
<211>  377
<212>  PRT
<213>  Homo sapiens

<400>  8

<210>  9
<211>  1449
<212>  DNA
<213>  Homo sapiens

<220>
<221>  CDS
<222>  (1)..(1449)
<223>  human histone deacetylase-1 (HDAC1) gene

<400>  9
atg gcg cag acg cag ggc acc cgg agg aaa gtc tgt tac tac tac gac      48
ggg gat gtt gga aat tac tat tat gga caa ggc cac cca atg aag cct      96
cac cga atc cgc atg act cat aat ttg ctg ctc aac tat ggt ctc tac      144
cga aaa atg gaa atc tat cgc cct cac aaa gcc aat gct gag gag atg      192
acc aag tac cac agc gat gac tac att aaa ttc ttg cgc tcc atc cgt      240
cca gat aac atg tcg gag tac agc aag cag atg cag aga ttc aac gtt      288
ggt gag gac tgt cca gta ttc gat ggc ctg ttt gag ttc tgt cag ttg      336
tct act ggt ggt tct gtg gca agt gct gtg aaa ctt aat aag cag cag      384
acg gac atc gct gtg aat tgg gct ggg ggc ctg cac cat gca aag aag      432
tcc gag gca tct ggc ttc tgt tac gtc aat gat atc gtc ttg gcc atc      480
ctg gaa ctg cta aag tat cac cag agg gtg ctg tac att gac att gat      528
att cac cat ggt gac ggc gtg gaa gag gcc ttc tac acc acg gac cgg      576
gtc atg act gtg tcc ttt cat aag tat gga gag tac ttc cca gga act     624
ggg gac cta cgg gat atc ggg gct ggc aaa ggc aag tat tat gct gtt     672
aac tac ccg ctc cga gac ggg att gat gac gag tcc tat gag gcc att     720
ttc aag ccg gtc atg tcc aaa gta atg gag atg ttc cag cct agt gcg     768
gtg gtc tta cag tgt ggc tca gac tcc cta tct ggg gat cgg tta ggt     816
tgc ttc aat cta act atc aaa gga cac gcc aag tgt gtg gaa ttt gtc     864
aag agc ttt aac ctg cct atg ctg atg ctg gga ggc ggt ggt tac acc     912
att cgt aac gtt gcc cgg tgc agg aca tat gag aca gct gtg gcc ctg     960
gat acg gag atc cct aat gag ctt cca tac aat gac tac ttt gaa tac     1008
ttt gga cca gat ttc aag ctc cac atc agt cct tcc aat atg act aac     1056
cag aac acg aat gag tac ctg gag aag atc aaa cag cga ctg ttt gag     1104
aac ctt aga atg ctg ccg cac gca cct ggg gtc caa atg cag gcg att     1152
cct gag gac gcc atc cct gag gag agt ggc gat gag gac gaa gac gac     1200
cct gac aag cgc atc tcg atc tgc tcc tct gac aaa cga att gcc tgt     1248
gag gaa gag ttc tcc gat tct gaa gag gag gga gag ggg ggc cgc aag     1296
aac tct tcc aac ttc aaa aaa gcc aag aga gtc aaa aca gag gat gaa     1344
aaa gag aaa gac cca gag gag aag aaa gaa gtc acc gaa gag gag aaa     1392
acc aag gag gag aag cca gaa gcc aaa ggg gtc aag gag gag gtc aag     1440
ttg gcc tga                                                         1449

<210> 10
<211> 482
<212> PRT
<213> Homo sapiens

<400> 10

<210>  11
<211>  1449
<212>  DNA
<213>  Artificial

<220>
<221>  CDS
<222>  (1)..(1449)
<223>  Dominant negative mutant (H140K, H141L) of human histone deacetylase-1
       (HDAC1) gene

<400>  11
atg gcg cag acg cag ggc acc cgg agg aaa gtc tgt tac tac tac gac       48
Met Ala Gln Thr Gln Gly Thr Arg Arg Lys Val Cys Tyr Tyr Tyr Asp
1               5                   10                  15

ggg gat gtt gga aat tac tat tat gga caa ggc cac cca atg aag cct       96

Gly Asp Val Gly Asn Tyr Tyr Tyr Gly Gln Gly His Pro Met Lys Pro
20                  25                  30

cac cga atc cgc atg act cat aat ttg ctg ctc aac tat ggt ctc tac      144
His Arg Ile Arg Met Thr His Asn Leu Leu Leu Asn Tyr Gly Leu Tyr
        35                  40                  45

cga aaa atg gaa atc tat cgc cct cac aaa gcc aat gct gag gag atg      192
Arg Lys Met Glu Ile Tyr Arg Pro His Lys Ala Asn Ala Glu Glu Met
    50                  55                  60

acc aag tac cac agc gat gac tac att aaa ttc ttg cgc tcc atc cgt      240
Thr Lys Tyr His Ser Asp Asp Tyr Ile Lys Phe Leu Arg Ser Ile Arg
65                  70                  75                  80

cca gat aac atg tcg gag tac agc aag cag atg cag aga ttc aac gtt      288
Pro Asp Asn Met Ser Glu Tyr Ser Lys Gln Met Gln Arg Phe Asn Val
                85                  90                  95

ggt gag gac tgt cca gta ttc gat ggc ctg ttt gag ttc tgt cag ttg      336
Gly Glu Asp Cys Pro Val Phe Asp Gly Leu Phe Glu Phe Cys Gln Leu
            100                 105                 110

tct act ggt ggt tct gtg gca agt gct gtg aaa ctt aat aag cag cag      384
Ser Thr Gly Gly Ser Val Ala Ser Ala Val Lys Leu Asn Lys Gln Gln
        115                 120                 125

acg gac atc gct gtg aat tgg gct ggg ggc ctg aag ctt gca aag aag      432
Thr Asp Ile Ala Val Asn Trp Ala Gly Gly Leu Lys Leu Ala Lys Lys
    130                 135                 140

tcc gag gca tct ggc ttc tgt tac gtc aat gat atc gtc ttg gcc atc      480
Ser Glu Ala Ser Gly Phe Cys Tyr Val Asn Asp Ile Val Leu Ala Ile
145                 150                 155                 160

ctg gaa ctg cta aag tat cac cag agg gtg ctg tac att gac att gat      528
Leu Glu Leu Leu Lys Tyr His Gln Arg Val Leu Tyr Ile Asp Ile Asp
                165                 170                 175

att cac cat ggt gac ggc gtg gaa gag gcc ttc tac acc acg gac cgg      576
Ile His His Gly Asp Gly Val Glu Glu Ala Phe Tyr Thr Thr Asp Arg
            180                 185                 190

gtc atg act gtg tcc ttt cat aag tat gga gag tac ttc cca gga act      624
Val Met Thr Val Ser Phe His Lys Tyr Gly Glu Tyr Phe Pro Gly Thr
        195                 200                 205

ggg gac cta cgg gat atc ggg gct ggc aaa ggc aag tat tat gct gtt      672
Gly Asp Leu Arg Asp Ile Gly Ala Gly Lys Gly Lys Tyr Tyr Ala Val
    210                 215                 220

aac tac ccg ctc cga gac ggg att gat gac gag tcc tat gag gcc att      720
Asn Tyr Pro Leu Arg Asp Gly Ile Asp Asp Glu Ser Tyr Glu Ala Ile
225                 230                 235                 240

ttc aag ccg gtc atg tcc aaa gta atg gag atg ttc cag cct agt gcg      768
Phe Lys Pro Val Met Ser Lys Val Met Glu Met Phe Gln Pro Ser Ala
                245                 250                 255

gtg gtc tta cag tgt ggc tca gac tcc cta tct ggg gat cgg tta ggt      816
Val Val Leu Gln Cys Gly Ser Asp Ser Leu Ser Gly Asp Arg Leu Gly
            260                 265                 270

tgc ttc aat cta act atc aaa gga cac gcc aag tgt gtg gaa ttt gtc      864
Cys Phe Asn Leu Thr Ile Lys Gly His Ala Lys Cys Val Glu Phe Val
        275                 280                 285

aag agc ttt aac ctg cct atg ctg atg ctg gga ggc ggt ggt tac acc      912
Lys Ser Phe Asn Leu Pro Met Leu Met Leu Gly Gly Gly Gly Tyr Thr
    290                 295                 300

att cgt aac gtt gcc cgg tgc agg aca tat gag aca gct gtg gcc ctg      960
Ile Arg Asn Val Ala Arg Cys Arg Thr Tyr Glu Thr Ala Val Ala Leu
305                 310                 315                 320

gat acg gag atc cct aat gag ctt cca tac aat gac tac ttt gaa tac 1008
Asp Thr Glu Ile Pro Asn Glu Leu Pro Tyr Asn Asp Tyr Phe Glu Tyr
                325                 330                 335

ttt gga cca gat ttc aag ctc cac atc agt cct tcc aat atg act aac 1056
Phe Gly Pro Asp Phe Lys Leu His Ile Ser Pro Ser Asn Met Thr Asn
            340                 345                 350

cag aac acg aat gag tac ctg gag aag atc aaa cag cga ctg ttt gag 1104
Gln Asn Thr Asn Glu Tyr Leu Glu Lys Ile Lys Gln Arg Leu Phe Glu
        355                 360                 365

aac ctt aga atg ctg ccg cac gca cct ggg gtc caa atg cag gcg att 1152
Asn Leu Arg Met Leu Pro His Ala Pro Gly Val Gln Met Gln Ala Ile
    370                 375                 380

cct gag gac gcc atc cct gag gag agt ggc gat gag gac gaa gac gac 1200
Pro Glu Asp Ala Ile Pro Glu Glu Ser Gly Asp Glu Asp Glu Asp Asp
385                 390                 395                 400

cct gac aag cgc atc tcg atc tgc tcc tct gac aaa cga att gcc tgt 1248
Pro Asp Lys Arg Ile Ser Ile Cys Ser Ser Asp Lys Arg Ile Ala Cys
                405                 410                 415

gag gaa gag ttc tcc gat tct gaa gag gag gga gag ggg ggc cgc aag 1296
Glu Glu Glu Phe Ser Asp Ser Glu Glu Glu Gly Glu Gly Gly Arg Lys
            420                 425                 430

aac tct tcc aac ttc aaa aaa gcc aag aga gtc aaa aca gag gat gaa 1344
Asn Ser Ser Asn Phe Lys Lys Ala Lys Arg Val Lys Thr Glu Asp Glu
        435                 440                 445

aaa gag aaa gac cca gag gag aag aaa gaa gtc acc gaa gag gag aaa 1392
Lys Glu Lys Asp Pro Glu Glu Lys Lys Glu Val Thr Glu Glu Glu Lys
    450                 455                 460

acc aag gag gag aag cca gaa gcc aaa ggg gtc aag gag gag gtc aag 1440
Thr Lys Glu Glu Lys Pro Glu Ala Lys Gly Val Lys Glu Glu Val Lys
465                 470                 475                 480

ttg gcc tga 1449
Leu Ala

<210> 12
<211> 482
<212> PRT
<213> Artificial

<220>
<223> Dominant negative mutant (H140K, H141L) of human histone deacetylase-1
(HDAC1) gene

<400> 12
Met Ala Gln Thr Gln Gly Thr Arg Arg Lys Val Cys Tyr Tyr Tyr Asp
1               5                   10                  15

Gly Asp Val Gly Asn Tyr Tyr Tyr Gly Gln Gly His Pro Met Lys Pro
            20                  25                  30

His Arg Ile Arg Met Thr His Asn Leu Leu Leu Asn Tyr Gly Leu Tyr
        35                  40                  45

Arg Lys Met Glu Ile Tyr Arg Pro His Lys Ala Asn Ala Glu Glu Met
    50                  55                  60

Thr Lys Tyr His Ser Asp Asp Tyr Ile Lys Phe Leu Arg Ser Ile Arg
65                  70                  75                  80

Pro Asp Asn Met Ser Glu Tyr Ser Lys Gln Met Gln Arg Phe Asn Val
                85                  90                  95

Gly Glu Asp Cys Pro Val Phe Asp Gly Leu Phe Glu Phe Cys Gln Leu
            100                 105                 110

Ser Thr Gly Gly Ser Val Ala Ser Ala Val Lys Leu Asn Lys Gln Gln
        115                 120                 125

Thr Asp Ile Ala Val Asn Trp Ala Gly Gly Leu Lys Leu Ala Lys Lys
    130                 135                 140

Ser Glu Ala Ser Gly Phe Cys Tyr Val Asn Asp Ile Val Leu Ala Ile
145                 150                 155                 160

Leu Glu Leu Leu Lys Tyr His Gln Arg Val Leu Tyr Ile Asp Ile Asp
                165                 170                 175

Ile His His Gly Asp Gly Val Glu Glu Ala Phe Tyr Thr Thr Asp Arg
            180                 185                 190

Val Met Thr Val Ser Phe His Lys Tyr Gly Glu Tyr Phe Pro Gly Thr
        195                 200                 205

Gly Asp Leu Arg Asp Ile Gly Ala Gly Lys Gly Lys Tyr Tyr Ala Val
    210                 215                 220

Asn Tyr Pro Leu Arg Asp Gly Ile Asp Asp Glu Ser Tyr Glu Ala Ile
225                 230                 235                 240

Phe Lys Pro Val Met Ser Lys Val Met Glu Met Phe Gln Pro Ser Ala
                245                 250                 255

Val Val Leu Gln Cys Gly Ser Asp Ser Leu Ser Gly Asp Arg Leu Gly
            260                 265                 270

Cys Phe Asn Leu Thr Ile Lys Gly His Ala Lys Cys Val Glu Phe Val
        275                 280                 285

Lys Ser Phe Asn Leu Pro Met Leu Met Leu Gly Gly Gly Gly Tyr Thr
    290                 295                 300

Ile Arg Asn Val Ala Arg Cys Arg Thr Tyr Glu Thr Ala Val Ala Leu
305                 310                 315                 320

Asp Thr Glu Ile Pro Asn Glu Leu Pro Tyr Asn Asp Tyr Phe Glu Tyr
                325                 330                 335

Phe Gly Pro Asp Phe Lys Leu His Ile Ser Pro Ser Asn Met Thr Asn
            340                 345                 350

Gln Asn Thr Asn Glu Tyr Leu Glu Lys Ile Lys Gln Arg Leu Phe Glu
        355                 360                 365

Asn Leu Arg Met Leu Pro His Ala Pro Gly Val Gln Met Gln Ala Ile
    370                 375                 380

Pro Glu Asp Ala Ile Pro Glu Glu Ser Gly Asp Glu Asp Glu Asp Asp
385                 390                 395                 400

Pro Asp Lys Arg Ile Ser Ile Cys Ser Ser Asp Lys Arg Ile Ala Cys
                405                 410                 415

Glu Glu Glu Phe Ser Asp Ser Glu Glu Glu Gly Glu Gly Gly Arg Lys
            420                 425                 430

Asn Ser Ser Asn Phe Lys Lys Ala Lys Arg Val Lys Thr Glu Asp Glu
        435                 440                 445

Lys Glu Lys Asp Pro Glu Glu Lys Lys Glu Val Thr Glu Glu Glu Lys
    450                 455                 460

Thr Lys Glu Glu Lys Pro Glu Ala Lys Gly Val Lys Glu Glu Val Lys
465                 470                 475                 480

Leu Ala

<210>  13
<211>  1449
<212>  DNA
<213>  Artificial

<220>
<221>  CDS
<222>  (1)..(1449)
<223>  Dominant negative mutant (H199L) of human histone deacetylase-1 (HDAC1)
       gene

<400>  13
atg gcg cag acg cag ggc acc cgg agg aaa gtc tgt tac tac tac gac        48
Met Ala Gln Thr Gln Gly Thr Arg Arg Lys Val Cys Tyr Tyr Tyr Asp
1               5                   10                  15

ggg gat gtt gga aat tac tat tat gga caa ggc cac cca atg aag cct        96
Gly Asp Val Gly Asn Tyr Tyr Tyr Gly Gln Gly His Pro Met Lys Pro
            20                  25                  30

cac cga atc cgc atg act cat aat ttg ctg ctc aac tat ggt ctc tac       144
His Arg Ile Arg Met Thr His Asn Leu Leu Leu Asn Tyr Gly Leu Tyr
        35                  40                  45

cga aaa atg gaa atc tat cgc cct cac aaa gcc aat gct gag gag atg       192
Arg Lys Met Glu Ile Tyr Arg Pro His Lys Ala Asn Ala Glu Glu Met
    50                  55                  60

acc aag tac cac agc gat gac tac att aaa ttc ttg cgc tcc atc cgt       240
Thr Lys Tyr His Ser Asp Asp Tyr Ile Lys Phe Leu Arg Ser Ile Arg
65                  70                  75                  80

cca gat aac atg tcg gag tac agc aag cag atg cag aga ttc aac gtt       288
Pro Asp Asn Met Ser Glu Tyr Ser Lys Gln Met Gln Arg Phe Asn Val
                85                  90                  95

ggt gag gac tgt cca gta ttc gat ggc ctg ttt gag ttc tgt cag ttg       336
Gly Glu Asp Cys Pro Val Phe Asp Gly Leu Phe Glu Phe Cys Gln Leu
            100                 105                 110

tct act ggt ggt tct gtg gca agt gct gtg aaa ctt aat aag cag cag       384
Ser Thr Gly Gly Ser Val Ala Ser Ala Val Lys Leu Asn Lys Gln Gln
        115                 120                 125

acg gac atc gct gtg aat tgg gct ggg ggc ctg cac cat gca aag aag       432
Thr Asp Ile Ala Val Asn Trp Ala Gly Gly Leu His His Ala Lys Lys

130                 135                 140

tcc gag gca tct ggc ttc tgt tac gtc aat gat atc gtc ttg gcc atc      480
Ser Glu Ala Ser Gly Phe Cys Tyr Val Asn Asp Ile Val Leu Ala Ile
145                 150                 155                 160

ctg gaa ctg cta aag tat cac cag agg gtg ctg tac att gac att gat      528
Leu Glu Leu Leu Lys Tyr His Gln Arg Val Leu Tyr Ile Asp Ile Asp
                165                 170                 175

att cac cat ggt gac ggc gtg gaa gag gcc ttc tac acc acg gac cgg      576
Ile His His Gly Asp Gly Val Glu Glu Ala Phe Tyr Thr Thr Asp Arg
            180                 185                 190

gtc atg act gtg tcc ttt ctt aag tat gga gag tac ttc cca gga act      624
Val Met Thr Val Ser Phe Leu Lys Tyr Gly Glu Tyr Phe Pro Gly Thr
        195                 200                 205

ggg gac cta cgg gat atc ggg gct ggc aaa ggc aag tat tat gct gtt      672
Gly Asp Leu Arg Asp Ile Gly Ala Gly Lys Gly Lys Tyr Tyr Ala Val
    210                 215                 220

aac tac ccg ctc cga gac ggg att gat gac gag tcc tat gag gcc att      720
Asn Tyr Pro Leu Arg Asp Gly Ile Asp Asp Glu Ser Tyr Glu Ala Ile
225                 230                 235                 240

ttc aag ccg gtc atg tcc aaa gta atg gag atg ttc cag cct agt gcg      768
Phe Lys Pro Val Met Ser Lys Val Met Glu Met Phe Gln Pro Ser Ala
                245                 250                 255

gtg gtc tta cag tgt ggc tca gac tcc cta tct ggg gat cgg tta ggt      816
Val Val Leu Gln Cys Gly Ser Asp Ser Leu Ser Gly Asp Arg Leu Gly
            260                 265                 270

tgc ttc aat cta act atc aaa gga cac gcc aag tgt gtg gaa ttt gtc      864
Cys Phe Asn Leu Thr Ile Lys Gly His Ala Lys Cys Val Glu Phe Val
        275                 280                 285

aag agc ttt aac ctg cct atg ctg atg ctg gga ggc ggt ggt tac acc      912
Lys Ser Phe Asn Leu Pro Met Leu Met Leu Gly Gly Gly Gly Tyr Thr
    290                 295                 300

att cgt aac gtt gcc cgg tgc agg aca tat gag aca gct gtg gcc ctg      960
Ile Arg Asn Val Ala Arg Cys Arg Thr Tyr Glu Thr Ala Val Ala Leu
305                 310                 315                 320

gat acg gag atc cct aat gag ctt cca tac aat gac tac ttt gaa tac      1008
Asp Thr Glu Ile Pro Asn Glu Leu Pro Tyr Asn Asp Tyr Phe Glu Tyr
                325                 330                 335

ttt gga cca gat ttc aag ctc cac atc agt cct tcc aat atg act aac      1056
Phe Gly Pro Asp Phe Lys Leu His Ile Ser Pro Ser Asn Met Thr Asn
            340                 345                 350

cag aac acg aat gag tac ctg gag aag atc aaa cag cga ctg ttt gag      1104
Gln Asn Thr Asn Glu Tyr Leu Glu Lys Ile Lys Gln Arg Leu Phe Glu
        355                 360                 365

aac ctt aga atg ctg ccg cac gca cct ggg gtc caa atg cag gcg att      1152
Asn Leu Arg Met Leu Pro His Ala Pro Gly Val Gln Met Gln Ala Ile
    370                 375                 380

cct gag gac gcc atc cct gag gag agt ggc gat gag gac gaa gac gac      1200
Pro Glu Asp Ala Ile Pro Glu Glu Ser Gly Asp Glu Asp Glu Asp Asp
385                 390                 395                 400

cct gac aag cgc atc tcg atc tgc tcc tct gac aaa cga att gcc tgt      1248
Pro Asp Lys Arg Ile Ser Ile Cys Ser Ser Asp Lys Arg Ile Ala Cys
                405                 410                 415

gag gaa gag ttc tcc gat tct gaa gag gag gga gag ggg ggc cgc aag      1296
Glu Glu Glu Phe Ser Asp Ser Glu Glu Glu Gly Glu Gly Gly Arg Lys
            420                 425                 430

aac tct tcc aac ttc aaa aaa gcc aag aga gtc aaa aca gag gat gaa      1344

Asn Ser Ser Asn Phe Lys Lys Ala Lys Arg Val Lys Thr Glu Asp Glu
        435                 440                 445

aaa gag aaa gac cca gag gag aag aaa gaa gtc acc gaa gag gag aaa 1392
Lys Glu Lys Asp Pro Glu Glu Lys Lys Glu Val Thr Glu Glu Glu Lys
    450                 455                 460

acc aag gag gag aag cca gaa gcc aaa ggg gtc aag gag gag gtc aag 1440
Thr Lys Glu Glu Lys Pro Glu Ala Lys Gly Val Lys Glu Glu Val Lys
465                 470                 475                 480

ttg gcc tga 1449
Leu Ala

<210> 14
<211> 482
<212> PRT
<213> Artificial

<220>
<223> Dominant negative mutant (H199L) of human histone deacetylase-1 (HDAC1)
gene

<400> 14
Met Ala Gln Thr Gln Gly Thr Arg Arg Lys Val Cys Tyr Tyr Tyr Asp
1               5                   10                  15

Gly Asp Val Gly Asn Tyr Tyr Tyr Gly Gln Gly His Pro Met Lys Pro
            20                  25                  30

His Arg Ile Arg Met Thr His Asn Leu Leu Leu Asn Tyr Gly Leu Tyr
        35                  40                  45

Arg Lys Met Glu Ile Tyr Arg Pro His Lys Ala Asn Ala Glu Glu Met
    50                  55                  60

Thr Lys Tyr His Ser Asp Asp Tyr Ile Lys Phe Leu Arg Ser Ile Arg
65                  70                  75                  80

Pro Asp Asn Met Ser Glu Tyr Ser Lys Gln Met Gln Arg Phe Asn Val
                85                  90                  95

Gly Glu Asp Cys Pro Val Phe Asp Gly Leu Phe Glu Phe Cys Gln Leu
            100                 105                 110

Ser Thr Gly Gly Ser Val Ala Ser Ala Val Lys Leu Asn Lys Gln Gln
        115                 120                 125

Thr Asp Ile Ala Val Asn Trp Ala Gly Gly Leu His His Ala Lys Lys
    130                 135                 140

Ser Glu Ala Ser Gly Phe Cys Tyr Val Asn Asp Ile Val Leu Ala Ile
145                 150                 155                 160

Leu Glu Leu Leu Lys Tyr His Gln Arg Val Leu Tyr Ile Asp Ile Asp
                165                 170                 175

Ile His His Gly Asp Gly Val Glu Glu Ala Phe Tyr Thr Thr Asp Arg
            180                 185                 190

Val Met Thr Val Ser Phe Leu Lys Tyr Gly Glu Tyr Phe Pro Gly Thr

        195                 200                 205

Gly Asp Leu Arg Asp Ile Gly Ala Gly Lys Gly Lys Tyr Tyr Ala Val
    210                 215                 220

Asn Tyr Pro Leu Arg Asp Gly Ile Asp Asp Glu Ser Tyr Glu Ala Ile
225                 230                 235                 240

Phe Lys Pro Val Met Ser Lys Val Met Glu Met Phe Gln Pro Ser Ala
                245                 250                 255

Val Val Leu Gln Cys Gly Ser Asp Ser Leu Ser Gly Asp Arg Leu Gly
            260                 265                 270

Cys Phe Asn Leu Thr Ile Lys Gly His Ala Lys Cys Val Glu Phe Val
        275                 280                 285

Lys Ser Phe Asn Leu Pro Met Leu Met Leu Gly Gly Gly Gly Tyr Thr
    290                 295                 300

Ile Arg Asn Val Ala Arg Cys Arg Thr Tyr Glu Thr Ala Val Ala Leu
305                 310                 315                 320

Asp Thr Glu Ile Pro Asn Glu Leu Pro Tyr Asn Asp Tyr Phe Glu Tyr
                325                 330                 335

Phe Gly Pro Asp Phe Lys Leu His Ile Ser Pro Ser Asn Met Thr Asn
            340                 345                 350

Gln Asn Thr Asn Glu Tyr Leu Glu Lys Ile Lys Gln Arg Leu Phe Glu
        355                 360                 365

Asn Leu Arg Met Leu Pro His Ala Pro Gly Val Gln Met Gln Ala Ile
    370                 375                 380

Pro Glu Asp Ala Ile Pro Glu Glu Ser Gly Asp Glu Asp Glu Asp Asp
385                 390                 395                 400

Pro Asp Lys Arg Ile Ser Ile Cys Ser Ser Asp Lys Arg Ile Ala Cys
                405                 410                 415

Glu Glu Glu Phe Ser Asp Ser Glu Glu Glu Gly Glu Gly Gly Arg Lys
            420                 425                 430

Asn Ser Ser Asn Phe Lys Lys Ala Lys Arg Val Lys Thr Glu Asp Glu
        435                 440                 445

Lys Glu Lys Asp Pro Glu Glu Lys Lys Glu Val Thr Glu Glu Glu Lys
    450                 455                 460

Thr Lys Glu Glu Lys Pro Glu Ala Lys Gly Val Lys Glu Glu Val Lys
465                 470                 475                 480

Leu Ala

<210> 15
<211> 1287
<212> DNA
<213> Homo sapiens

<220>
<221> CDS
<222> (1)..(1287)
<223> human histone deacetylase-3 (HDAC3) gene

<400> 15
atg gcc aag acc gtg gcc tat ttc tac gac ccc gac gtg ggc aac ttc 48
cac tac gga gct gga cac cct atg aag ccc cat cgc ctg gca ttg acc 96
cat agc ctg gtc ctg cat tac ggt ctc tat aag aag atg atc gtc ttc 144
aag cca tac cag gcc tcc caa cat gac atg tgc cgc ttc cac tcc gag 192
gac tac att gac ttc ctg cag aga gtc agc ccc acc aat atg caa ggc 240
ttc acc aag agt ctt aat gcc ttc aac gta ggc gat gac tgc cca gtg 288
ttt ccc ggg ctc ttt gag ttc tgc tcg cgt tac aca ggc gca tct ctg 336
caa gga gca acc cag ctg aac aac aag atc tgt gat att gcc att aac 384
tgg gct ggt ggt ctg cac cat gcc aag aag ttt gag gcc tct ggc ttc 432
tgc tat gtc aac gac att gtg att ggc atc ctg gag ctg ctc aag tac 480
cac cct cgg gtg ctc tac att gac att gac atc cac cat ggt gac ggg 528
gtt caa gaa gct ttc tac ctc act gac cgg gtc atg acg gtg tcc ttc 576
cac aaa tac gga aat tac ttc ttc cct ggc aca ggt gac atg tat gaa 624
gtc ggg gca gag agt ggc cgc tac tac tgt ctg aac gtg ccc ctg cgg 672
gat ggc att gat gac cag agt tac aag cac ctt ttc cag ccg gtt atc 720
aac cag gta gtg gac ttc tac caa ccc acg tgc att gtg ctc cag tgt 768
gga gct gac tct ctg ggc tgt gat cga ttg ggc tgc ttt aac ctc agc     816
atc cga ggg cat ggg gaa tgc gtt gaa tat gtc aag agc ttc aat atc     864
cct cta ctc gtg ctg ggt ggt ggt ggt tat act gtc cga aat gtt gcc     912
cgc tgc tgg aca tat gag aca tcg ctg ctg gta gaa gag gcc att agt     960
gag gag ctt ccc tat agt gaa tac ttc gag tac ttt gcc cca gac ttc     1008
aca ctt cat cca gat gtc agc acc cgc atc gag aat cag aac tca cgc     1056
cag tat ctg gac cag atc ctc cag aca atc ttt gaa aac ctg aag atg     1104
ctg aac cat gca cct agt gtc cag att cat gac gtg cct gca gac ctc     1152
ctg acc tat gac agg act gat gag gct gat gca gag gag agg ggt cct     1200
gag gag aac tat agc agg cca gag gca ccc aat gag ttc tat gat gga     1248
gac cat gac aat gac aag gaa agc gat gtg gag att taa                 1287

<210>  16
<211>  428
<212>  PRT
<213>  Homo sapiens

<400>  16

<210> 17
<211> 1287
<212> DNA
<213> Artificial

<220>
<221> CDS
<222> (1)..(1287)
<223> Dominant negative mutant (H134K, H135L) of human histone deacetylase-3
      (HDAC3) gene

<400> 17
atg gcc aag acc gtg gcc tat ttc tac gac ccc gac gtg ggc aac ttc      48
Met Ala Lys Thr Val Ala Tyr Phe Tyr Asp Pro Asp Val Gly Asn Phe
1               5                   10                  15

cac tac gga gct gga cac cct atg aag ccc cat cgc ctg gca ttg acc      96
His Tyr Gly Ala Gly His Pro Met Lys Pro His Arg Leu Ala Leu Thr
            20                  25                  30

cat agc ctg gtc ctg cat tac ggt ctc tat aag aag atg atc gtc ttc      144
His Ser Leu Val Leu His Tyr Gly Leu Tyr Lys Lys Met Ile Val Phe
        35                  40                  45

aag cca tac cag gcc tcc caa cat gac atg tgc cgc ttc cac tcc gag      192
Lys Pro Tyr Gln Ala Ser Gln His Asp Met Cys Arg Phe His Ser Glu
    50                  55                  60

gac tac att gac ttc ctg cag aga gtc agc ccc acc aat atg caa ggc      240
Asp Tyr Ile Asp Phe Leu Gln Arg Val Ser Pro Thr Asn Met Gln Gly
65                  70                  75                  80

ttc acc aag agt ctt aat gcc ttc aac gta ggc gat gac tgc cca gtg      288
Phe Thr Lys Ser Leu Asn Ala Phe Asn Val Gly Asp Asp Cys Pro Val
                85                  90                  95

ttt ccc ggg ctc ttt gag ttc tgc tcg cgt tac aca ggc gca tct ctg      336
Phe Pro Gly Leu Phe Glu Phe Cys Ser Arg Tyr Thr Gly Ala Ser Leu
            100                 105                 110

caa gga gca acc cag ctg aac aac aag atc tgt gat att gcc att aac      384
Gln Gly Ala Thr Gln Leu Asn Asn Lys Ile Cys Asp Ile Ala Ile Asn
        115                 120                 125

tgg gct ggt ggt ctg aag ctt gcc aag aag ttt gag gcc tct ggc ttc      432
Trp Ala Gly Gly Leu Lys Leu Ala Lys Lys Phe Glu Ala Ser Gly Phe
    130                 135                 140

tgc tat gtc aac gac att gtg att ggc atc ctg gag ctg ctc aag tac      480
Cys Tyr Val Asn Asp Ile Val Ile Gly Ile Leu Glu Leu Leu Lys Tyr
145                 150                 155                 160

cac cct cgg gtg ctc tac att gac att gac atc cac cat ggt gac ggg      528
His Pro Arg Val Leu Tyr Ile Asp Ile Asp Ile His His Gly Asp Gly
                165                 170                 175

gtt caa gaa gct ttc tac ctc act gac cgg gtc atg acg gtg tcc ttc      576
Val Gln Glu Ala Phe Tyr Leu Thr Asp Arg Val Met Thr Val Ser Phe
            180                 185                 190

cac aaa tac gga aat tac ttc ttc cct ggc aca ggt gac atg tat gaa      624
His Lys Tyr Gly Asn Tyr Phe Phe Pro Gly Thr Gly Asp Met Tyr Glu
        195                 200                 205

gtc ggg gca gag agt ggc cgc tac tac tgt ctg aac gtg ccc ctg cgg      672
Val Gly Ala Glu Ser Gly Arg Tyr Tyr Cys Leu Asn Val Pro Leu Arg

210                 215                 220

gat ggc att gat gac cag agt tac aag cac ctt ttc cag ccg gtt atc        720
Asp Gly Ile Asp Asp Gln Ser Tyr Lys His Leu Phe Gln Pro Val Ile
225                 230                 235                 240

aac cag gta gtg gac ttc tac caa ccc acg tgc att gtg ctc cag tgt        768
Asn Gln Val Val Asp Phe Tyr Gln Pro Thr Cys Ile Val Leu Gln Cys
                245                 250                 255

gga gct gac tct ctg ggc tgt gat cga ttg ggc tgc ttt aac ctc agc        816
Gly Ala Asp Ser Leu Gly Cys Asp Arg Leu Gly Cys Phe Asn Leu Ser
            260                 265                 270

atc cga ggg cat ggg gaa tgc gtt gaa tat gtc aag agc ttc aat atc        864
Ile Arg Gly His Gly Glu Cys Val Glu Tyr Val Lys Ser Phe Asn Ile
        275                 280                 285

cct cta ctc gtg ctg ggt ggt ggt ggt tat act gtc cga aat gtt gcc        912
Pro Leu Leu Val Leu Gly Gly Gly Gly Tyr Thr Val Arg Asn Val Ala
    290                 295                 300

cgc tgc tgg aca tat gag aca tcg ctg ctg gta gaa gag gcc att agt        960
Arg Cys Trp Thr Tyr Glu Thr Ser Leu Leu Val Glu Glu Ala Ile Ser
305                 310                 315                 320

gag gag ctt ccc tat agt gaa tac ttc gag tac ttt gcc cca gac ttc        1008
Glu Glu Leu Pro Tyr Ser Glu Tyr Phe Glu Tyr Phe Ala Pro Asp Phe
                325                 330                 335

aca ctt cat cca gat gtc agc acc cgc atc gag aat cag aac tca cgc        1056
Thr Leu His Pro Asp Val Ser Thr Arg Ile Glu Asn Gln Asn Ser Arg
            340                 345                 350

cag tat ctg gac cag atc ctc cag aca atc ttt gaa aac ctg aag atg        1104
Gln Tyr Leu Asp Gln Ile Leu Gln Thr Ile Phe Glu Asn Leu Lys Met
        355                 360                 365

ctg aac cat gca cct agt gtc cag att cat gac gtg cct gca gac ctc        1152
Leu Asn His Ala Pro Ser Val Gln Ile His Asp Val Pro Ala Asp Leu
    370                 375                 380

ctg acc tat gac agg act gat gag gct gat gca gag gag agg ggt cct        1200
Leu Thr Tyr Asp Arg Thr Asp Glu Ala Asp Ala Glu Glu Arg Gly Pro
385                 390                 395                 400

gag gag aac tat agc agg cca gag gca ccc aat gag ttc tat gat gga        1248
Glu Glu Asn Tyr Ser Arg Pro Glu Ala Pro Asn Glu Phe Tyr Asp Gly
                405                 410                 415

gac cat gac aat gac aag gaa agc gat gtg gag att taa                    1287
Asp His Asp Asn Asp Lys Glu Ser Asp Val Glu Ile
            420                 425

<210>  18
<211>  428
<212>  PRT
<213>  Artificial

<220>
<223>  Dominant negative mutant (H134K, H135L) of human histone deacetylase-3
       (HDAC3) gene

<400>  18
Met Ala Lys Thr Val Ala Tyr Phe Tyr Asp Pro Asp Val Gly Asn Phe
1               5                   10                  15

His Tyr Gly Ala Gly His Pro Met Lys Pro His Arg Leu Ala Leu Thr
            20                  25                  30

His Ser Leu Val Leu His Tyr Gly Leu Tyr Lys Lys Met Ile Val Phe
        35                  40                  45

Lys Pro Tyr Gln Ala Ser Gln His Asp Met Cys Arg Phe His Ser Glu
50                  55                  60

Asp Tyr Ile Asp Phe Leu Gln Arg Val Ser Pro Thr Asn Met Gln Gly
65                  70                  75                  80

Phe Thr Lys Ser Leu Asn Ala Phe Asn Val Gly Asp Asp Cys Pro Val
                85                  90                  95

Phe Pro Gly Leu Phe Glu Phe Cys Ser Arg Tyr Thr Gly Ala Ser Leu
            100                 105                 110

Gln Gly Ala Thr Gln Leu Asn Asn Lys Ile Cys Asp Ile Ala Ile Asn
        115                 120                 125

Trp Ala Gly Gly Leu Lys Leu Ala Lys Lys Phe Glu Ala Ser Gly Phe
    130                 135                 140

Cys Tyr Val Asn Asp Ile Val Ile Gly Ile Leu Glu Leu Leu Lys Tyr
145                 150                 155                 160

His Pro Arg Val Leu Tyr Ile Asp Ile Asp Ile His His Gly Asp Gly
                165                 170                 175

Val Gln Glu Ala Phe Tyr Leu Thr Asp Arg Val Met Thr Val Ser Phe
            180                 185                 190

His Lys Tyr Gly Asn Tyr Phe Phe Pro Gly Thr Gly Asp Met Tyr Glu
        195                 200                 205

Val Gly Ala Glu Ser Gly Arg Tyr Tyr Cys Leu Asn Val Pro Leu Arg
    210                 215                 220

Asp Gly Ile Asp Asp Gln Ser Tyr Lys His Leu Phe Gln Pro Val Ile
225                 230                 235                 240

Asn Gln Val Val Asp Phe Tyr Gln Pro Thr Cys Ile Val Leu Gln Cys
                245                 250                 255

Gly Ala Asp Ser Leu Gly Cys Asp Arg Leu Gly Cys Phe Asn Leu Ser
            260                 265                 270

Ile Arg Gly His Gly Glu Cys Val Glu Tyr Val Lys Ser Phe Asn Ile
        275                 280                 285

Pro Leu Leu Val Leu Gly Gly Gly Gly Tyr Thr Val Arg Asn Val Ala
    290                 295                 300

Arg Cys Trp Thr Tyr Glu Thr Ser Leu Leu Val Glu Glu Ala Ile Ser
305                 310                 315                 320

Glu Glu Leu Pro Tyr Ser Glu Tyr Phe Glu Tyr Phe Ala Pro Asp Phe
                325                 330                 335

Thr Leu His Pro Asp Val Ser Thr Arg Ile Glu Asn Gln Asn Ser Arg

340                 345                 350

Gln Tyr Leu Asp Gln Ile Leu Gln Thr Ile Phe Glu Asn Leu Lys Met
        355                 360                 365

Leu Asn His Ala Pro Ser Val Gln Ile His Asp Val Pro Ala Asp Leu
    370                 375                 380

Leu Thr Tyr Asp Arg Thr Asp Glu Ala Asp Ala Glu Glu Arg Gly Pro
385                 390                 395                 400

Glu Glu Asn Tyr Ser Arg Pro Glu Ala Pro Asn Glu Phe Tyr Asp Gly
                405                 410                 415

Asp His Asp Asn Asp Lys Glu Ser Asp Val Glu Ile
            420                 425

<210>  19
<211>  1287
<212>  DNA
<213>  Artificial

<220>
<221>  CDS
<222>  (1)..(1287)
<223>  Dominant negative mutant (H193L) of human histone deacetylase-3 (HDAC3)
       gene

<400>  19
atg gcc aag acc gtg gcc tat ttc tac gac ccc gac gtg ggc aac ttc      48
Met Ala Lys Thr Val Ala Tyr Phe Tyr Asp Pro Asp Val Gly Asn Phe
1               5                   10                  15

cac tac gga gct gga cac cct atg aag ccc cat cgc ctg gca ttg acc      96
His Tyr Gly Ala Gly His Pro Met Lys Pro His Arg Leu Ala Leu Thr
            20                  25                  30

cat agc ctg gtc ctg cat tac ggt ctc tat aag aag atg atc gtc ttc      144
His Ser Leu Val Leu His Tyr Gly Leu Tyr Lys Lys Met Ile Val Phe
        35                  40                  45

aag cca tac cag gcc tcc caa cat gac atg tgc cgc ttc cac tcc gag      192
Lys Pro Tyr Gln Ala Ser Gln His Asp Met Cys Arg Phe His Ser Glu
    50                  55                  60

gac tac att gac ttc ctg cag aga gtc agc ccc acc aat atg caa ggc      240
Asp Tyr Ile Asp Phe Leu Gln Arg Val Ser Pro Thr Asn Met Gln Gly
65                  70                  75                  80

ttc acc aag agt ctt aat gcc ttc aac gta ggc gat gac tgc cca gtg      288
Phe Thr Lys Ser Leu Asn Ala Phe Asn Val Gly Asp Asp Cys Pro Val
                85                  90                  95

ttt ccc ggg ctc ttt gag ttc tgc tcg cgt tac aca ggc gca tct ctg      336
Phe Pro Gly Leu Phe Glu Phe Cys Ser Arg Tyr Thr Gly Ala Ser Leu
            100                 105                 110

caa gga gca acc cag ctg aac aac aag atc tgt gat att gcc att aac      384
Gln Gly Ala Thr Gln Leu Asn Asn Lys Ile Cys Asp Ile Ala Ile Asn
        115                 120                 125

tgg gct ggt ggt ctg cac cat gcc aag aag ttt gag gcc tct ggc ttc      432
Trp Ala Gly Gly Leu His His Ala Lys Lys Phe Glu Ala Ser Gly Phe
    130                 135                 140

tgc tat gtc aac gac att gtg att ggc atc ctg gag ctg ctc aag tac      480
Cys Tyr Val Asn Asp Ile Val Ile Gly Ile Leu Glu Leu Leu Lys Tyr
145                 150                 155                 160

cac cct cgg gtg ctc tac att gac att gac atc cac cat ggt gac ggg 528
His Pro Arg Val Leu Tyr Ile Asp Ile Asp Ile His His Gly Asp Gly
                165                 170                 175

gtt caa gaa gct ttc tac ctc act gac cgg gtc atg acg gtg tcc ttc 576
Val Gln Glu Ala Phe Tyr Leu Thr Asp Arg Val Met Thr Val Ser Phe
            180                 185                 190

ctt aaa tac gga aat tac ttc ttc cct ggc aca ggt gac atg tat gaa 624
Leu Lys Tyr Gly Asn Tyr Phe Phe Pro Gly Thr Gly Asp Met Tyr Glu
        195                 200                 205

gtc ggg gca gag agt ggc cgc tac tac tgt ctg aac gtg ccc ctg cgg 672
Val Gly Ala Glu Ser Gly Arg Tyr Tyr Cys Leu Asn Val Pro Leu Arg
    210                 215                 220

gat ggc att gat gac cag agt tac aag cac ctt ttc cag ccg gtt atc 720
Asp Gly Ile Asp Asp Gln Ser Tyr Lys His Leu Phe Gln Pro Val Ile
225                 230                 235                 240

aac cag gta gtg gac ttc tac caa ccc acg tgc att gtg ctc cag tgt 768
Asn Gln Val Val Asp Phe Tyr Gln Pro Thr Cys Ile Val Leu Gln Cys
                245                 250                 255

gga gct gac tct ctg ggc tgt gat cga ttg ggc tgc ttt aac ctc agc 816
Gly Ala Asp Ser Leu Gly Cys Asp Arg Leu Gly Cys Phe Asn Leu Ser
            260                 265                 270

atc cga ggg cat ggg gaa tgc gtt gaa tat gtc aag agc ttc aat atc 864
Ile Arg Gly His Gly Glu Cys Val Glu Tyr Val Lys Ser Phe Asn Ile
        275                 280                 285

cct cta ctc gtg ctg ggt ggt ggt ggt tat act gtc cga aat gtt gcc 912
Pro Leu Leu Val Leu Gly Gly Gly Gly Tyr Thr Val Arg Asn Val Ala
    290                 295                 300

cgc tgc tgg aca tat gag aca tcg ctg ctg gta gaa gag gcc att agt 960
Arg Cys Trp Thr Tyr Glu Thr Ser Leu Leu Val Glu Glu Ala Ile Ser
305                 310                 315                 320

gag gag ctt ccc tat agt gaa tac ttc gag tac ttt gcc cca gac ttc 1008
Glu Glu Leu Pro Tyr Ser Glu Tyr Phe Glu Tyr Phe Ala Pro Asp Phe
                325                 330                 335

aca ctt cat cca gat gtc agc acc cgc atc gag aat cag aac tca cgc 1056
Thr Leu His Pro Asp Val Ser Thr Arg Ile Glu Asn Gln Asn Ser Arg
            340                 345                 350

cag tat ctg gac cag atc ctc cag aca atc ttt gaa aac ctg aag atg 1104
Gln Tyr Leu Asp Gln Ile Leu Gln Thr Ile Phe Glu Asn Leu Lys Met
        355                 360                 365

ctg aac cat gca cct agt gtc cag att cat gac gtg cct gca gac ctc 1152
Leu Asn His Ala Pro Ser Val Gln Ile His Asp Val Pro Ala Asp Leu
    370                 375                 380

ctg acc tat gac agg act gat gag gct gat gca gag gag agg ggt cct 1200
Leu Thr Tyr Asp Arg Thr Asp Glu Ala Asp Ala Glu Glu Arg Gly Pro
385                 390                 395                 400

gag gag aac tat agc agg cca gag gca ccc aat gag ttc tat gat gga 1248
Glu Glu Asn Tyr Ser Arg Pro Glu Ala Pro Asn Glu Phe Tyr Asp Gly
                405                 410                 415

gac cat gac aat gac aag gaa agc gat gtg gag att taa 1287
Asp His Asp Asn Asp Lys Glu Ser Asp Val Glu Ile
            420                 425

<210> 20
<211> 428
<212> PRT
<213> Artificial

<220>

<223>  Dominant negative mutant (H193L) of human histone deacetylase-3 (HDAC3)
       gene

<400>  20
Met Ala Lys Thr Val Ala Tyr Phe Tyr Asp Pro Asp Val Gly Asn Phe
1               5                   10                  15

His Tyr Gly Ala Gly His Pro Met Lys Pro His Arg Leu Ala Leu Thr
            20                  25                  30

His Ser Leu Val Leu His Tyr Gly Leu Tyr Lys Lys Met Ile Val Phe
        35                  40                  45

Lys Pro Tyr Gln Ala Ser Gln His Asp Met Cys Arg Phe His Ser Glu
    50                  55                  60

Asp Tyr Ile Asp Phe Leu Gln Arg Val Ser Pro Thr Asn Met Gln Gly
65                  70                  75                  80

Phe Thr Lys Ser Leu Asn Ala Phe Asn Val Gly Asp Asp Cys Pro Val
                85                  90                  95

Phe Pro Gly Leu Phe Glu Phe Cys Ser Arg Tyr Thr Gly Ala Ser Leu
            100                 105                 110

Gln Gly Ala Thr Gln Leu Asn Asn Lys Ile Cys Asp Ile Ala Ile Asn
        115                 120                 125

Trp Ala Gly Gly Leu His His Ala Lys Lys Phe Glu Ala Ser Gly Phe
    130                 135                 140

Cys Tyr Val Asn Asp Ile Val Ile Gly Ile Leu Glu Leu Leu Lys Tyr
145                 150                 155                 160

His Pro Arg Val Leu Tyr Ile Asp Ile Asp Ile His His Gly Asp Gly
                165                 170                 175

Val Gln Glu Ala Phe Tyr Leu Thr Asp Arg Val Met Thr Val Ser Phe
            180                 185                 190

Leu Lys Tyr Gly Asn Tyr Phe Phe Pro Gly Thr Gly Asp Met Tyr Glu
        195                 200                 205

Val Gly Ala Glu Ser Gly Arg Tyr Tyr Cys Leu Asn Val Pro Leu Arg
    210                 215                 220

Asp Gly Ile Asp Asp Gln Ser Tyr Lys His Leu Phe Gln Pro Val Ile
225                 230                 235                 240

Asn Gln Val Val Asp Phe Tyr Gln Pro Thr Cys Ile Val Leu Gln Cys
                245                 250                 255

Gly Ala Asp Ser Leu Gly Cys Asp Arg Leu Gly Cys Phe Asn Leu Ser
            260                 265                 270

Ile Arg Gly His Gly Glu Cys Val Glu Tyr Val Lys Ser Phe Asn Ile
        275                 280                 285

Pro Leu Leu Val Leu Gly Gly Gly Gly Tyr Thr Val Arg Asn Val Ala
    290                 295                 300

Arg Cys Trp Thr Tyr Glu Thr Ser Leu Leu Val Glu Glu Ala Ile Ser
305                 310                 315                 320

Glu Glu Leu Pro Tyr Ser Glu Tyr Phe Glu Tyr Phe Ala Pro Asp Phe
                325                 330                 335

Thr Leu His Pro Asp Val Ser Thr Arg Ile Glu Asn Gln Asn Ser Arg
            340                 345                 350

Gln Tyr Leu Asp Gln Ile Leu Gln Thr Ile Phe Glu Asn Leu Lys Met
        355                 360                 365

Leu Asn His Ala Pro Ser Val Gln Ile His Asp Val Pro Ala Asp Leu
    370                 375                 380

Leu Thr Tyr Asp Arg Thr Asp Glu Ala Asp Ala Glu Glu Arg Gly Pro
385                 390                 395                 400

Glu Glu Asn Tyr Ser Arg Pro Glu Ala Pro Asn Glu Phe Tyr Asp Gly
                405                 410                 415

Asp His Asp Asn Asp Lys Glu Ser Asp Val Glu Ile
            420                 425

<210> 21
<211> 1458
<212> DNA
<213> Homo sapiens

<220>
<221> CDS
<222> (1)..(1458)
<223> human N-CoR (RD3) gene

<400> 21
gaa gtc ctt cag cct gct cca cat caa gtg ata act aat ctc cct gaa 48
Glu Val Leu Gln Pro Ala Pro His Gln Val Ile Thr Asn Leu Pro Glu
1               5                   10                  15

ggc gtt cgg ctt ccg aca act cga cca acc agg cca ccg ccc cct ctc 96
Gly Val Arg Leu Pro Thr Thr Arg Pro Thr Arg Pro Pro Pro Pro Leu
            20                  25                  30

atc ccg tca tcc aaa acc aca gtg gct tca gaa aaa cca tct ttt ata 144
Ile Pro Ser Ser Lys Thr Thr Val Ala Ser Glu Lys Pro Ser Phe Ile
        35                  40                  45

atg gga ggc tcc atc tca cag gga aca cca ggc act tat ttg act tct 192
Met Gly Gly Ser Ile Ser Gln Gly Thr Pro Gly Thr Tyr Leu Thr Ser
    50                  55                  60

cat aat cag gct tcc tac act caa gaa aca ccc aag ccg tca gta gga 240
His Asn Gln Ala Ser Tyr Thr Gln Glu Thr Pro Lys Pro Ser Val Gly
65                  70                  75                  80

tct atc tct ctt gga ctg cca cgg caa cag gaa tct gcc aaa tca gct 288
Ser Ile Ser Leu Gly Leu Pro Arg Gln Gln Glu Ser Ala Lys Ser Ala
                85                  90                  95

act ttg ccc tac atc aag cag gaa gaa ttt tct ccc cga agc caa aac 336
Thr Leu Pro Tyr Ile Lys Gln Glu Glu Phe Ser Pro Arg Ser Gln Asn
            100                 105                 110

tca caa cct gag ggt ctg ttg gtc agg gcc caa cat gaa ggt gta gtc 384
Ser Gln Pro Glu Gly Leu Leu Val Arg Ala Gln His Glu Gly Val Val
        115                 120                 125

aga ggt acc gca gga gcc ata caa gaa gga agt ata act cgg gga act 432
Arg Gly Thr Ala Gly Ala Ile Gln Glu Gly Ser Ile Thr Arg Gly Thr
    130                 135                 140

cca acc agc aaa att tca gtg gag agc att cca tcc cta cgg ggc tct 480
Pro Thr Ser Lys Ile Ser Val Glu Ser Ile Pro Ser Leu Arg Gly Ser
145                 150                 155                 160

atc act cag ggc acc ccg gct ctg ccc cag act ggc ata cca aca gag 528
Ile Thr Gln Gly Thr Pro Ala Leu Pro Gln Thr Gly Ile Pro Thr Glu
                165                 170                 175

gct ttg gtg aag ggg tcc att tcg aga atg ccc att gaa gac agc agt 576
Ala Leu Val Lys Gly Ser Ile Ser Arg Met Pro Ile Glu Asp Ser Ser
            180                 185                 190

cct gag aaa ggc aga gag gaa gct gca tcc aaa ggc cat gtt att tat 624
Pro Glu Lys Gly Arg Glu Glu Ala Ala Ser Lys Gly His Val Ile Tyr
        195                 200                 205

gaa ggc aaa agt gga cat atc ttg tca tat gat aat att aag aat gcc 672
Glu Gly Lys Ser Gly His Ile Leu Ser Tyr Asp Asn Ile Lys Asn Ala
    210                 215                 220

cga gaa ggg act agg agt cca aga aca gct cat gaa atc agt tta aag 720
Arg Glu Gly Thr Arg Ser Pro Arg Thr Ala His Glu Ile Ser Leu Lys
225                 230                 235                 240

aga agc tat gaa tca gtg gaa gga aat ata aag caa ggg atg tca atg 768
Arg Ser Tyr Glu Ser Val Glu Gly Asn Ile Lys Gln Gly Met Ser Met
                245                 250                 255

agg gag tct cct gta tca gca ccg tta gag ggg ctg ata tgc cga gca 816
Arg Glu Ser Pro Val Ser Ala Pro Leu Glu Gly Leu Ile Cys Arg Ala
            260                 265                 270

tta ccc agg ggg agt cct cat tct gac ctc aaa gaa agg act gta ttg 864
Leu Pro Arg Gly Ser Pro His Ser Asp Leu Lys Glu Arg Thr Val Leu
        275                 280                 285

tct ggc tcc ata atg cag ggg aca cca aga gca aca act gaa agc ttt 912
Ser Gly Ser Ile Met Gln Gly Thr Pro Arg Ala Thr Thr Glu Ser Phe
    290                 295                 300

gaa gat ggc ctt aaa tat ccc aaa caa att aaa agg gaa agt cct ccc 960
Glu Asp Gly Leu Lys Tyr Pro Lys Gln Ile Lys Arg Glu Ser Pro Pro
305                 310                 315                 320

ata cga gca ttt gaa ggt gcc att acc aaa gga aaa cca tat gat ggc 1008
Ile Arg Ala Phe Glu Gly Ala Ile Thr Lys Gly Lys Pro Tyr Asp Gly
                325                 330                 335

atc acc acc atc aaa gaa atg ggg cgt tcc att cat gag att cca agg 1056
Ile Thr Thr Ile Lys Glu Met Gly Arg Ser Ile His Glu Ile Pro Arg
            340                 345                 350

caa gat att tta act cag gaa agt cgg aaa act cca gaa gtg gtc cag 1104
Gln Asp Ile Leu Thr Gln Glu Ser Arg Lys Thr Pro Glu Val Val Gln
        355                 360                 365

agc aca cgg ccg ata att gag ggt tcc att tcc cag ggc aca cca ata 1152
Ser Thr Arg Pro Ile Ile Glu Gly Ser Ile Ser Gln Gly Thr Pro Ile
    370                 375                 380

aag ttt gac aac aac tca ggt caa tct gcc atc aaa cac aat gtc aaa 1200
Lys Phe Asp Asn Asn Ser Gly Gln Ser Ala Ile Lys His Asn Val Lys
385                 390                 395                 400

tcc tta atc acg ggg cct agc aaa cta tcc cgt gga atg cct ccg ctg 1248
Ser Leu Ile Thr Gly Pro Ser Lys Leu Ser Arg Gly Met Pro Pro Leu

405                    410                    415

gaa att gtg cca gag aac ata aaa gtg gta gaa cgg gga aaa tat gag        1296
Glu Ile Val Pro Glu Asn Ile Lys Val Val Glu Arg Gly Lys Tyr Glu
            420                 425                 430

gat gtg aaa gca ggc gag acc gtg cgt tcc cgg cac acg tca gtg gta        1344
Asp Val Lys Ala Gly Glu Thr Val Arg Ser Arg His Thr Ser Val Val
        435                 440                 445

agc tct ggc ccc tcc gtt ctt agg tcc aca ctg cat gaa gct ccc aaa        1392
Ser Ser Gly Pro Ser Val Leu Arg Ser Thr Leu His Glu Ala Pro Lys
    450                 455                 460

gca caa ctg agc cct ggg att tat gat gac acc agt gca cgg agg acc        1440
Ala Gln Leu Ser Pro Gly Ile Tyr Asp Asp Thr Ser Ala Arg Arg Thr
465                 470                 475                 480

cct gtg agt tat caa aac                                                1458
Pro Val Ser Tyr Gln Asn
                485

<210>  22
<211>  486
<212>  PRT
<213>  Homo sapiens

<400>  22
Glu Val Leu Gln Pro Ala Pro His Gln Val Ile Thr Asn Leu Pro Glu
1               5                   10                  15

Gly Val Arg Leu Pro Thr Thr Arg Pro Thr Arg Pro Pro Pro Pro Leu
            20                  25                  30

Ile Pro Ser Ser Lys Thr Thr Val Ala Ser Glu Lys Pro Ser Phe Ile
        35                  40                  45

Met Gly Gly Ser Ile Ser Gln Gly Thr Pro Gly Thr Tyr Leu Thr Ser
    50                  55                  60

His Asn Gln Ala Ser Tyr Thr Gln Glu Thr Pro Lys Pro Ser Val Gly
65                  70                  75                  80

Ser Ile Ser Leu Gly Leu Pro Arg Gln Gln Glu Ser Ala Lys Ser Ala
                85                  90                  95

Thr Leu Pro Tyr Ile Lys Gln Glu Glu Phe Ser Pro Arg Ser Gln Asn
            100                 105                 110

Ser Gln Pro Glu Gly Leu Leu Val Arg Ala Gln His Glu Gly Val Val
        115                 120                 125

Arg Gly Thr Ala Gly Ala Ile Gln Glu Gly Ser Ile Thr Arg Gly Thr
    130                 135                 140

Pro Thr Ser Lys Ile Ser Val Glu Ser Ile Pro Ser Leu Arg Gly Ser
145                 150                 155                 160

Ile Thr Gln Gly Thr Pro Ala Leu Pro Gln Thr Gly Ile Pro Thr Glu
                165                 170                 175

Ala Leu Val Lys Gly Ser Ile Ser Arg Met Pro Ile Glu Asp Ser Ser
            180                 185                 190

Pro Glu Lys Gly Arg Glu Glu Ala Ala Ser Lys Gly His Val Ile Tyr
        195                 200                 205

Glu Gly Lys Ser Gly His Ile Leu Ser Tyr Asp Asn Ile Lys Asn Ala
    210                 215                 220

Arg Glu Gly Thr Arg Ser Pro Arg Thr Ala His Glu Ile Ser Leu Lys
225                 230                 235                 240

Arg Ser Tyr Glu Ser Val Glu Gly Asn Ile Lys Gln Gly Met Ser Met
                245                 250                 255

Arg Glu Ser Pro Val Ser Ala Pro Leu Glu Gly Leu Ile Cys Arg Ala
            260                 265                 270

Leu Pro Arg Gly Ser Pro His Ser Asp Leu Lys Glu Arg Thr Val Leu
        275                 280                 285

Ser Gly Ser Ile Met Gln Gly Thr Pro Arg Ala Thr Thr Glu Ser Phe
    290                 295                 300

Glu Asp Gly Leu Lys Tyr Pro Lys Gln Ile Lys Arg Glu Ser Pro Pro
305                 310                 315                 320

Ile Arg Ala Phe Glu Gly Ala Ile Thr Lys Gly Lys Pro Tyr Asp Gly
                325                 330                 335

Ile Thr Thr Ile Lys Glu Met Gly Arg Ser Ile His Glu Ile Pro Arg
            340                 345                 350

Gln Asp Ile Leu Thr Gln Glu Ser Arg Lys Thr Pro Glu Val Val Gln
        355                 360                 365

Ser Thr Arg Pro Ile Ile Glu Gly Ser Ile Ser Gln Gly Thr Pro Ile
    370                 375                 380

Lys Phe Asp Asn Asn Ser Gly Gln Ser Ala Ile Lys His Asn Val Lys
385                 390                 395                 400

Ser Leu Ile Thr Gly Pro Ser Lys Leu Ser Arg Gly Met Pro Pro Leu
                405                 410                 415

Glu Ile Val Pro Glu Asn Ile Lys Val Val Glu Arg Gly Lys Tyr Glu
            420                 425                 430

Asp Val Lys Ala Gly Glu Thr Val Arg Ser Arg His Thr Ser Val Val
        435                 440                 445

Ser Ser Gly Pro Ser Val Leu Arg Ser Thr Leu His Glu Ala Pro Lys
    450                 455                 460

Ala Gln Leu Ser Pro Gly Ile Tyr Asp Asp Thr Ser Ala Arg Arg Thr
465                 470                 475                 480

Pro Val Ser Tyr Gln Asn

                485

<210> 23
<211> 731
<212> DNA
<213> Homo sapiens

<220>
<221> misc_feature
<222> (1)..(731)
<223> 5'-flanking region of Human interleukin-2 (IL-2) gene

<400> 23
ctgagtattt aacaatcgca ccctttaaaa aatgtacaat agacattaag agacttaaac 60

agatatataa tcattttaaa ttaaaatagc gttaaacagt acctcaagct caataagcat 120

tttaagtatt ctaatcttag tatttctcta gctgacatgt aagaagcaat ctatcttatt 180

gtatgcaatt agctcattgt gtggataaaa aggtaaaacc attctgaaac aggaaaccaa 240

tacacttcct gtttaatcaa caaatctaaa catttattct tttcatctgt ttactcttgc 300

tcttgtccac cacaatatgc tattcacatg ttcagtgtag ttttaggaca aagaaaattt 360

tctgagttac ttttgtatcc ccaccccctt aaagaaagga ggaaaaactg tttcatacag 420

aaggcgttaa ttgcatgaat tagagctatc acctaagtgt gggctaatgt aacaaagagg 480

gatttcacct acatccattc agtcagtctt tgggggttta aagaaattcc aaagagtcat 540

cagaagagga aaaatgaagg taatgttttt tcagacaggt aaagtctttg aaaatatgtg 600

taatatgtaa aacattttga cacccccata atatttttcc agaattaaca gtataaattg 660

catctcttgt tcaagagttc cctatcactc tctttaatca ctactcacag taacctcaac 720

tcctgccaca a 731

<210> 24
<211> 527
<212> DNA
<213> Homo sapiens

<220>
<221> misc_feature
<222> (73)..(506)
<223> 5'-flanking region of Human interleukin-2 (IL-2) gene

<400> 24
gggnnnnnng gngatcctct accactatag ggcgaattga atttagcggc cgcganttcg 60

cccttcgcta gctgctcttg tccaccacaa tatgctattc acatgttcag tgtagtttta 120

ggacaaagaa aattttctga gttacttttg tatccccacc cccttaaaga aaggaggaaa 180

aactgtttca tacagaaggc gttaattgca tgaattagag ctatcaccta agtgtgggct 240

aatgtaacaa agagggattt cacctacatc cattcagtca gtctttgggg gtttaaagaa 300

attccaaaga gtcatcagaa gaggaaaaat gaaggtaatg ttttttcaga caggtaaagt 360

ctttgaaaat atgtgtaata tgtaaaacat tttgacaccc ccataatatt tttccagaat 420

taacagtata aattgcatct cttgttcaag agttccctat cactctcttt aatcactact 480

cacagtaacc tcaactcctg ccacaagctt cgaagggcga attcgtt 527

<210> 25
<211> 900
<212> DNA
<213> Homo sapiens

<220>
<221> misc_feature
<222> (57)..(875)
<223> Promoter region of Human GATA-1 gene

<400> 25
tgcctcttta ctatagggcg aattgaattt agcggccgcg aattcgccct tgatctatcc 60

ctggctccca cctcagtttc ccgcctccaa ggcagcatgg cgggcaagaa gttgaggcca 120

ctgtccctgg gtgttcctac ccccacaccc tcaccccaag acagcctgtt actgcggcgc 180

caacagccac ggtcgcctac atctgataag acttatctgc tgccccaggg caggccggag 240

ctggcgtaag ccccagtggg gcgctaagtg agtgtgcccc tgcctcccgc cagcactggc 300

ctggcctgca ggcttagcct gggtcatcaa ggtatcccac aggctctagt tcaaatccag 360

cagaacctct ctgagcctca ctcttctcac ctgcaaaatg ggtacagcca catcccttct 420

ctccctgcag ccaggaagac gcacatacac aggagtctag cccacaccgg ccccgcacaa 480

attaagggct ttactctctg aaaagcccag tgaagtcatg aaaccatatc tgctattttc 540

atttatcttg gtttcagcct attttgcttg tctggacact acagtccacg ggagcctagg 600

tcgagcgagg tccaagaatc cccagggtgg gcagggaggg tggaagaggg cctccagtgc 660

ccaagaggtg ccccacaagc atgggacccg ccccctcccc tggactgccc cacccactgg 720

ggcaccagcc actccctggg gaggagggag gagggagaag ggagggaggg agggagggag 780

gaagggagcc tcaaaggcca aggccagcca ggacaccccc tgggatcaca ctgagcttgc 840

cacatcccca aggcggccga accctccgca accaccaaag cttataaggg cgaattcgtt 900


<210> 26
<211> 660
<212> DNA
<213> Homo sapiens

<220>
<221> misc_feature
<222> (11)..(647)
<223> Promoter region of Human GATA-1 gene

<400> 26
ttatggtacc accccagaag atgccaggag ggagtgagcc agtcagggaa ggcttccgag 60

aagagaggac attgaagaag agtctcaaac ttaggcctga cggagaagac gcgcggccag 120

gacaccccac ccccgccctc gtctccccca aagcctgatc tggccccact gattccctta 180

tctgcccact cccagctgcc tccttgctgg ctgaactgtc gccgcagact tctgagcctg 240

cgccccctcc acggggatgg gggagggaat ggggtgaggc ctggcctcac agcctcgggg 300

tttccagctc ttgctggagg cagggctctg ggcgccccta ctcctcaccc ttggcttctc 360

ttcctgagcg ctctgtgctc tccagaaatg aagaaatggg gtgagtccag cggccaaacc 420

cttgtcttag ctcttagaca tgcctcgagc ctgccattcc ctgtgaggac agatttccct 480

atgttgcgac cgctgcttct aataataata atgatgatga taattcccat ttacagagca 540

caccatttat ggtgtgccag caggccctgt gctgagtggt tcctacccac gtggggggct 600

aggactttac ccgttttcca gatgaagaaa ctgaggctca gagggcgcta gcataagggc 660


<210> 27
<211> 32
<212> DNA

<213>  Artificial

<220>
<223>  PCR primer for amplifying the 5'-flanking region of Human interleukin-2
       (IL-2) gene

<400>  27
tcgctagcct gagtatttaa caatcgcacc ct                                    32


<210>  28
<211>  30
<212>  DNA
<213>  Artificial

<220>
<223>  PCR primer for amplifying the 5'-flanking region of Human interleukin-2
       (IL-2) gene

<400>  28
cgaagcttgt ggcaggagtt gaggttactg                                       30


<210>  29
<211>  777
<212>  DNA
<213>  Homo sapiens

<220>
<221>  misc_feature
<222>  (1)..(777)
<223>  Corresponding to the sequence (+661 to +1437) in the GenBank database
       (Acession: HSIL05)

<400>  29
atcagtatcc ttgaatcgaa acctttttct gagtatttaa caatcgcacc ctttaaaaaa      60

tgtacataga cattaagaga cttaaacaga tatataatca ttttaaatta aaatagcgtt     120

aaacagtacc tcaagctcaa taagcatttt aagtattcta atcttagtat ttctctagct     180

gacatgtaag aagcaatcta tcttattgta tgcaattagc tctttgtgtg gataaaaagg     240

taaaaccatt ctgaaacagg aaaccaatac acttcctgtt taatcaacaa atctaaacat     300

ttattctttt catctgttta ctcttgctct tgtccaccac aatatgctat tcacatgttc     360

agtgtagttt tatgacaaag aaaattttct gagttacttt tgtatcccca cccccttaaa     420

gaaaggagga aaaactgttt catacagaag gcgttaattg catgaattag agctatcacc     480

taagtgtggg ctaatgtaac aaagagggat ttcacctaca tccattcagt cagtctttgg     540

gggtttaaag aaattccaaa gagtcatcag aagaggaaaa atgaaggtaa tgttttttca     600

gactggtaaa gtctttgaaa atatgtgtaa tatgtaaaac attttgacac ccccataata     660

tttttccaga attaacagta taaattgcat ctcttgttca agagttccct atcactcttt     720

aatcactact cacagtaacc tcaactcctg ccacaatgta caggatgcaa ctcctgt        777


<210>  30
<211>  30
<212>  DNA
<213>  Artificial

<220>
<223>  PCR primer for amplifying the 5'-flanking region of Human interleukin-2
       (IL-2) gene

<400>  30
cgctagctgc tcttgtccac cacaatatgc                                       30

<210>  31
<211>  538
<212>  DNA
<213>  Homo sapiens

<220>
<221>  misc_feature
<222>  (1)..(538)
<223>  Corresponding to the sequence (+901 to +1438) in the GenBank database
       (Acession: HSIL05)

<400>  31
taaaaccatt ctgaaacagg aaaccaatac acttcctgtt taatcaacaa atctaaacat      60

ttattctttt catctgttta ctcttgctct tgtccaccac aatatgctat tcacatgttc     120

agtgtagttt tatgacaaag aaaattttct gagttacttt tgtatcccca cccccttaaa     180

gaaaggagga aaaactgttt catacagaag gcgttaattg catgaattag agctatcacc     240

taagtgtggg ctaatgtaac aaagagggat ttcacctaca tccattcagt cagtctttgg     300

gggtttaaag aaattccaaa gagtcatcag aagaggaaaa atgaaggtaa tgttttttca     360

gactggtaaa gtctttgaaa atatgtgtaa tatgtaaaac attttgacac ccccataata     420

tttttccaga attaacagta taaattgcat ctcttgttca agagttccct atcactcttt     480

aatcactact cacagtaacc tcaactcctg ccacaatgta caggatgcaa ctcctgtc       538


<210>  32
<211>  28
<212>  DNA
<213>  Artificial

<220>
<223>  PCR primer for amplifying the promoter region of Human GATA-1 gene

<400>  32
atagatctat ccctggctcc cacctcag                                          28


<210>  33
<211>  28
<212>  DNA
<213>  Artificial

<220>
<223>  PCR primer for amplifying the promoter region of Human GATA-1 gene

<400>  33
ataagctttg gtggttgcgg agggttcg                                          28


<210>  34
<211>  28
<212>  DNA
<213>  Artificial

<220>
<223>  PCR primer for amplifying the promoter region of Human GATA-1 gene

<400>  34
atggtaccac cccagaagat gccaggag                                          28


<210>  35
<211>  28
<212>  DNA
<213>  Artificial

<220>
<223>  PCR primer for amplifying the promoter region of Human GATA-1 gene

<400>  35
atgctagcgc cctctgagcc tcagtttc                                          28


<210>  36
<211>  23
<212>  DNA
<213>  Artificial

<220>
<223>  PCR primer for amplifying a human histone deacetylase-1 (HDAC1) gene

<400>  36
gaggaattca agatggcgca gac                                               23


<210>  37
<211>  25
<212>  DNA
<213>  Artificial

<220>
<223>  PCR primer for amplifying a human histone deacetylase-1 (HDAC1) gene

<400>  37
ggagcggccg cttcaggcca acttg                                             25


<210>  38
<211>  24
<212>  DNA
<213>  Artificial

<220>
<223>  PCR primer for amplifying a human histone deacetylase-2 (HDAC2) gene

<400>  38
ggggatccat ggcgtacagt caag                                              24


<210>  39
<211>  29
<212>  DNA
<213>  Artificial

<220>
<223>  PCR primer for amplifying a human histone deacetylase-2 (HDAC2) gene

<400>  39
ggtgcggccg ccaaattcag gggttgctg                                         29


<210>  40
<211>  22
<212>  DNA
<213>  Artificial

<220>
<223>  PCR primer for amplifying a human histone deacetylase-3 (HDAC3) gene

<400>  40
ccggatccac catggccaag ac                                                22


<210>  41
<211>  28
<212>  DNA
<213>  Artificial

<220>
<223>  PCR primer for amplifying a human histone deacetylase-3 (HDAC3) gene

<400>  41
gcagcggccg ccactcttaa atctccac                                          28

<210>  42
<211>  28
<212>  DNA
<213>  Artificial

<220>
<223>  PCR primer for amplifying a human histone deacetylase-4 (HDAC4) gene

<400>  42
gggaattcat gagctcccaa agccatcc                                          28


<210>  43
<211>  29
<212>  DNA
<213>  Artificial

<220>
<223>  PCR primer for amplifying a human histone deacetylase-4 (HDAC4) gene

<400>  43
aaggcgccgc agcttcgagg gagtgctac                                         29


<210>  44
<211>  27
<212>  DNA
<213>  Artificial

<220>
<223>  PCR primer for amplifying a human histone deacetylase-5 (HDAC5) gene


<400>  44
ggcaagctta tgaactctcc caacgag                                           27


<210>  45
<211>  29
<212>  DNA
<213>  Artificial

<220>
<223>  PCR primer for amplifying a human histone deacetylase-5 (HDAC5) gene

<400>  45
gggcggccgc gtcacagggc aggctcctg                                         29


<210>  46
<211>  27
<212>  DNA
<213>  Artificial

<220>
<223>  PCR primer for amplifying a human histone deacetylase-6 (HDAC6) gene

<400>  46
gcgaagctta tgacctcaac cggccag                                           27


<210>  47
<211>  33
<212>  DNA
<213>  Artificial

<220>
<223>  PCR primer for amplifying a human histone deacetylase-6 (HDAC6) gene

<400>  47
gcatgcggcc gcttagtgtg ggtggggcat atc                                    33


<210>  48

<211>  26
<212>  DNA
<213>  Artificial

<220>
<223>  PCR primer for amplifying a human histone deacetylase-7 (HDAC7) gene

<400>  48
cgaattcagc cgcagcccat ggacct                                          26


<210>  49
<211>  26
<212>  DNA
<213>  Artificial

<220>
<223>  PCR primer for amplifying a human histone deacetylase-7 (HDAC7) gene

<400>  49
cggtaccctg tgcacccgga tcacgg                                          26


<210>  50
<211>  26
<212>  DNA
<213>  Artificial

<220>
<223>  PCR primer for amplifying a human histone deacetylase-8 (HDAC8) gene

<400>  50
cgaattcttt aagcggaaga tggagg                                          26


<210>  51
<211>  26
<212>  DNA
<213>  Artificial

<220>
<223>  PCR primer for amplifying a human histone deacetylase-8 (HDAC8) gene

<400>  51
aggtaccgac cacatgcttc agattc                                          26


<210>  52
<211>  24
<212>  DNA
<213>  Artificial

<220>
<223>  PCR primer for amplifying a human histone deacetylase-3 (HDAC3) gene

<400>  52
ctgaattcac catggccaag accg                                            24


<210>  53
<211>  61
<212>  DNA
<213>  Artificial

<220>
<223>  PCR primer for adding a Flag sequence to a human histone deacetylase-3
       (HDAC3) gene

<400>  53
gggcggccgc ctacttgtca tcgtcgtcct tgtaatcggt accaatctcc acatcgcttt      60

c                                                                     61


<210>  54

<211>  8
<212>  PRT
<213>  Artificial

<220>
<223>  Flag sequence

<400>  54
Asp Tyr Lys Asp Asp Asp Asp Lys
1               5


<210>  55
<211>  31
<212>  DNA
<213>  Artificial

<220>
<223>  PCR primer for adding a Kpn I site to a human histone deacetylase-1
       (HDAC1) gene

<400>  55
atcggtaccg gccaacttga cctcctcctt g                                    31


<210>  56
<211>  29
<212>  DNA
<213>  Artificial

<220>
<223>  PCR primer for adding a Kpn I site to a human histone deacetylase-2
       (HDAC2) gene

<400>  56
atcggtaccg gggttgctga gctgttctg                                       29


<210>  57
<211>  27
<212>  DNA
<213>  Artificial

<220>
<223>  PCR primer for adding a Bgl II site to a human histone deacetylase-4
       (HDAC4) gene

<400>  57
gatagatctc aggggcggct cctcttc                                         27


<210>  58
<211>  46
<212>  DNA
<213>  Artificial

<220>
<223>  Nucleotide sequence having a Flag nucleotide sequence

<400>  58
aattcctgca gagatctgat tacaaggacg acgatgacaa gtaggc                    46


<210>  59
<211>  46
<212>  DNA
<213>  Artificial

<220>
<223>  Nucleotide sequence having a Flag nucleotide sequence

<400>  59
ggccgcctac ttgtcatcgt cgtccttgta atcagatctc tgcagg                    46

<210> 60
<211> 23
<212> DNA
<213> Artificial

<220>
<223> PCR primer for creating a dominant negative mutant (H802K, H803L) of human
histone deacetylase-4 (HDAC4) gene

<400> 60
cagtgacacc atatggaacg agg 23

<210> 61
<211> 20
<212> DNA
<213> Artificial

<220>
<223> PCR primer for creating a dominant negative mutant (H802K, H803L) of human
histone deacetylase-4 (HDAC4) gene

<400> 61
gcaagctttc caggggggcg 20

<210> 62
<211> 20
<212> DNA
<213> Artificial

<220>
<223> PCR primer for creating a dominant negative mutant (H802K, H803L) of human
histone deacetylase-4 (HDAC4) gene

<400> 62
   ccccctggaa agcttgcgga 20

<210> 63
<211> 22
<212> DNA
<213> Artificial

<220>
<223> PCR primer for creating a dominant negative mutant (H802K, H803L) of human
histone deacetylase-4 (HDAC4) gene

<400> 63
gacatgtaca ggacgctagg gt 22

<210> 64
<211> 21
<212> DNA
<213> Artificial

<220>
<223> PCR primer for creating a dominant negative mutant (H863L) of human
histone deacetylase-4 (HDAC4) gene

<400> 64
cctgtacatg tccctccttc g 21

<210> 65
<211> 20
<212> DNA
<213> Artificial

<220>
<223> PCR primer for creating a dominant negative mutant (H863L) of human
histone deacetylase-4 (HDAC4) gene

<400> 65

atagatctag acaggggcgg                                                20

<210>  66
<211>  23
<212>  DNA
<213>  Artificial

<220>
<223>  PCR primer for creating a dominant negative mutant (H140K, H141L) of human
       histone deacetylase-1 (HDAC1) gene

<400>  66
gaggaattca agatggcgca gac                                             23

<210>  67
<211>  25
<212>  DNA
<213>  Artificial

<220>
<223>  PCR primer for creating a dominant negative mutant (H140K, H141L) of human
       histone deacetylase-1 (HDAC1) gene

<400>  67
ctcggacttc tttgcaagct tcagg                                           25

<210>  68
<211>  24
<212>  DNA
<213>  Artificial

<220>
<223>  PCR primer for creating a dominant negative mutant (H140K, H141L) of human
       histone deacetylase-1 (HDAC1) gene

<400>  68
gggcctgaag cttgcaaaga agtc                                            24

<210>  69
<211>  25
<212>  DNA
<213>  Artificial

<220>
<223>  PCR primer for creating a dominant negative mutant (H140K, H141L) of human
       histone deacetylase-1 (HDAC1) gene

<400>  69
gtgaatatca atgtcaatgt acagc                                           25

<210>  70
<211>  21
<212>  DNA
<213>  Artificial

<220>
<223>  PCR primer for creating a dominant negative mutant (H199L) of human
       histone deacetylase-1 (HDAC1) gene

<400>  70
gacggcgtgg aagaggcctt c                                               21

<210>  71
<211>  26
<212>  DNA
<213>  Artificial

<220>
<223>  PCR primer for creating a dominant negative mutant (H199L) of human

histone deacetylase-1 (HDAC1) gene

<400> 71
gaagtactct ccatacttaa gaaagg 26

<210> 72
<211> 26
<212> DNA
<213> Artificial

<220>
<223> PCR primer for creating a dominant negative mutant (H199L) of human
histone deacetylase-1 (HDAC1) gene

<400> 72
ctgtgtcctt tcttaagtat ggagag 26

<210> 73
<211> 22
<212> DNA
<213> Artificial

<220>
<223> PCR primer for creating a dominant negative mutant (H199L) of human
histone deacetylase-1 (HDAC1) gene

<400> 73
tctcggagcg ggtagttaac ag 22

<210> 74
<211> 23
<212> DNA
<213> Artificial

<220>
<223> PCR primer for creating a dominant negative mutant (H134K, H135L) of human
histone deacetylase-3 (HDAC3) gene

<400> 74
aacccagctg aacaacaaga tctg 23

<210> 75
<211> 26
<212> DNA
<213> Artificial

<220>
<223> PCR primer for creating a dominant negative mutant (H134K, H135L) of human
histone deacetylase-3 (HDAC3) gene

<400> 75
ctcaaacttc ttggcaagct tcagac 26

<210> 76
<211> 25
<212> DNA
<213> Artificial

<220>
<223> PCR primer for creating a dominant negative mutant (H134K, H135L) of human
histone deacetylase-3 (HDAC3) gene

<400> 76
tggtggtctg aagcttgcca agaag 25

<210> 77
<211> 21
<212> DNA
<213> Artificial

<220>
<223> PCR primer for creating a dominant negative mutant (H134K, H135L) of human
histone deacetylase-3 (HDAC3) gene

<400> 77
cccgtcacca tggtggatgt c 21

<210> 78
<211> 23
<212> DNA
<213> Artificial

<220>
<223> PCR primer for creating a dominant negative mutant (H193L) of human
histone deacetylase-3 (HDAC3) gene

<400> 78
tgacattgac atccaccatg gtg 23

<210> 79
<211> 26
<212> DNA
<213> Artificial

<220>
<223> PCR primer for creating a dominant negative mutant (H193L) of human
histone deacetylase-3 (HDAC3) gene

<400> 79
gaagtaattt ccgtacttaa ggaagg 26

<210> 80
<211> 26
<212> DNA
<213> Artificial

<220>
<223> PCR primer for creating a dominant negative mutant (H193L) of human
histone deacetylase-3 (HDAC3) gene

<400> 80
tgtccttcct taagtacgga aattac 26

<210> 81
<211> 22
<212> DNA
<213> Artificial

<220>
<223> PCR primer for creating a dominant negative mutant (H193L) of human
histone deacetylase-3 (HDAC3) gene

<400> 81
gagcacaatg cacgtgggtt gg 22

<210> 82
<211> 29
<212> DNA
<213> Artificial

<220>
<223> PCR primer for amplifying RD3 sequence of human N-CoR gene

<400> 82
gggatccgtg aagtccttca gcctgctcc 29

<210> 83
<211> 29

<212>  DNA
<213>  Artificial

<220>
<223>  PCR primer for amplifying RD3 sequence of human N-CoR gene

<400>  83
tgcggccgcc atgggtgagc ctctggaca                                          29


<210>  84
<211>  960
<212>  DNA
<213>  homo sapiens

<220>
<221>  misc_feature
<222>  (1)..(960)
<223>  Corresponding to the sequence (+5281 to +6240) in the GenBank database
       (Acession: AF196971)

<400>  84
gcctctgtct ctcccactcc acccctttcc tttcctaccc tatcccactc ctcgaggaat      60

catccctggc tcccacctca gtttcccgcc tccaaggcag catggcgggc aagaagttga     120

ggccactgtc cctgggtgtt cctaccccca caccctcacc ccaagacagc ctgttactgc     180

ggcgccaaca gccacggtcg cctacatctg ataagactta tctgctgccc cagggcaggc     240

cggagctggc gtaagcccca gtggggcgct aagtgagtgt gcccctgcct cccgccagca     300

ctggcctggc ctgcaggctt agcctgggtc atcaaggtat cccacaggct ctagttcaaa     360

tccagcagaa cctctctgag cctcactctt ctcacctgca aaatgggtac agccacatcc     420

cttctctccc tgcagccagg aagacgcaca tacacaggag tctagcccac accggccccg     480

cacaaattaa gggctttact ctctgaaaag cccagtgaag tcatgaaacc atatctgcta     540

ttttcattta tcttggtttc agcctatttt gcttgtctgg acactacagt ccacgggagc     600

ctaggtcgag cgaggtccaa gaatccccag ggtgggcagg gagggtggaa gagggcctcc     660

agtgcccaag aggtgcccca caagcatggg acccgccccc tcccctggac tgccccaccc     720

actggggcac cagccactcc ctggggagga gggaggaggg agaagggagg gagggaggga     780

gggaggaagg gagcctcaaa ggccaaggcc agccaggaca ccccctggga tcacactgag     840

cttgccacat ccccaaggcg gccgaaccct ccgcaaccac cagcccaggt cagtctcagc     900

ccccagagag cccccaccaa ggcaaccctg ggcctgctgc ccctaccctt atgcttgctc     960


<210>  85
<211>  720
<212>  DNA
<213>  homo sapiens

<220>
<221>  misc_feature
<222>  (1)..(720)
<223>  Corresponding to the sequence (+2321 to +3040) in the GenBank database
       (Acession: AF196971)

<400>  85
gtacaaatgc catggggcct ggaccaagca ggggctctag aaccccagaa gatgccagga      60

gggagtgagc cagtcaggga aggcttccga gaagagagga cattgaagaa gagtctcaaa     120

cttaggcctg acggagaaga cgcgcggcca ggacacccca cccccgccct cgtctccccc     180

aaagcctgat ctggccccac tgattccctt atctgcccac tcccagctgc ctccttgctg     240

gctgaactgt cgccgcagac ttctgagcct gcgccccctc cacggggatg ggggagggaa 300

tggggtgagg cctggcctca cagcctcggg gtttccagct cttgctggag gcagggctct 360

ggggcgccct actcctcacc cttggcttct cttcctgagc gctctgtgct ctccagaaat 420

gaagaaatgg ggtgagtcca gcggccaaac ccttgtctta gctcttagac atgcctcgag 480

cctgccattc cctgtgagga cagatttccc tatgttgcga ccgctgcttc taataataat 540

aatgatgatg ataattccca tttacagagc acaccattta tggtgtgcca gcaggccctg 600

tgctgagtgg ttcctaccca cgtggggggc taggacttta cccgttttcc agatgaagaa 660

actgaggctc agagggcgtc tggcccagga atcacacagc aaatcacaca gcaaatcaga 720

<210> 86
<211> 12
<212> DNA
<213> homo sapiens

<220>
<221> misc_feature
<222> (1)..(12)
<223> Palindrome sequence founed on the 3' side of the GATA-E-box motif in
promoter reagion of human GATA-1 gene

<400> 86
ctgtggccac ag 12